# EUROPEAN PATENT APPLICATION

(11) **EP 4 840 846 A1**
(43) Date of publication of application: **18.11.2026**
(21) Application number: 25840477.1
(22) Date of filing: 18.07.2025
(51) Int. Cl.: C07D 207/09, C07D 209/24, A61K 31/404, A61P 25/00

(54) **PYRROLE-RING-CONTAINING COMPOUND, PHARMACEUTICAL COMPOSITION THEREOF AND USE THEREOF**

(30) Priority: 19.07.2024 CN 202410973274; 21.08.2024 CN 202411153198; 30.09.2024 CN 202411381982; 24.01.2025 CN 202510119403; 28.02.2025 CN 202510235749; 11.07.2025 CN 202510966139
(71) Applicant: Neushen Therapeutics (Shanghai) Co., Ltd, Shanghai 201210 (CN)
(72) Inventor: ZHAO, Lele, Shanghai 201210 (CN); JIA, Haifei, Shanghai 201210 (CN); CHEN, Deheng, Shanghai 201210 (CN); WU, Lingyun, Shanghai 201210 (CN); SHEN, Huaqiong, Shanghai 201210 (CN)
(74) Representative: dompatent
(86) International application number: PCT/CN2025/109386
(87) International publication number: WO 2026/017160

(57) **Abstract**

Disclosed in the present invention are a pyrrole-ring-containing compound, a pharmaceutical composition thereof and the use thereof. Specifically, disclosed in the present invention are a compound as represented by formula (II), a pharmaceutically acceptable salt thereof, a solvate thereof, or a solvate of the pharmaceutically acceptable salt thereof. The compound of the present invention exhibits good agonistic activity against a 5-HT_{2A} receptor.

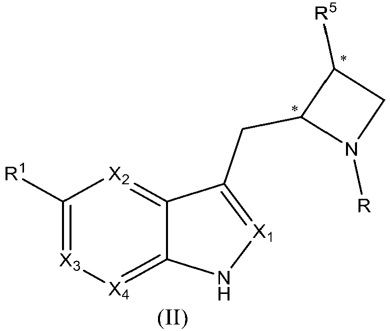

## Description

The present application claims priority to the following Chinese patent applications:
Chinese patent application 2024109732742, filed on July 19, 2024;
Chinese patent application 2024111531987, filed on August 21, 2024;
Chinese patent application 2024113819823, filed on September 30, 2024;
Chinese patent application 2025101194036, filed on January 24, 2025;
Chinese patent application 2025102357492, filed on February 28, 2025;
Chinese patent application 202510966139X, filed on July 11, 2025.

The contents of the above Chinese patent applications are incorporated herein by reference in their entireties.

### TECHNICAL FIELD

The present disclosure relates to a pyrrole-ring-containing compound, a pharmaceutical composition thereof, and a use thereof.

### BACKGROUND

Currently, drugs targeting the regulation of the serotonergic system, such as selective serotonin reuptake inhibitors, serotonin and norepinephrine reuptake inhibitors, and monoamine oxidase inhibitors, have been widely used in the treatment of mental disorders including depression, anxiety disorders, and schizophrenia. However, the aforementioned therapies exhibit slow onset of action, insufficient efficacy or drug resistance in some patients, as well as side effects such as insomnia, changes in blood pressure and body weight, leading to poor patient compliance.

Alterations in synaptic connectivity and plasticity have been observed in the brains of individuals suffering from neurological diseases and disorders. In recent years, preclinical and clinical studies have found that hallucinogens such as ketamine, psilocybin, ibogaine, and lysergic acid diethylamide (LSD) exhibit rapid onset of action, and their long-lasting effects may stem from their unique receptor affinity and regulation of neuroplasticity. Therefore, the development of drugs that regulate neuroplasticity holds broad application prospects for the treatment of neuropsychiatric disorders.

### SUMMARY

The technical problem to be solved by the present disclosure is to provide a 5-HT_{2A} receptor partial agonist with a novel structure. The compounds of the present disclosure exhibit good agonistic activity on the 5-HT_{2A} receptor.

The present disclosure solves the above technical problem through the following technical solutions.

The present disclosure provides a compound represented by formula (II), a pharmaceutically acceptable salt thereof, a solvate thereof, or a solvate of the pharmaceutically acceptable salt thereof: 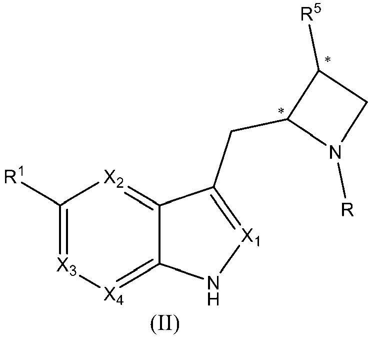 wherein
the configuration of the carbon atom marked with "*" is R configuration, S configuration, or a mixture thereof;
R is C₁-C₆ alkyl, C₃-C₆ cycloalkyl, C₂-C₆ alkynyl, or C₁-C₆ alkyl substituted with one or more R^{a};
R^{a} is D, OH, C₃-C₆ cycloalkyl, cyano, halogen, or -S(=O)₂C₁-C₆ alkyl;
X₁ is N or CH;
X₂ is N or CR²;
R² is H or halogen;
R¹ is H, hydroxyl, halogen, CN, C₁-C₆ alkyl, -S(=O)₂C₁-C₆ alkyl, C₁-C₆ alkoxy, C₃-C₆ cycloalkyl, C₁-C₆ alkyl substituted with one or more R¹⁻¹, or C₁-C₆ alkoxy substituted with one or more R¹⁻²;
R¹⁻¹ and R¹⁻² are each independently halogen.
alternatively, R¹ and R², together with the atoms to which they are attached, form a 5-to 6-membered heterocycloalkene, wherein the heteroatom in the 5- to 6-membered heterocycloalkene is one or more types selected from the group consisting of N, O, and S, and the number of heteroatoms is 1, 2, or 3;
X₃ is N or CR³;
R³ is H or halogen;
alternatively, R³ and R¹, together with the atoms to which they are attached, form a 5-to 6-membered heterocycloalkene, wherein the heteroatom in the 5- to 6-membered heterocycloalkene is one or more types selected from the group consisting of N, O, and S, and the number of heteroatoms is 1, 2, or 3;
X₄ is N or CR⁴;
R⁴ is H or halogen;
R⁵ is H or C₁-C₆ alkyl;
the compound represented by formula (I) is not any one of the following compounds: 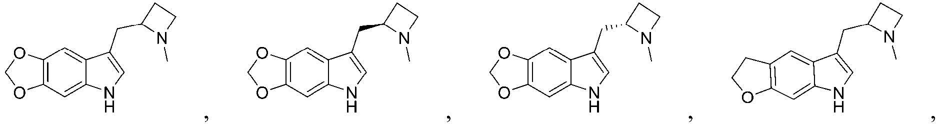 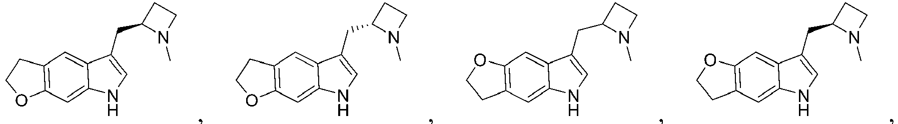 or

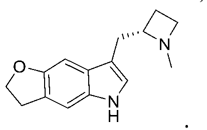

In certain preferred embodiments of the present disclosure, some groups in the compound represented by formula (II), the pharmaceutically acceptable salt thereof, the solvate thereof, or the solvate of the pharmaceutically acceptable salt thereof are defined as follows, and groups not mentioned are as described in any of the embodiments of the present disclosure (abbreviated as "in one embodiment of the present disclosure").

In one embodiment of the present disclosure, in the compound represented by formula (II),
the configuration of the carbon atom marked with "*" is R configuration, S configuration, or a mixture thereof;
R is C₁-C₆ alkyl, C₃-C₆ cycloalkyl, C₂-C₆ alkynyl, or C₁-C₆ alkyl substituted with one or more R^{a};
R^{a} is D, OH, C₃-C₆ cycloalkyl, cyano, halogen, or -S(=O)₂C₁-C₆ alkyl;
X₁ is N or CH;
X₂ is N or CR²;
R² is H or halogen;
R¹ is H, halogen, CN, C₁-C₆ alkyl, -S(=O)₂C₁-C₆ alkyl, C₁-C₆ alkoxy, C₃-C₆ cycloalkyl, C₁-C₆ alkyl substituted with one or more R¹⁻¹, or C₁-C₆ alkoxy substituted with one or more R¹⁻²;
R¹⁻¹ and R¹⁻² are each independently halogen;
alternatively, R¹ and R², together with the atoms to which they are attached, form a 5-to 6-membered heterocycloalkene, wherein the heteroatom in the 5- to 6-membered heterocycloalkene is one or more types selected from the group consisting of N, O, and S, and the number of heteroatoms is 1, 2, or 3;
X₃ is N or CR³;
R³ is H or halogen;
alternatively, R³ and R¹, together with the atoms to which they are attached, form a 5-to 6-membered heterocycloalkene, wherein the heteroatom in the 5- to 6-membered heterocycloalkene is one or more types selected from the group consisting of N, O, and S, and the number of heteroatoms is 1, 2, or 3;
X₄ is N or CR⁴;
R⁴ is H or halogen;
R⁵ is H or C₁-C₆ alkyl;
the compound represented by formula (I) is not any one of the following compounds: 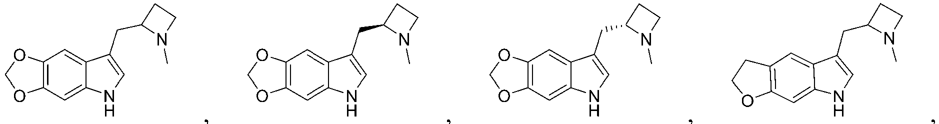 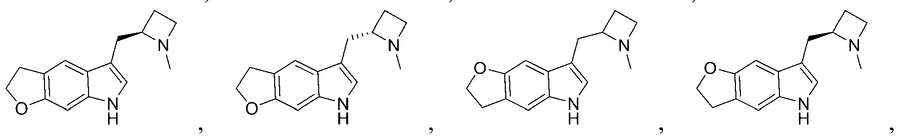 or

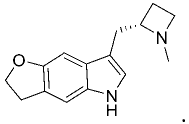

In one embodiment of the present disclosure, the compound represented by formula (II) is a compound represented by formula (I) as shown below: 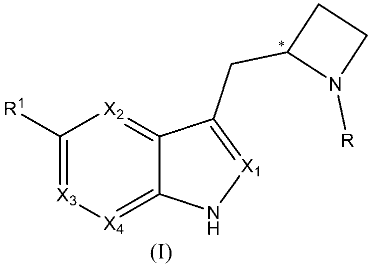 wherein 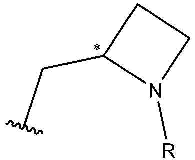 is 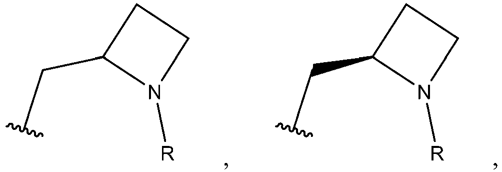 or 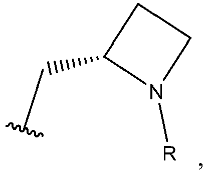 preferably 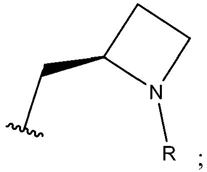
R is C₁-C₆ alkyl, C₃-C₆ cycloalkyl, C₂-C₆ alkynyl, or C₁-C₆ alkyl substituted with one or more R^{a};
R^{a} is D, OH, C₃-C₆ cycloalkyl, cyano, halogen, or -S(=O)₂C₁-C₆ alkyl;
X₁ is N or CH;
X₂ is N or CR²;
R² is H or halogen;
R¹ is H, halogen, CN, C₁-C₆ alkyl, -S(=O)₂C₁-C₆ alkyl, C₁-C₆ alkoxy, C₃-C₆ cycloalkyl, C₁-C₆ alkyl substituted with one or more R¹⁻¹, or C₁-C₆ alkoxy substituted with one or more R¹⁻²;
R¹⁻¹ and R¹⁻² are each independently halogen;
alternatively, R¹ and R², together with the atoms to which they are attached, form a 5-to 6-membered heterocycloalkene, wherein the heteroatom in the 5- to 6-membered heterocycloalkene is one or more types selected from the group consisting of N, O, and S, and the number of heteroatoms is 1, 2, or 3;
X₃ is N or CR³;
R³ is H or halogen;
alternatively, R³ and R¹, together with the atoms to which they are attached, form a 5-to 6-membered heterocycloalkene, wherein the heteroatom in the 5- to 6-membered heterocycloalkene is one or more types selected from the group consisting of N, O, and S, and the number of heteroatoms is 1, 2, or 3;
X₄ is N or CR⁴;
R⁴ is H or halogen;
the compound represented by formula (I) is not any one of the following compounds: 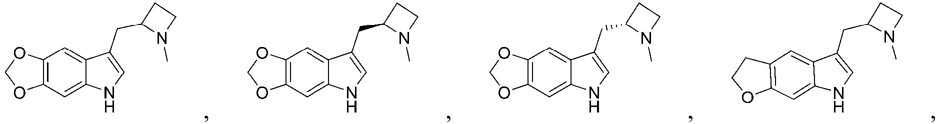 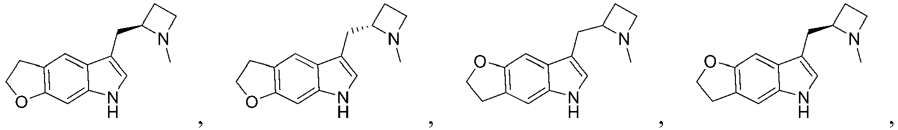 or

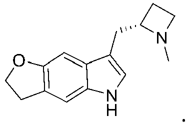

In one embodiment of the present disclosure, the compound represented by formula (I) or the compound represented by formula (II) is also not any one of the following compounds: 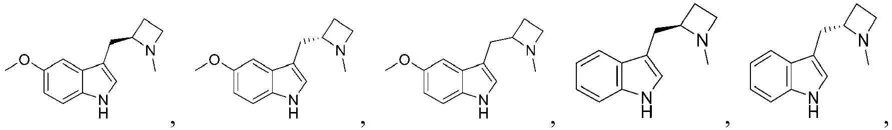 and

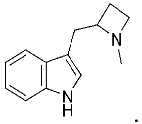

In one embodiment of the present disclosure, each "halogen" is independently F, Cl, Br, or I, for example, F.

In one embodiment of the present disclosure, each "C₁-C₆ alkoxy" is independently methoxy, ethoxy, n-propoxy, isopropoxy, n-butoxy, isobutoxy, sec-butoxy, or *tert-butoxy,* for example, methoxy, ethoxy, or isopropoxy.

In one embodiment of the present disclosure, each "C₁-C₆ alkyl" is independently methyl, ethyl, n-propyl, isopropyl, n-butyl, isobutyl, sec-butyl, or *tert-butyl,* for example, methyl, ethyl, isopropyl, or isobutyl.

In one embodiment of the present disclosure, each "C₂-C₆ alkynyl" is independently ethynyl, propynyl, or propargyl, for example, ethynyl.

In one embodiment of the present disclosure, each "C₃-C₆ cycloalkyl" is independently cyclopropyl, cyclobutyl, cyclopentyl, or cyclohexyl, for example, cyclopropyl.

In one embodiment of the present disclosure, each "5- to 6-membered heterocycloalkene" is independently a 5- to 6-membered heterocycloalkene with 1 or 2 heteroatoms being O, for example, a dihydrofuran ring or a dihydropyran ring, further for example,

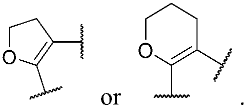

In one embodiment of the present disclosure, 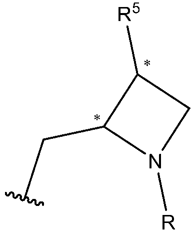 is 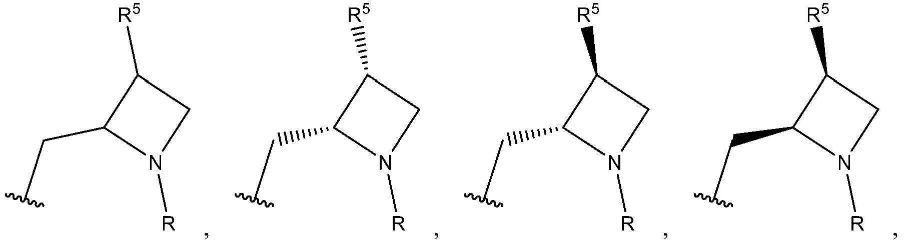 or 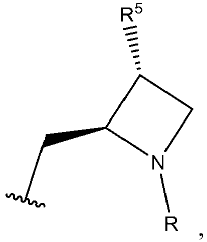 preferably

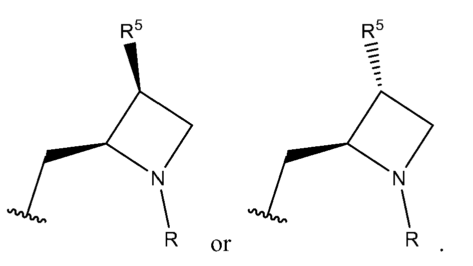

In one embodiment of the present disclosure, R is -CH₃, -CD₃, -CH₂CH₃, 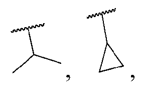 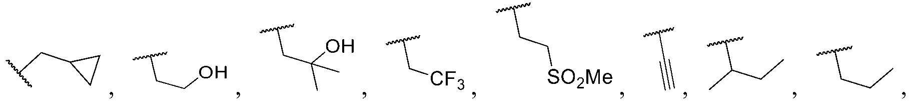 or 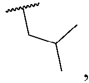 for example, -CH₃ or

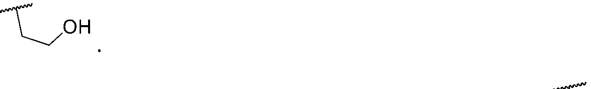

In one embodiment of the present disclosure, R is -CH₃, -CD₃, -CH₂CH₃, 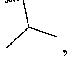 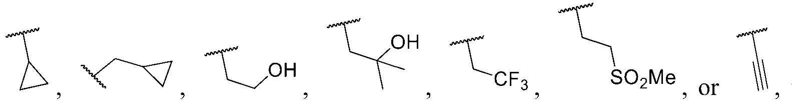 for example, - CH₃.

In one embodiment of the present disclosure, R is C₁-C₆ alkyl.

In one embodiment of the present disclosure, R² is H or F; alternatively, R¹ and R², together with the atoms to which they are attached, form

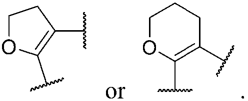

In one embodiment of the present disclosure, R² is H.

In one embodiment of the present disclosure, R¹ is -H, -F, -CN, -CF₃, -OCH₃, 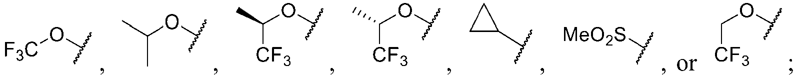 alternatively, R¹ and R², together with the atoms to which they are attached, form

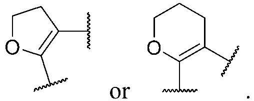

In one embodiment of the present disclosure, R' is -H, -F, -CN, -CH₃, -CF₃, -OCH₃,

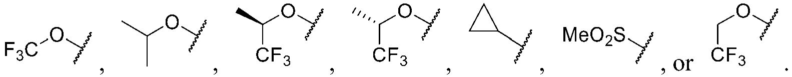

In one embodiment of the present disclosure, R¹ and R², together with the atoms to which they are attached, form

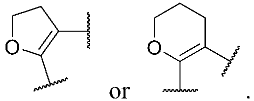

In one embodiment of the present disclosure, R¹ is C₁-C₆ alkoxy or halogen, for example, methoxy or F.

In one embodiment of the present disclosure, R¹ is C₁-C₆ alkoxy, for example, methoxy.

In one embodiment of the present disclosure, R³ is H or F, for example, H.

In one embodiment of the present disclosure, R⁴ is H or F, for example, H.

In one embodiment of the present disclosure, R⁵ is -H or -CH₃.

In one embodiment of the present disclosure, R⁵ is -H.

In one embodiment of the present disclosure, R⁵ is C₁-C₆ alkyl, for example, methyl.

In one embodiment of the present disclosure, 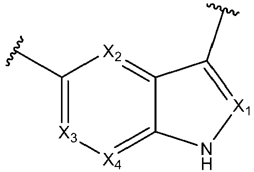 is

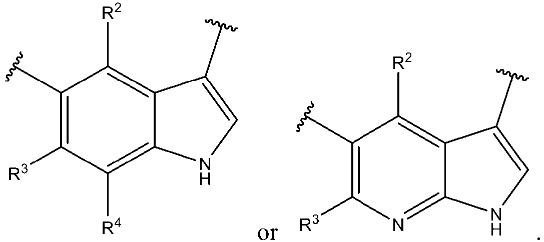

In one embodiment of the present disclosure, 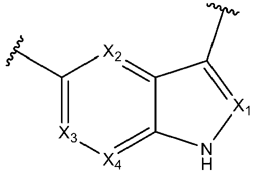 is

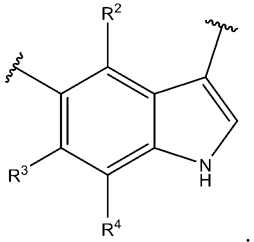

In one embodiment of the present disclosure, 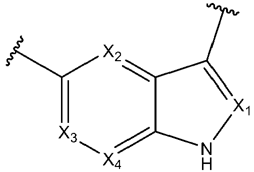 is

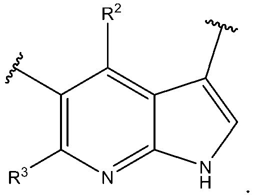

In one embodiment of the present disclosure, 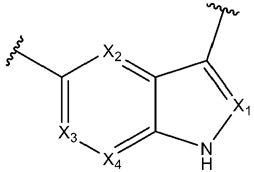 is

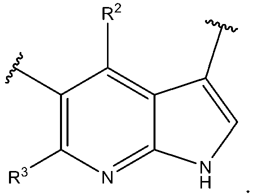

In one embodiment of the present disclosure, 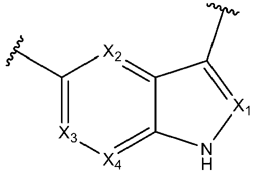 is

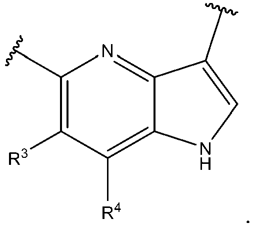

In one embodiment of the present disclosure, 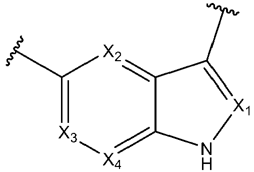 is

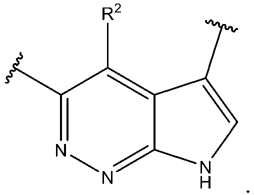

In one embodiment of the present disclosure, 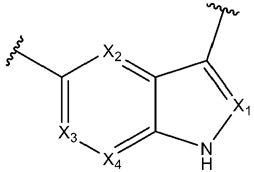 is

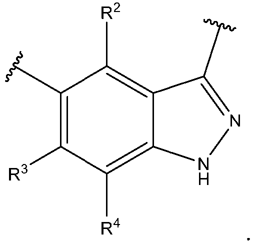

In one embodiment of the present disclosure, X₂ is CR²; R¹ and R², together with the atoms to which they are attached, form a 5- to 6-membered heterocycloalkene.

In one embodiment of the present disclosure, in the compound represented by formula (I), 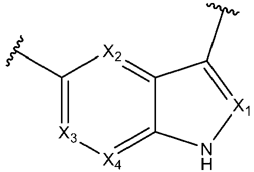 is 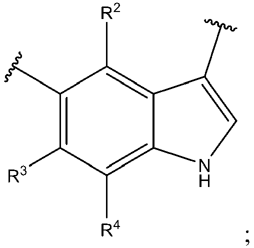
R is C₁-C₆ alkyl or C₁-C₆ alkyl substituted with one or more hydroxyl groups, for example, C₁-C₆ alkyl;
R' is halogen or C₁-C₆ alkoxy;
R², R³, and R⁴ are each independently H or halogen.

In one embodiment of the present disclosure, in the compound represented by formula (I), 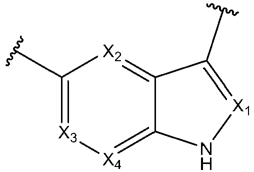 is 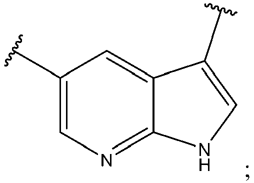
R¹is halogen or C₁-C₆ alkoxy;
R is C₁-C₆ alkyl.

In one embodiment of the present disclosure, in the compound represented by formula (I), 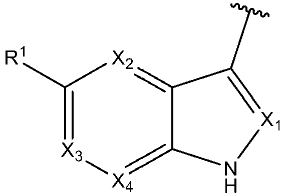 is 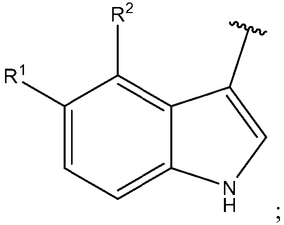
R' and R², together with the atoms to which they are attached, form a 5- to 6-membered heterocycloalkene, wherein the heteroatom in the 5- to 6-membered heterocycloalkene is O, and the number of heteroatoms is 1;
R is C₁-C₆ alkyl or C₁-C₆ alkyl substituted with one or more hydroxyl groups.

In one embodiment of the present disclosure, in the compound represented by formula (I), 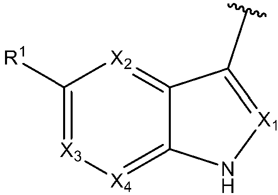 is 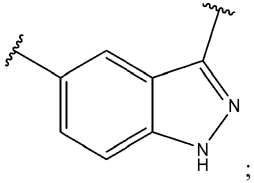
R¹ is C₁-C₆ alkoxy;
R is C₁-C₆ alkyl.

In one embodiment of the present disclosure, the compound represented by formula (II) is any one of the following compounds:

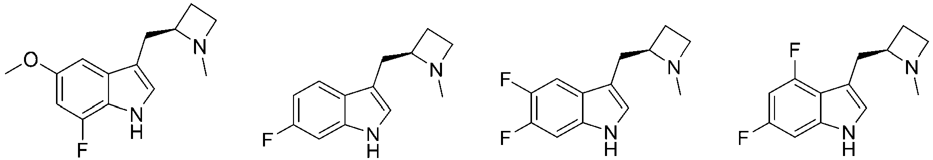

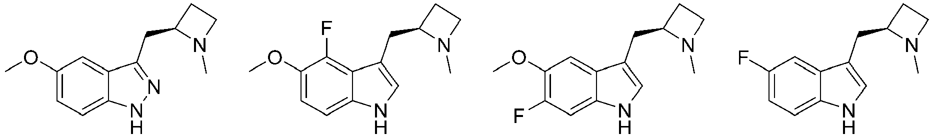

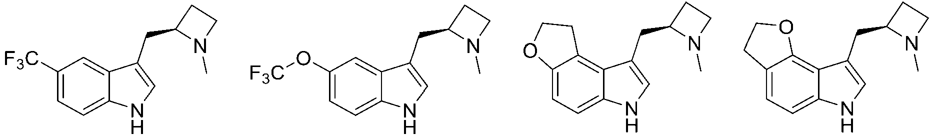

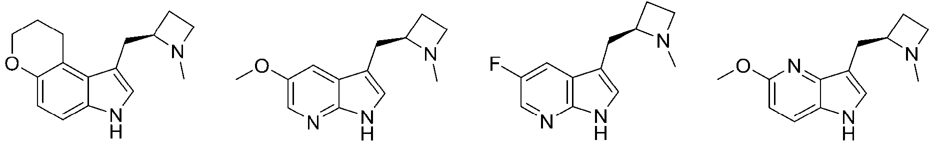

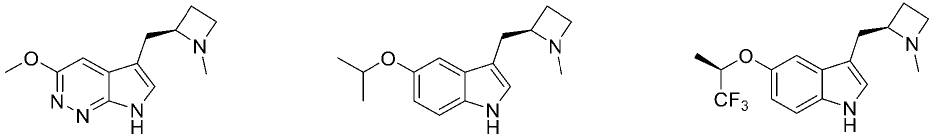

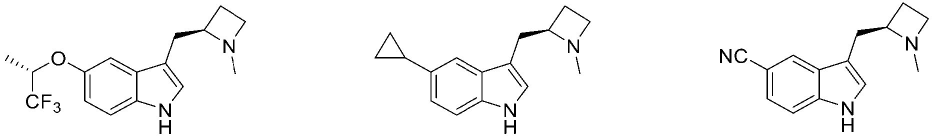

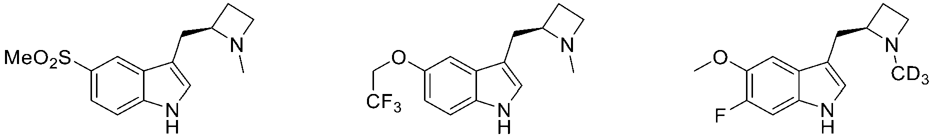

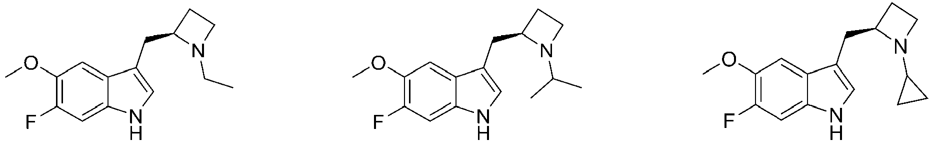

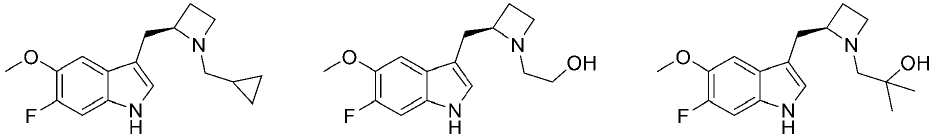

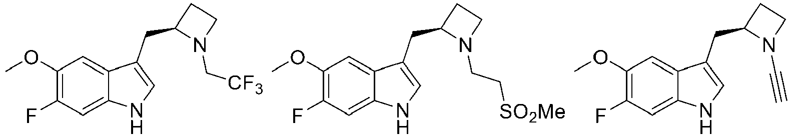

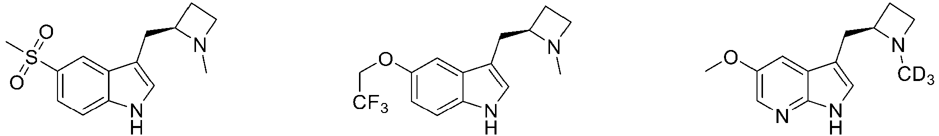

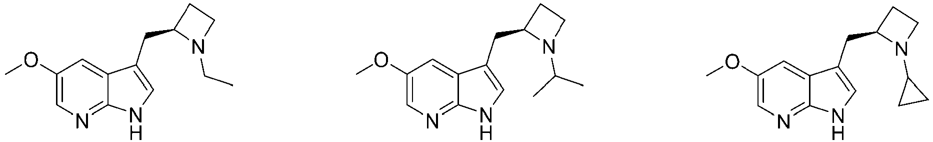

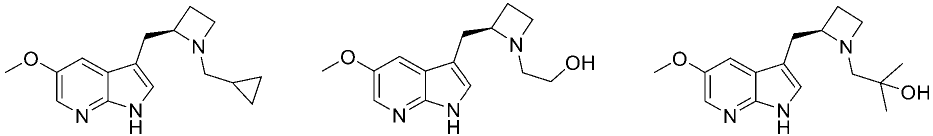

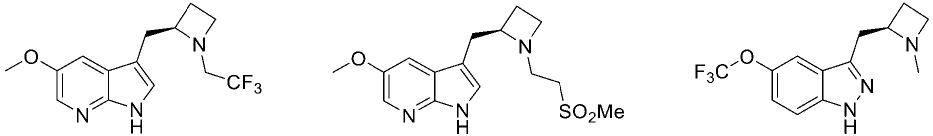

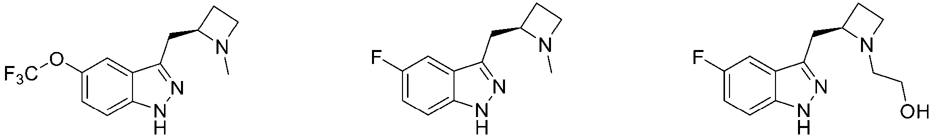

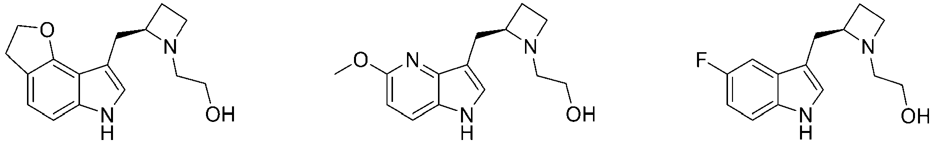

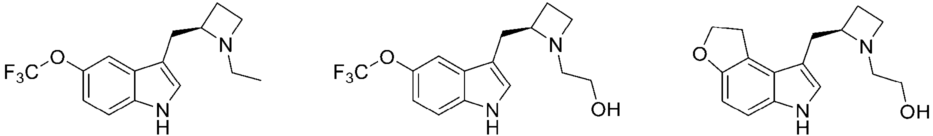

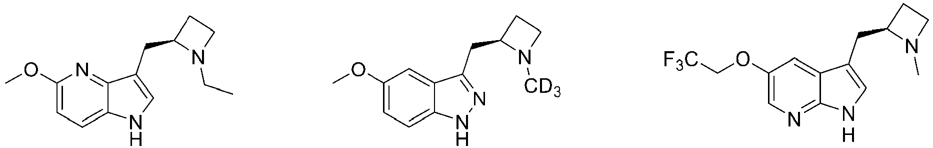

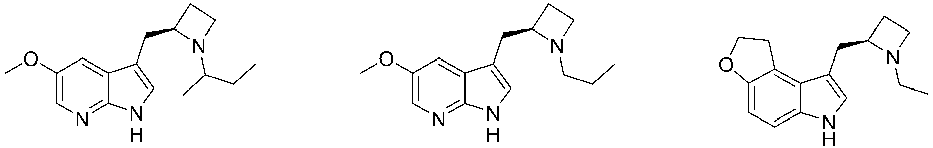

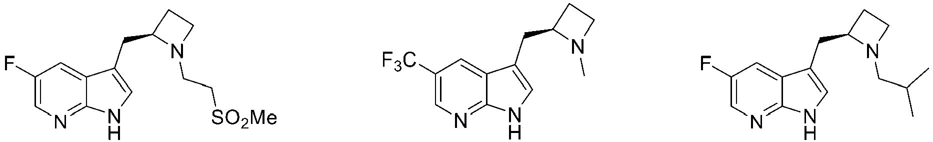

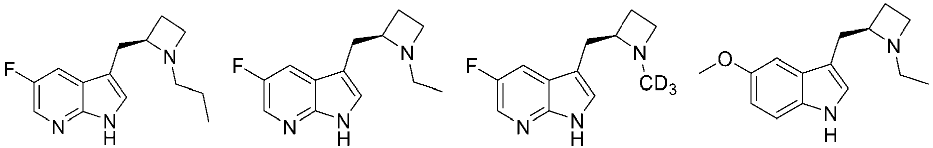

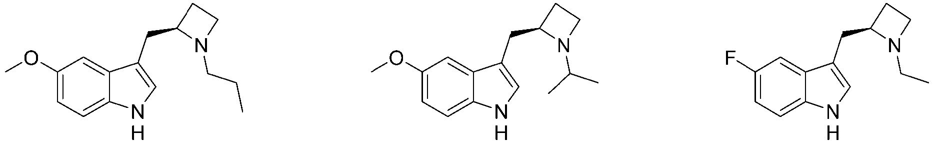

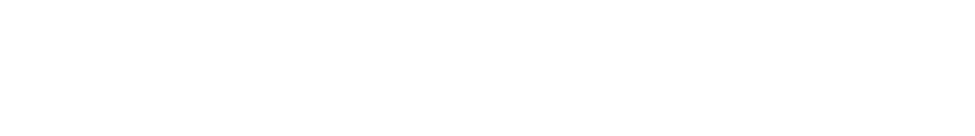

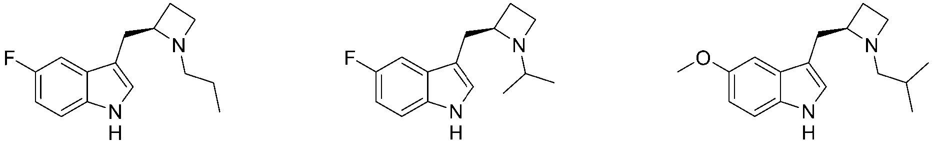

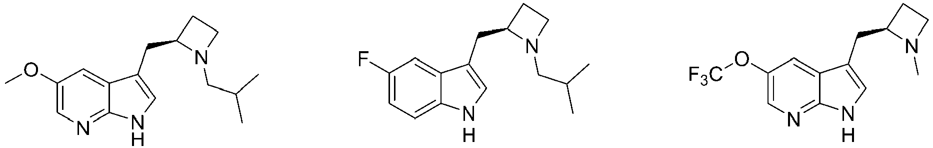

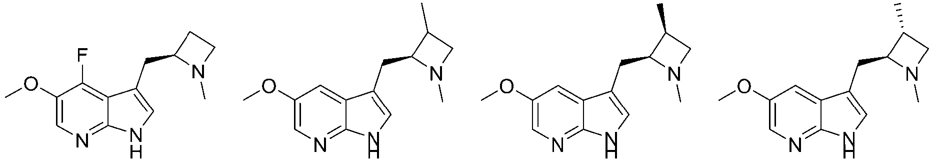

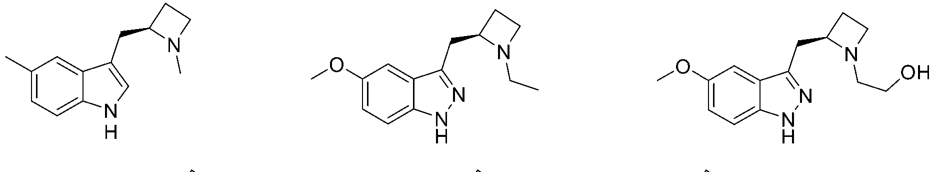

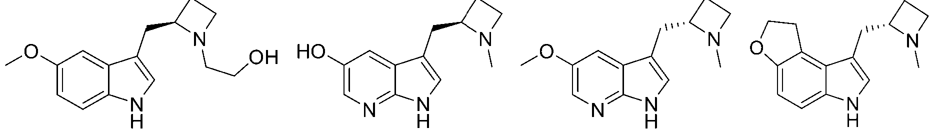

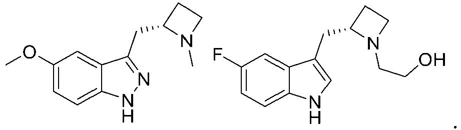

The present disclosure further provides a pharmaceutical composition comprising the compound, the pharmaceutically acceptable salt thereof, the solvate thereof, or the solvate of the pharmaceutically acceptable salt thereof according to any one of the above embodiments, and a pharmaceutically acceptable excipient.

The present disclosure further provides a use of the compound, the pharmaceutically acceptable salt thereof, the solvate thereof, or the solvate of the pharmaceutically acceptable salt thereof according to any one of the above embodiments, or the above pharmaceutical composition in the manufacture of a medicament for regulating neuronal plasticity.

The present disclosure further provides a use of the compound, the pharmaceutically acceptable salt thereof, the solvate thereof, or the solvate of the pharmaceutically acceptable salt thereof according to any one of the above embodiments, or the above pharmaceutical composition in the manufacture of a medicament for preventing and/or treating depression, schizophrenia, anxiety, or post-traumatic stress disorder.

The present disclosure further provides a use of the compound, the pharmaceutically acceptable salt thereof, the solvate thereof, or the solvate of the pharmaceutically acceptable salt thereof according to any one of the above embodiments, or the above pharmaceutical composition in the manufacture of a 5-HT_{2A} receptor agonist; preferably, the 5-HT_{2A} receptor agonist is a 5-HT_{2A} selective receptor agonist, for example, a selective receptor agonist for 5-HT_{2A} relative to 5-HT_{2B}.

The present disclosure further provides a use of the compound, the pharmaceutically acceptable salt thereof, the solvate thereof, or the solvate of the pharmaceutically acceptable salt thereof according to any one of the above embodiments, or the above pharmaceutical composition in the manufacture of a medicament for preventing and/or treating a disease associated with a 5-HT_{2A} receptor; preferably, the disease associated with the 5-HT_{2A} receptor is depression, schizophrenia, anxiety, or post-traumatic stress disorder.

In addition to the foregoing, when used in the specification and claims of the present application, unless otherwise specified, the following terms have the meanings set forth below:

The term "pharmaceutically acceptable salt" refers to a salt prepared from the compound of the present disclosure with a relatively non-toxic, pharmaceutically acceptable acid or base. When the compound of the present disclosure contains a relatively acidic functional group, a base addition salt can be obtained by bringing the neutral form of such compound into contact with a sufficient amount of a pharmaceutically acceptable base in a pure solution or a suitable inert solvent. When the compound of the present disclosure contains a relatively basic functional group, an acid addition salt can be obtained by bringing the neutral form of such compound into contact with a sufficient amount of a pharmaceutically acceptable acid in a pure solution or a suitable inert solvent.

The term "solvate" refers to a substance formed by the combination of a compound with a solvent. Solvates are classified into stoichiometric solvates and non-stoichiometric solvates.

The term "solvate of a pharmaceutically acceptable salt" refers to a substance formed by the combination of a compound with a pharmaceutically acceptable acid or base and a solvent. Herein, the amount of the solvent may be stoichiometric or non-stoichiometric.

The term "halogen" refers to F, Cl, Br, or I.

The term "alkyl" refers to a straight or branched, saturated monovalent hydrocarbon group having a specified number of carbon atoms (e.g., C₁₋₆). Alkyl includes, but is not limited to: methyl, ethyl, n-propyl, isopropyl, n-butyl, isobutyl, *sec*-butyl, tert-butyl, n-pentyl, n-hexyl, *etc.*

The term "alkoxy" refers to the group -O-R_{X}, wherein R_{X} is alkyl as defined above.

The term "alkynyl" refers to a straight or branched hydrocarbon group having one or more triple bonds and a specific number of carbon atoms (e.g., C₂-C₆ alkynyl). The one or more carbon-carbon triple bonds may be internal or terminal.

The term "cycloalkyl" refers to a saturated cyclic group having a specified number of ring carbon atoms (e.g., C₃-C₆), wherein the ring atoms are composed solely of carbon atoms.

The term "heterocycloalkene" refers to an unsaturated cyclic group having a specified number of ring atoms *(e.g.,* 5- to 6-membered), a specified number of heteroatoms *(e.g., 1,* 2, or 3), and a specified type of heteroatoms (one or more of N, O, and S), which is non-aromatic.

Those skilled in the art will appreciate that, in accordance with conventions used in the art, 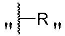 used in the structural formula of a group described in the present disclosure indicates that the corresponding group R is connected to other moieties or groups in the compound via that site.

The term "one or more" refers to 1, 2, 3, 4, or more.

The term "pharmaceutically acceptable excipient" refers to excipients and additives used in the manufacture of pharmaceuticals and the formulation of prescriptions, which are all substances included in the pharmaceutical preparation other than the active ingredient. Reference may be made to the Pharmacopoeia of the People's Republic of China (2020 Edition, Part IV) or the Handbook of Pharmaceutical Excipients (Raymond C Rowe, 2009 Sixth Edition).

On the basis of common knowledge in the art, the above preferred conditions can be arbitrarily combined to obtain preferred examples of the present disclosure.

The reagents and raw materials used in the present disclosure are all commercially available.

The positive and progressive effects of the present disclosure lie in that the compounds of the present disclosure have one or more of the following advantages:
(1) The compounds of the present disclosure exhibit good agonistic activity on the 5-HT_{2A} receptor;
(2) Compared to 5-HT_{2B}, the compounds of the present disclosure have good selective agonistic activity on the 5-HT_{2A} receptor;
(3) The compounds of the present disclosure can promote the growth of primary rat cortical neurons and have the ability to regulate neuronal dendritic neuroplasticity;
(4) The compounds of the present disclosure exhibit low hepatic microsomal clearance and favorable metabolic stability;
(5) The compounds of the present disclosure have a low risk of inhibiting the hERG potassium channel, with an IC₅₀ even reaching 10 µM or above;
(6) The compounds of the present disclosure exhibit good pharmacokinetic properties;
(7) The compounds of the present disclosure are not hallucinogenic;
(8) The compounds of the present disclosure have no effect on the spontaneous activity of test subjects;
(9) The compounds of the present disclosure demonstrate excellent antidepressant efficacy.

### DETAILED DESCRIPTION OF THE PREFERRED EMBODIMENT

The present disclosure is further illustrated by way of examples below, but the present disclosure is not limited to the scope of the described examples. Experimental methods that do not specify specific conditions in the following examples are selected according to conventional methods and conditions or in accordance with the product instructions.

### Example 1

### Synthetic route:

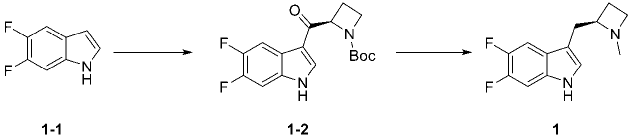

### Step 1

(*R*)-*N*-tert-Butoxycarbonyl-azetidine-2-carboxylic acid (4.40 g, 21.8 mmol) was dissolved in dichloromethane (50 mL). Oxalyl chloride (2.24 mL, 26.2 mmol) was added dropwise at 0°C, and the reaction mixture was stirred at 0°C for 10 minutes. *N,N-*Dimethylformamide (160 mg, 2.32 mmol) was slowly added, and the reaction mixture was stirred at 25°C for 1 hour. The reaction mixture was concentrated under reduced pressure to obtain a crude intermediate. Compound 1-1 (2.00 g, 13.1 mmol) was dissolved in dichloromethane (20 mL). Under a nitrogen atmosphere, ethylmagnesium bromide (6.86 mL, 14.4 mmol, 2 mol/L) was slowly added dropwise at 0°C, and the mixture was stirred at 0°C for 0.5 hours. The crude intermediate was dissolved in dichloromethane (30 mL) and added dropwise to the reaction mixture at 0°C. The reaction mixture was stirred at 0°C for 0.5 hours. Saturated aqueous citric acid solution (100 mL) was added to the reaction mixture, and the mixture was extracted with dichloromethane (60 mL × 3). The organic phases were combined and washed with saturated brine (100 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 1-2. ESI-MS calculated for: [M+H-56]⁺ = 281.13, found 281.0.

### Step 2

Compound 1-2 (250 mg, 0.74 mmol) was dissolved in tetrahydrofuran (20 mL). A solution of lithium aluminum hydride in tetrahydrofuran (2.96 mL, 7.40 mmol, 2.5 mol/L) was added dropwise at 0°C. Under a nitrogen atmosphere, the reaction mixture was stirred at 60°C for 12 hours. The reaction mixture was cooled to room temperature, quenched by the addition of ice water (0.29 mL), and then 15% aqueous sodium hydroxide solution (0.29 mL) and water (0.87 mL) were added. The mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 25-30%, retention time: 10.95-13.35 min, run time: 18 min) to obtain compound 1. ¹H NMR (400 MHz, DMSO-*d*₆): δ 10.95 (s, 1H), 7.49 (dd, J = 11.6, 7.6 Hz, 1H), 7.30 (dd, J = 11.6, 7.6 Hz, 1H), 7.17 (d, J = 1.6 Hz, 1H), 3.24-3.19 (m, 1H), 3.12-3.04 (m, 1H), 2.88-2.83 (m, 1H), 2.74-2.68 (m, 1H), 2.62-2.56 (m, 1H), 2.09 (s, 3H), 1.94-1.87 (m, 1H), 1.80-1.71 (m, 1H), ESI-MS calculated for: [M+H]⁺ = 237.11, found: 237.0.

### Example 2

### Synthetic route:

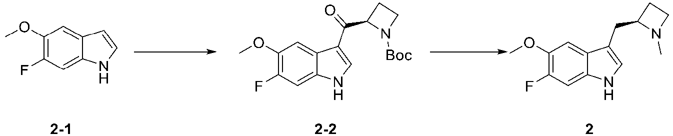

### Step 1

(*R*)-*N*-tert-Butoxycarbonyl-azetidine-2-carboxylic acid (2.20 g, 10.9 mmol) was dissolved in dichloromethane (25 mL). Oxalyl chloride (1.12 mL, 13.1 mmol) was added dropwise at 0°C, and the reaction mixture was stirred at 0°C for 10 minutes. *N,N-*Dimethylformamide (80.0 mg, 1.16 mmol) was slowly added, and the reaction mixture was stirred at 25°C for 1 hour. The reaction mixture was concentrated under reduced pressure to obtain a crude intermediate. Compound 2-1 (1.00 g, 6.05 mmol) was dissolved in dichloromethane (10 mL). Under a nitrogen atmosphere, ethylmagnesium bromide (3.18 mL, 6.35 mmol, 2 mol/L) was slowly added dropwise at 0°C, and the mixture was stirred at 0°C for 0.5 hours. The crude intermediate was dissolved in dichloromethane (15 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (30 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 2-2. ESI-MS calculated for: [M+H-56]⁺ = 293.15, found 293.1.

### Step 2

Compound 2-2 (94.0 mg, 0.27 mmol) was dissolved in tetrahydrofuran (9 mL). A solution of lithium aluminum hydride in tetrahydrofuran (1.08 mL, 2.70 mmol, 2.5 mol/L) was added dropwise at 0°C. Under a nitrogen atmosphere, the reaction mixture was stirred at 60°C for 12 hours. The reaction mixture was cooled to room temperature, quenched by the addition of ice water (0.1 mL), and then 15% aqueous sodium hydroxide solution (0.1 mL) and water (0.3 mL) were added. The mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 19-26%, retention time: 9.80-11.80 min, run time: 17 min) to obtain compound 2. ¹H NMR (400 MHz, DMSO-d₆): δ 10.67 (s, 1H), 7.17 (d, J = 8.4 Hz, 1H), 7.13 (d, J = 11.6 Hz, 1H), 7.04 (d, J = 1.8 Hz, 1H), 3.83 (s, 3H), 3.26-3.19 (m, 1H), 3.13-3.05 (m, 1H), 2.89-2.84 (m, 1H), 2.75-2.69 (m, 1H), 2.63-2.59 (m, 1H), 2.11 (s, 3H), 1.97-1.91 (m, 1H), 1.83-1.75 (m, 1H). ESI-MS calculated for: [M+H]⁺ = 249.13, found: 249.1.

### Example 3

### Synthetic route:

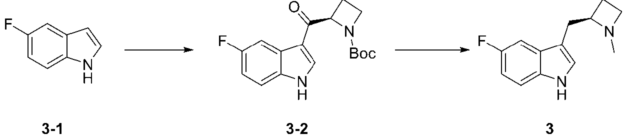

### Step 1

(*R*)-*N*-*tert*-Butoxycarbonyl-azetidine-2-carboxylic acid (4.40 g, 21.8 mmol) was dissolved in dichloromethane (50 mL). Oxalyl chloride (2.24 mL, 26.2 mmol) was added dropwise at 0°C, and the reaction mixture was stirred at 0°C for 10 minutes. *N,N-*Dimethylformamide (160 mg, 2.32 mmol) was slowly added, and the reaction mixture was stirred at 25°C for 1 hour. The reaction mixture was concentrated under reduced pressure to obtain a crude intermediate. Compound 3-1 (2.00 g, 14.8 mmol) was dissolved in dichloromethane (15 mL). Under a nitrogen atmosphere, ethylmagnesium bromide (7.77 mL, 6.45 mmol, 2 mol/L) was slowly added dropwise at 0°C, and the mixture was stirred at 0°C for 0.5 hours. The crude intermediate was dissolved in dichloromethane (10 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 3-2. ESI-MS calculated for: [M+H]⁺ = 319.14, found: 319.0.

### Step 2

Compound 3-2 (100 mg, 0.31 mmol) was dissolved in tetrahydrofuran (15 mL). A solution of lithium aluminum hydride in tetrahydrofuran (1.24 mL, 3.10 mmol, 2.5 mol/L) was added dropwise at 0°C. Under a nitrogen atmosphere, the reaction mixture was stirred at 60°C for 12 hours. The reaction mixture was cooled to room temperature, quenched by the addition of ice water (0.1 mL), and then 15% aqueous sodium hydroxide solution (0.1 mL) and water (0.3 mL) were added. The mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 21-31%, retention time: 9.20-11.40 min, run time: 17 min) to obtain compound 3. ¹H NMR (400 MHz, DMSO-*d*₆): δ 10.87 (s, 1H), 7.30 (dd, *J* = 8.8, 4.4 Hz, 1H), 7.25 (dd, J = 9.8, 2.4 Hz, 1H), 7.17 (d, J = 2.0 Hz, 1H), 6.90-6.85 (m, 1H), 3.23-3.19 (m, 1H), 3.10-3.05 (m, 1H), 2.87-2.83 (m, 1H), 2.76-2.69 (m, 1H), 2.67-2.59 (m, 1H), 2.09 (s, 3H), 1.94-1.87 (m, 1H), 1.80-1.74 (m, 1H). ESI-MS calculated for: [M+H]⁺ = 219.12, found: 219.1.

### Example 4

### Synthetic route:

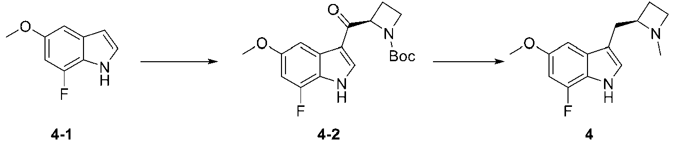

### Step 1

(*R*)-*N*-*tert*-Butoxycarbonyl-azetidine-2-carboxylic acid (1.47 g, 7.27 mmol) was dissolved in dichloromethane (20 mL). Oxalyl chloride (0.75 mL, 8.73 mmol) was added dropwise at 0°C, and the reaction mixture was stirred at 0°C for 10 minutes. *N,N-*Dimethylformamide (53.3 mg, 0.77 mmol) was slowly added, and the reaction mixture was stirred at 25°C for 1 hour. The reaction mixture was concentrated under reduced pressure to obtain a crude intermediate. Compound 4-1 (1.00 g, 6.05 mmol) was dissolved in dichloromethane (10 mL). Under a nitrogen atmosphere, ethylmagnesium bromide (3.18 mL, 6.35 mmol, 2 mol/L) was slowly added dropwise at 0°C, and the mixture was stirred at 0°C for 0.5 hours. The crude intermediate was dissolved in dichloromethane (15 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 4-2. ESI-MS calculated for: [M+H]⁺ = 349.15, found: 349.0.

### Step 2

Compound 4-2 (350 mg, 1.00 mmol) was dissolved in tetrahydrofuran (10 mL). A solution of lithium aluminum hydride in tetrahydrofuran (4.00 mL, 10.0 mmol, 2.5 mol/L) was added dropwise at 0°C. Under a nitrogen atmosphere, the reaction mixture was stirred at 60°C for 12 hours. The reaction mixture was cooled to room temperature, quenched by the addition of ice water (0.35 mL), and then 15% aqueous sodium hydroxide solution (0.35 mL) and water (1.05 mL) were added. The mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 20-25%, retention time: 11.25-14.50 min, run time: 19 min) to obtain compound 4. ¹H NMR (400 MHz, DMSO-d₆): δ 11.09 (s, 1H), 7.12 (d, J = 2.0 Hz, 1H), 6.85 (d, J = 1.6 Hz, 1H), 6.59 (dd, J = 12.8, 2.0 Hz, 1H), 3.77 (s, 3H), 3.24-3.21 (m, 1H), 3.14-3.07 (m, 1H), 2.89-2.84 (m, 1H), 2.73-2.69 (m, 1H), 2.64-2.57 (m, 1H), 2.11 (s, 3H), 1.96-1.91 (m, 1H), 1.84-1.75 (m, 1H). ESI-MS calculated for: [M+H]⁺ = 249.13, found: 249.0.

### Example 5

### Synthetic route:

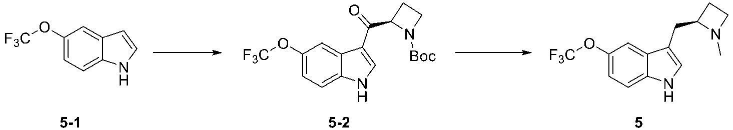

### Step 1

(*R*)-*N*-*tert*-Butoxycarbonyl-azetidine-2-carboxylic acid (1.47 g, 7.27 mmol) was dissolved in dichloromethane (20 mL). Oxalyl chloride (0.75 mL, 8.73 mmol) was added dropwise at 0°C, and the reaction mixture was stirred at 0°C for 10 minutes. *N,N-*Dimethylformamide (53.3 mg, 0.77 mmol) was slowly added, and the reaction mixture was stirred at 25°C for 1 hour. The reaction mixture was concentrated under reduced pressure to obtain a crude intermediate. Compound 5-1 (1.10 g, 6.05 mmol) was dissolved in dichloromethane (15 mL). Under a nitrogen atmosphere, ethylmagnesium bromide (2.87 mL, 5.74 mmol, 2 mol/L) was slowly added dropwise at 0°C, and the mixture was stirred at 0°C for 0.5 hours. The crude intermediate was dissolved in dichloromethane (15 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 5-2. ESI-MS calculated for: [M+H]⁺ = 385.36, found: 385.0.

### Step 2

Compound 5-2 (170 mg, 0.44 mmol) was dissolved in tetrahydrofuran (10 mL). A solution of lithium aluminum hydride in tetrahydrofuran (1.76 mL, 4.40 mmol, 2.5 mol/L) was added dropwise at 0°C. Under a nitrogen atmosphere, the reaction mixture was stirred at 60°C for 12 hours. The reaction mixture was cooled to room temperature, quenched by the addition of ice water (0.17 mL), and then 15% aqueous sodium hydroxide solution (0.17 mL) and water (0.51 mL) were added. The mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-0.1% formic acid in water, gradient: 18-28%, retention time: 6.2-7.8 min, run time: 17 min) to obtain the monoformate salt of compound 5. ¹H NMR (400 MHz, DMSO-*d*₆): δ 11.14 (s, 1H), 8.26 (s, 1H), 7.52 (s, 1H), 7.41 (d, J = 8.4 Hz, 1H), 7.29 (s, 1H), 7.03 (d, J = 8.0 Hz, 1H), 3.43-3.40 (m, 2H), 3.01-2.98 (m, 1H), 2.93-2.84 (m, 2H), 2.21 (s, 3H), 2.07-2.00 (m, 1H), 1.93-1.85 (m, 1H). ESI-MS calculated for: [M+H]⁺ =285.28, found 285.0.

### Examples 6 and 7

### Synthetic route:

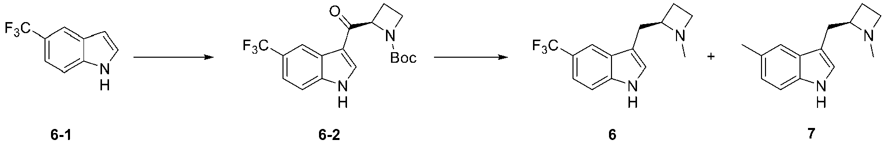

### Step 1

(*R*)-*N*-*tert*-Butoxycarbonyl-azetidine-2-carboxylic acid (1.69 g, 8.36 mmol) was dissolved in dichloromethane (20 mL). Oxalyl chloride (0.86 mL, 10.0 mmol) was added dropwise at 0°C, and the reaction mixture was stirred at 0°C for 10 minutes. *N,N-*Dimethylformamide (61.3 mg, 0.89 mmol) was slowly added, and the reaction mixture was stirred at 25°C for 1 hour. The reaction mixture was concentrated under reduced pressure to obtain a crude intermediate. Compound 6-1 (1.30 g, 7.02 mmol) was dissolved in dichloromethane (20 mL). Under a nitrogen atmosphere, ethylmagnesium bromide (3.86 mL, 7.72 mmol, 2 mol/L) was slowly added dropwise at 0°C, and the mixture was stirred at 0°C for 0.5 hours. The crude intermediate was dissolved in dichloromethane (20 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 6-2. ESI-MS calculated for: [M+H]⁺ = 369.13, found: 368.9.

### Step 2

Compound 6-2 (120 mg, 0.33 mmol) was dissolved in tetrahydrofuran (10 mL). A solution of lithium aluminum hydride in tetrahydrofuran (1.32 mL, 3.30 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.10 mL), and then 15% aqueous sodium hydroxide solution (0.20 mL) and water (0.10 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-0.1% formic acid in water, gradient: 20-30%, run time: 17 min) to obtain the monoformate salt of compound 6 (retention time: 7.0-8.5 min). ¹H NMR (400 MHz, DMSO-d₆): δ 11.31 (s, 1H), 8.25 (s, 1H), 7.92 (s, 1H), 7.51 (d, J = 8.4 Hz, 1H), 7.34 (d, J = 9.6 Hz, 2H), 3.34-3.29 (m, 2H), 3.07-2.99 (m, 1H), 2.90-2.84 (m, 1H), 2.80-2.74 (m, 1H), 2.15 (s, 3H), 1.99-1.95 (m, 1H), 1.88-1.80 (m, 1H). ESI-MS calculated for: [M+H]⁺ = 269.12, found 269.1. And the monoformate salt of compound 7 (retention time: 4.0-5.0 min). ¹H NMR (400 MHz, DMSO-d₆): δ 10.61 (s, 1H), 8.23 (s, 1H), 7.28 (s, 1H), 7.19 (d, J = 8.4 Hz, 1H), 7.03 (d, J = 2.0 Hz, 1H), 6.86 (d, J = 8.4 Hz, 1H), 3.20-3.17 (m, 1H), 3.12-3.04 (m, 1H), 2.90-2.85 (m, 1H), 2.73-2.65 (m, 1H), 2.62-2.57 (m, 1H), 2.37 (s, 3H), 2.11 (s, 3H), 1.93-1.88 (m, 1H), 1.80-1.73 (m, 1H). ESI-MS calculated for: [M+H]⁺ = 215.15, found 215.0.

### Example 8

### Synthetic route:

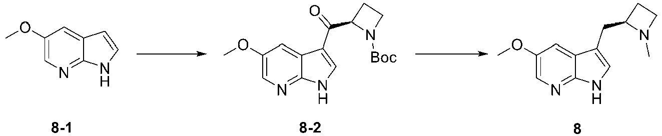

### Step 1

(*R*)-*N*-*tert*-Butoxycarbonyl-azetidine-2-carboxylic acid (4.41 g, 21.8 mmol) was dissolved in dichloromethane (50 mL). Oxalyl chloride (2.25 mL, 26.2 mmol) was added dropwise at 0°C, and the reaction mixture was stirred at 0°C for 10 minutes. *N,N-*Dimethylformamide (160 mg, 2.31 mmol) was slowly added, and the reaction mixture was stirred at 25°C for 1 hour. The reaction mixture was concentrated under reduced pressure to obtain a crude intermediate. Compound 8-1 (3.00 g, 20.3 mmol) was dissolved in dichloromethane (30 mL). Under a nitrogen atmosphere, ethylmagnesium bromide (10.6 mL, 21.3 mmol, 2 mol/L) was slowly added dropwise at 0°C, and the mixture was stirred at 0°C for 0.5 hours. The crude intermediate was dissolved in dichloromethane (30 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 8-2. ESI-MS calculated for: [M+H]⁺ = 332.15, found: 332.0.

### Step 2

Compound **8-2** (120 mg, 0.36 mmol) was dissolved in tetrahydrofuran (5 mL). A solution of lithium aluminum hydride in tetrahydrofuran (1.44 mL, 3.60 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.15 mL), and then 15% aqueous sodium hydroxide solution (0.15 mL) and water (0.45 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-0.1% formic acid in water, gradient: 6-16%, retention time: 6.8-7.8 min, run time: 17 min) to obtain the monoformate salt of compound 8. ¹H NMR (400 MHz, DMSO-*d*₆): δ 11.18 (s, 1H), 8.26 (s, 1H), 7.93 (d, J = 2.8 Hz, 1H), 7.52 (d, J = 2.8 Hz, 1H), 7.20 (d, J = 2.0 Hz, 1H), 3.82 (s, 3H), 3.29-3.25 (m, 1H), 3.24-3.20 (m, 1H), 2.93-2.85 (m, 1H), 2.82-2.75 (m, 1H), 2.73-2.69 (m, 1H), 2.14 (s, 3H), 1.99-1.93 (m, 1H), 1.87-1.78 (m, 1H). ESI-MS calculated for: [M+H]⁺ = 232.14, found 232.0.

### Example 9

### Synthetic route:

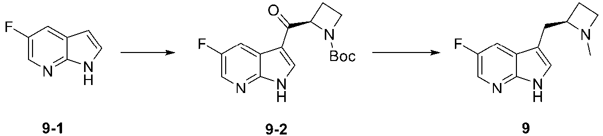

### Step 1

(*R*)-*N*-*tert*-Butoxycarbonyl-azetidine-2-carboxylic acid (5.29 g, 26.2 mmol) was dissolved in dichloromethane (50 mL). Oxalyl chloride (2.70 mL, 31.4 mmol) was added dropwise at 0°C, and the reaction mixture was stirred at 0°C for 10 minutes. *N,N-*Dimethylformamide (192 mg, 2.77 mmol) was slowly added, and the reaction mixture was stirred at 25°C for 1 hour. The reaction mixture was concentrated under reduced pressure to obtain a crude intermediate. Compound 9-1 (3.00 g, 22.0 mmol) was dissolved in dichloromethane (30 mL). Under a nitrogen atmosphere, ethylmagnesium bromide (11.6 mL, 23.1 mmol, 2 mol/L) was slowly added dropwise at 0°C, and the mixture was stirred at 0°C for 0.5 hours. The crude intermediate was dissolved in dichloromethane (30 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 9-2. ESI-MS calculated for: [M+H]⁺ = 320.13, found: 320.0.

### Step 2

Compound 9-2 (105 mg, 0.33 mmol) was dissolved in tetrahydrofuran (5 mL). A solution of lithium aluminum hydride in tetrahydrofuran (1.72 mL, 4.29 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.17 mL), and then 15% aqueous sodium hydroxide solution (0.17 mL) and water (0.51 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-0.04% aqueous ammonia solution + 7.5 mmol/L ammonium bicarbonate solution, gradient: 20-30%, retention time: 9.0-10.0 min, run time: 17 min) to obtain compound 9. ¹H NMR (400 MHz, DMSO-d₆): δ 11.49 (s, 1H), 8.14 (s, 1H), 7.83 (dd, J = 9.6, 2.4 Hz, 1H), 7.33 (s, 1H), 3.24-3.18 (m, 1H), 3.11-3.06 (m, 1H), 2.90-2.83 (m, 1H), 2.78-2.71 (m, 1H), 2.62-2.56 (m, 1H), 2.08 (s, 3H), 1.92-1.85 (m, 1H), 1.81-1.74 (m, 1H). ESI-MS calculated for: [M+H]⁺ = 220.12, found 220.1.

### Example 10

### Synthetic route:

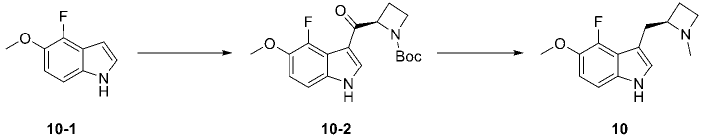

### Step 1

(*R*)-*N*-tert-Butoxycarbonyl-azetidine-2-carboxylic acid (1.32 g, 6.55 mmol) was dissolved in dichloromethane (20 mL). Oxalyl chloride (0.675 mL, 7.85 mmol) was added dropwise at 0°C, and the reaction mixture was stirred at 0°C for 10 minutes. *N,N-*Dimethylformamide (48.0 mg, 0.693 mmol) was slowly added, and the reaction mixture was stirred at 25°C for 1 hour. The reaction mixture was concentrated under reduced pressure to obtain a crude intermediate. Compound 10-1 (1.00 g, 6.05 mmol) was dissolved in dichloromethane (20 mL). Under a nitrogen atmosphere, ethylmagnesium bromide (3.18 mL, 6.35 mmol, 2 mol/L) was slowly added dropwise at 0°C, and the mixture was stirred at 0°C for 0.5 hours. The crude intermediate was dissolved in dichloromethane (30 mL) and added dropwise to the reaction mixture at 0°C. The reaction mixture was stirred at 0°C for 0.5 hours. Saturated aqueous citric acid solution (30 mL) was added to the reaction mixture, and the mixture was extracted with dichloromethane (30 mL × 3). The organic phases were combined and washed with saturated brine (30 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound 10-2. ESI-MS calculated for: [M+H]⁺ = 349.15, found: 349.0.

### Step 2

Compound **10-2** (250 mg, 0.72 mmol) was dissolved in tetrahydrofuran (10 mL). A solution of lithium aluminum hydride in tetrahydrofuran (2.88 mL, 7.20 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.28 mL), and then 15% aqueous sodium hydroxide solution (0.28 mL) and water (0.84 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 21-27%, retention time: 9.1-11.3 min, run time: 18 min) to obtain compound 10. ¹H NMR (400 MHz, DMSO-d₆): δ 10.85 (s, 1H), 7.07 (d, J = 2.0 Hz, 1H), 7.05 (d, J = 8.8 Hz, 1H), 6.93 (t, J = 8.4 Hz, 1H), 3.80 (s, 3H), 3.23-3.19 (m, 1H), 3.16-3.09 (m, 1H), 2.98-2.93 (m, 1H), 2.80-2.74 (m, 1H), 2.63-2.54 (m, 1H), 2.10 (s, 3H), 1.90-1.84 (m, 1H), 1.82-1.71 (m, 1H). ESI-MS calculated for: [M+H]⁺ = 249.13, found 249.0.

### Example 11

### Synthetic route:

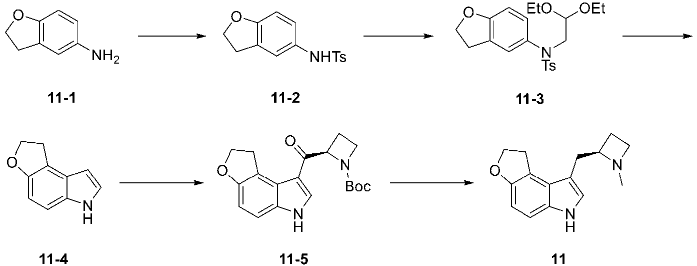

### Step 1

Compound **11-1** (12.5 g, 92.5 mmol) and pyridine (29.3 g, 370 mmol) were dissolved in dichloromethane (125 mL). Tosyl chloride (19.4 g, 102 mmol) was added portionwise at 0°C, and the reaction mixture was stirred at 25°C for 16 hours. Water (250 mL) was added to the reaction mixture, and the mixture was extracted with dichloromethane (250 mL × 3). The organic phases were combined and washed with saturated brine (200 mL × 3). The organic phase was dried over anhydrous sodium sulfate, filtered, and concentrated under reduced pressure to obtain compound 11-2. ¹H NMR (400 MHz, CDCl₃): δ 7.59 (d, J = 8.4 Hz, 2H), 7.22 (d, J = 8.4 Hz, 2H), 7.01 (s, 1H), 6.70-6.62 (m, 2H), 6.57 (d, J = 8.4 Hz, 1H), 4.54 (t, J = 8.8 Hz, 2H), 3.14 (t, J = 8.8 Hz, 2H), 2.38 (s, 3H). ESI-MS calculated for: [M+Na]⁺ = 312.08, found 312.2.

### Step 2

Compound **11-2** (15.0 g, 51.8 mmol), cesium carbonate (42.2 g, 130 mmol), potassium iodide (1.72 g, 10.4 mmol), and bromoacetaldehyde diethyl acetal (102 g, 518 mmol) were dissolved in N,Ndimethylformamide (450 mL), and the reaction mixture was stirred at 110°C for 16 hours. The reaction mixture was cooled to room temperature and filtered. The filter cake was washed with ethyl acetate (500 mL). Water (1000 mL) was added to the filtrate, and the mixture was extracted with ethyl acetate (800 mL × 3). The organic phases were combined and washed with saturated brine (500 mL × 3). The organic phase was dried over anhydrous sodium sulfate, filtered, and concentrated under reduced pressure. The crude product was purified by silica gel column chromatography (petroleum ether/ethyl acetate, 10/1, v/v) to obtain compound 11-3. ¹H NMR (400 MHz, CDCl₃): δ 7.50 (d, J = 8.0 Hz, 2H), 7.24 (d, *J* = 8.0 Hz, 2H), 7.00 (s, 1H), 6.62-6.56 (m, 2H), 4.61-4.55 (m, 3H), 3.67-3.59 (m, 2H), 3.58 (d, J = 5.6 Hz, 2H), 3.52-3.45 (m, 2H), 3.17 (t, J = 8.8 Hz, 2H), 2.42 (s, 3H), 1.14 (t, J = 7.2 Hz, 6H).

### Step 3

Compound **11-3** (10.5 g, 25.9 mmol) was dissolved in toluene (400 mL), and a solution of titanium tetrachloride (7.37 g, 38.8 mmol) in toluene (240 mL) was added dropwise at 110°C. The reaction mixture was stirred at 110°C for 1 hour. The reaction mixture was cooled to room temperature, then saturated aqueous sodium bicarbonate solution (400 mL) was added, and the mixture was extracted with ethyl acetate (750 mL × 3). The organic phases were combined and washed with saturated brine (500 mL × 3). The organic phase was dried over anhydrous sodium sulfate, filtered, and concentrated under reduced pressure. The resulting crude product was dissolved in ethanol (75 mL), tetrahydrofuran (50 mL), and water (25 mL). Potassium hydroxide (16.3 g, 290 mmol) was added in portions, and the reaction mixture was stirred at 80°C for 16 hours. The reaction mixture was cooled to room temperature, then water (300 mL) was added, and the mixture was extracted with ethyl acetate (300 mL × 3). The organic phases were combined and washed with saturated brine (200 mL × 3). The organic phase was dried over anhydrous sodium sulfate, filtered, and concentrated under reduced pressure. The crude product was purified by silica gel column chromatography (petroleum ether/ethyl acetate, 3/1, v/v) to obtain compound **11-4.** ¹H NMR (400 MHz, CDCl₃) δ 8.09 (s, 1H), 7.22 (t, J = 2.8 Hz, 1H), 7.16 (dd, J = 8.4, 0.8 Hz, 1H), 6.78 (d, J = 8.4 Hz, 1H), 6.37 (dd, J = 3.6, 1.6 Hz, 1H), 4.65 (t, J = 8.8 Hz, 2H), 3.38 (t, J = 8.8 Hz, 2H). ESI-MS calculated for: [M+H]⁺ = 160.07, found: 160.0.

### Step 4

(*R*)-*N*-*tert*-Butoxycarbonyl-azetidine-2-carboxylic acid (2.64 g, 13.1 mmol) was dissolved in dichloromethane (30 mL). Oxalyl chloride (1.35 mL, 15.7 mmol) was added dropwise at 0°C, and the reaction mixture was stirred at 0°C for 10 minutes. *N,N-*Dimethylformamide (96.0 mg, 1.39 mmol) was slowly added, and the reaction mixture was stirred at 25°C for 1 hour. The reaction mixture was concentrated under reduced pressure to obtain a crude intermediate. Compound **11-4** (1.67 g, 10.5 mmol) was dissolved in dichloromethane (20 mL). Under a nitrogen atmosphere, ethylmagnesium bromide (5.51 mL, 11.0 mmol, 2 mol/L) was slowly added dropwise at 0°C, and the mixture was stirred at 0°C for 0.5 hours. The crude intermediate was dissolved in dichloromethane (30 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound **11-5.** ESI-MS calculated for: [M+H]⁺ = 343.16, found: 343.0.

### Step 5

Compound **11-5** (263 mg, 0.77 mmol) was dissolved in tetrahydrofuran (10 mL). A solution of lithium aluminum hydride in tetrahydrofuran (3.08 mL, 7.70 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.3 mL), and then 15% aqueous sodium hydroxide solution (0.3 mL) and water (0.9 mL) were added. The mixture was filtered, and the filtrate was concentrated under reduced pressure to obtain a crude product containing the target compound. The crude product was purified by silica gel column chromatography (dichloromethane/methanol, 9/1, v/v) to obtain compound **11.** ¹H NMR (400 MHz, DMSO-d₆): δ 10.59 (s, 1H), 7.06-7.02 (m, 2H), 6.56 (d, J = 8.4 Hz, 1H), 4.51 (t, J = 8.8 Hz, 2H), 3.48-3.44 (m, 2H), 3.26-3.18 (m, 1H), 3.12-3.03 (m, 1H), 2.97-2.92 (m, 1H), 2.75-2.65 (m, 1H), 2.61-2.54 (m, 1H), 2.12 (s, 3H), 1.95-1.89 (m, 1H), 1.82-1.75 (m, 1H). ESI-MS calculated for: [M+H]⁺ = 243.14, found 243.0.

### Example 12

### Synthetic route:

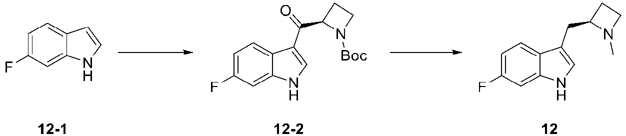

### Step 1

(*R*)-*N*-*tert*-Butoxycarbonyl-azetidine-2-carboxylic acid (3.30 g, 19.7 mmol) was dissolved in dichloromethane (40 mL). Oxalyl chloride (1.69 mL, 19.6 mmol) was added dropwise at 0°C, and the reaction mixture was stirred at 0°C for 10 minutes. *N,N-*Dimethylformamide (120 mg, 1.73 mmol) was slowly added, and the reaction mixture was stirred at 25°C for 1 hour. The reaction mixture was concentrated under reduced pressure to obtain a crude intermediate. Compound **12-1** (2.00 g, 14.8 mmol) was dissolved in dichloromethane (20 mL). Under a nitrogen atmosphere, ethylmagnesium bromide (7.77 mL, 15.5 mmol, 2 mol/L) was slowly added dropwise at 0°C, and the mixture was stirred at 0°C for 0.5 hours. The crude intermediate was dissolved in dichloromethane (30 mL) and added dropwise to the reaction mixture at 0°C. The reaction mixture was stirred at 0°C for 0.5 hours. Saturated aqueous citric acid solution (30 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound **12-2.** ESI-MS calculated for: [M+H]⁺ = 319.14, found: 319.0.

### Step 2

Compound **12-2** (80.0 mg, 0.25 mmol) was dissolved in tetrahydrofuran (10 mL). A solution of lithium aluminum hydride in tetrahydrofuran (1 mL, 2.50 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.1 mL), and then 15% aqueous sodium hydroxide solution (0.1 mL) and water (0.3 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 26-37%, retention time: 10.2-11.8 min, run time: 18 min) to obtain compound 12. ¹H NMR (400 MHz, DMSO-d₆): δ 10.84 (s, 1H), 7.50 (dd, J = 8.4, 5.6 Hz, 1H), 7.11-7.07 (m, 2H), 6.82 (t, J = 8.4 Hz, 1H), 3.22 (t, J = 6.4 Hz, 1H), 3.13-3.06 (m, 1H), 2.91-2.86 (m, 1H), 2.75-2.70 (m, 1H), 2.62-2.56 (m, 1H), 2.10 (s, 3H), 1.96-1.85 (m, 1H), 1.80-1.72 (m, 1H). ESI-MS calculated for: [M+H]⁺ = 219.12, found 219.0.

### Example 13

### Synthetic route:

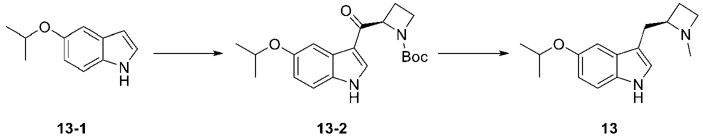

### Step 1

(*R*)-*N*-tert-Butoxycarbonyl-azetidine-2-carboxylic acid (1.32 g, 6.55 mmol) was dissolved in dichloromethane (20 mL). Oxalyl chloride (0.675 mL, 7.85 mmol) was added dropwise at 0°C, and the reaction mixture was stirred at 0°C for 10 minutes. *N,N-*Dimethylformamide (48.0 mg, 0.693 mmol) was slowly added, and the reaction mixture was stirred at 25°C for 1 hour. The reaction mixture was concentrated under reduced pressure to obtain a crude intermediate. Compound **13-1** (1.00 g, 5.71 mmol) was dissolved in dichloromethane (15 mL). Under a nitrogen atmosphere, ethylmagnesium bromide (3.00 mL, 6.00 mmol, 2 mol/L) was slowly added dropwise at 0°C, and the mixture was stirred at 0°C for 0.5 hours. The crude intermediate was dissolved in dichloromethane (20 mL) and added dropwise to the reaction mixture at 0°C. The reaction mixture was stirred at 0°C for 0.5 hours. Saturated aqueous citric acid solution (30 mL) was added to the reaction mixture, and the mixture was extracted with dichloromethane (30 mL × 3). The organic phases were combined and washed with saturated brine (30 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound **13-2.** ESI-MS calculated for: [M+H]⁺ = 359.19, found: 359.0.

### Step 2

Compound **13-2** (100 mg, 0.28 mmol) was dissolved in tetrahydrofuran (5 mL). A solution of lithium aluminum hydride in tetrahydrofuran (1.12 mL, 5.00 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.12 mL), and then 15% aqueous sodium hydroxide solution (0.12 mL) and water (0.36 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 24-33%, retention time: 8.45-10.35 min, run time: 16 min) to obtain compound **13.** ¹H NMR (400 MHz, DMSO-*d*₆): δ 10.58 (s, 1H), 7.18 (d, J = 8.8 Hz, 1H), 7.03 (d, J = 2.0 Hz, 1H), 6.99 (d, J = 2.0 Hz, 1H), 6.68 (dd, J = 8.8, 2.4 Hz, 1H), 4.54-4.46 (m, 1H), 3.25-3.19 (m, 1H), 3.11-3.04 (m, 1H), 2.88-2.80 (m, 1H), 2.73-2.66 (m, 1H), 2.62-2.57 (m, 1H), 2.11 (s, 3H), 1.95-1.88 (m, 1H), 1.82-1.75 (m, 1H), 1.25 (d, J = 6.0 Hz, 6H). ESI-MS calculated for: [M+H]⁺ = 259.17, found 259.0.

### Example 14

### Synthetic route:

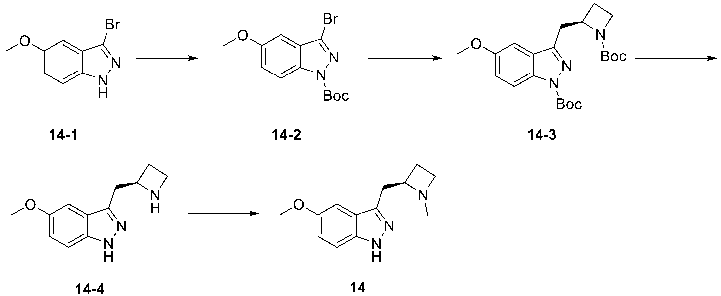

### Step 1

Compound **14-1** (700 mg, 3.08 mmol) was dissolved in dichloromethane (10 mL), and then triethylamine (620 mg, 6.16 mmol), di-tert-butyl dicarbonate (810 mg, 3.70 mmol), and 4-dimethylaminopyridine (38.0 mg, 0.31 mmol) were added. The mixture was stirred at 25°C for 4 hours. Saturated aqueous ammonium chloride solution (20 mL) was added to the reaction mixture, and the mixture was extracted with dichloromethane (20 mL × 3). The organic phases were combined and washed with saturated brine (30 mL × 2). The organic phase was dried over anhydrous sodium sulfate, filtered, and concentrated under reduced pressure. The crude product was purified by silica gel column chromatography (petroleum ether/ethyl acetate, 10/1, v/v) to obtain compound **14-2.** ¹H NMR (400 MHz, DMSO-d₆): δ 8.00 (d, J = 9.2 Hz, 1H), 7.33 (dd, J = 9.2, 2.4 Hz, 1H), 7.06 (d, J = 2.4 Hz, 1H), 3.88 (s, 3H), 1.64 (s, 9H).

### Step 2

In a glove box filled with nitrogen, compound **14-2** (196 mg, 0.60 mmol), *(2R)-tert-*butyl 2-(bromomethyl)azetidine-1-carboxylate (100 mg, 0.40 mmol), anhydrous sodium carbonate (84.8 mg, 0.80 mmol), tris(trimethylsilyl)silane (99.5 mg, 0.40 mmol), nickel(II) chloride dimethoxyethane (4.4 mg, 0.02 mmol), 4,4'-di-tert-butyl-2,2'-dipyridine (5.4 mg, 0.02 mmol), and bis[2-(2,4-difluorophenyl)-5-trifluoromethylpyridine][2-2'-bi(4-tert-butylpyridine)]iridium(III) bis(hexafluorophosphate) (4.5 mg, 0.004 mmol) were dissolved in ethylene glycol dimethyl ether (6 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with dichloromethane (20 mL × 3). The organic phases were combined and washed with saturated brine (30 mL × 2). The organic phase was dried over anhydrous sodium sulfate, filtered, and concentrated under reduced pressure. The crude product was purified by silica gel column chromatography (petroleum ether/ethyl acetate, 2/1, v/v) to obtain compound **14-3.** ESI-MS calculated for: [M+H]⁺ = 418.23, found 418.0.

### Step 3

Compound **14-3** (200 mg, 0.48 mmol) was dissolved in 1,2-dichloromethane (5 mL). Trifluoroacetic acid (1.5 mL) was added to the reaction mixture, and the reaction mixture was stirred at 25°C for 0.5 hours. After completion of the reaction, the mixture was concentrated under reduced pressure to obtain compound **14-4.** ESI-MS calculated for: [M+H]⁺ = 218.12, found 218.0.

### Step 4

Compound **14-4** (104 mg, 0.48 mmol) was dissolved in methanol (5 mL). Triethylamine (150 mg, 1.44 mmol) and paraformaldehyde (42.9 mg, 0.53 mmol) were added to the reaction mixture, and the reaction mixture was stirred at 25°C for 0.5 hours. Sodium cyanoborohydride (18.1 mg, 0.29 mmol) was added, and the reaction mixture was stirred at 25°C for 2 hours. Saturated aqueous sodium bicarbonate solution (5 mL) was added to the reaction mixture, and the mixture was extracted with dichloromethane (10 mL × 3). The organic phases were combined, dried over anhydrous sodium sulfate, filtered, and concentrated under reduced pressure. The crude product was purified by high-performance liquid chromatography (Waters-Agilent-C18-10 µm-19*250 mm, mobile phase: acetonitrile-0.1% formic acid in water, gradient: 7-17%, retention time: 9.5-10.1 min, run time: 16 min) to obtain the monoformate salt of compound **14.** ¹H NMR (400 MHz, DMSO-d₆): δ 12.81 (s, 1H), 9.81 (s, 1H), 7.42 (d, J = 8.8 Hz, 1H), 7.22 (s, 1H), 7.03 (dd, J = 8.8, 2.0 Hz, 1H), 4.73-4.64 (m, 1H), 4.08-3.99 (m, 1H), 3.87-3.76 (m, 4H), 3.57-3.52 (m, 1H), 3.45-3.38 (m, 1H), 2.76 (s, 3H), 2.46-2.41 (m, 1H), 2.35-2.28 (m, 1H). ESI-MS calculated for: [M+H]⁺ = 232.14, found 232.0.

### Example 15

### Synthetic route:

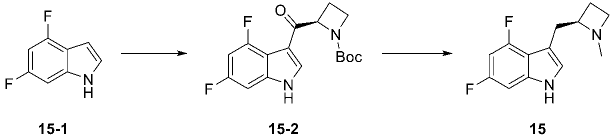

### Step 1

(*R*)-*N*-*tert*-Butoxycarbonyl-azetidine-2-carboxylic acid (5.28 g, 26.2 mmol) was dissolved in dichloromethane (100 mL). Oxalyl chloride (2.70 mL, 31.4 mmol) was added dropwise at 0°C, and the reaction mixture was stirred at 0°C for 10 minutes. *N,N-*Dimethylformamide (192 mg, 2.77 mmol) was slowly added, and the reaction mixture was stirred at 25°C for 1 hour. The reaction mixture was concentrated under reduced pressure to obtain a crude intermediate. Compound **15-1** (3.10 g, 20.2 mmol) was dissolved in dichloromethane (30 mL). Under a nitrogen atmosphere, ethylmagnesium bromide (10.6 mL, 21.3 mmol, 2 mol/L) was slowly added dropwise at 0°C, and the mixture was stirred at 0°C for 0.5 hours. The crude intermediate was dissolved in dichloromethane (50 mL) and added dropwise to the reaction mixture at 0°C. The reaction mixture was stirred at 0°C for 0.5 hours. Saturated aqueous citric acid solution (200 mL) was added to the reaction mixture, and the mixture was extracted with dichloromethane (200 mL × 3). The organic phases were combined and washed with saturated brine (200 mL × 2). The organic phase was dried over anhydrous sodium sulfate, filtered, and concentrated under reduced pressure. The crude product was purified by silica gel column chromatography (dichloromethane/ethyl acetate, 1/1, v/v) to obtain compound **15-2.** ESI-MS calculated for: [M+H]⁺ = 337.13, found: 336.9.

### Step 2

Compound **15-2** (690 mg, 2.05 mmol) was dissolved in tetrahydrofuran (20 mL). A solution of lithium aluminum hydride in tetrahydrofuran (8.20 mL, 20.5 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.8 mL), and then 15% aqueous sodium hydroxide solution (0.8 mL) and water (2.4 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 20-45%, retention time: 7.5-9.2 min, run time: 16 min) to obtain compound 15. ¹H NMR (400 MHz, DMSO-d₆): δ 11.16 (s, 1H), 7.11 (s, 1H), 6.97 (dd, J = 9.6, 2.0 Hz, 1H), 6.75-6.68 (m, 1H), 3.23-3.19 (m, 1H), 3.16-3.11 (m, 1H), 2.97-2.92 (m, 1H), 2.80-2.75 (m, 1H), 2.64-2.58 (m, 1H), 2.09 (s, 3H), 1.92-1.84 (m, 1H), 1.80-1.73 (m, 1H). ESI-MS calculated for: [M+H]⁺ = 237.11, found 237.0.

### Example 16

### Synthetic route:

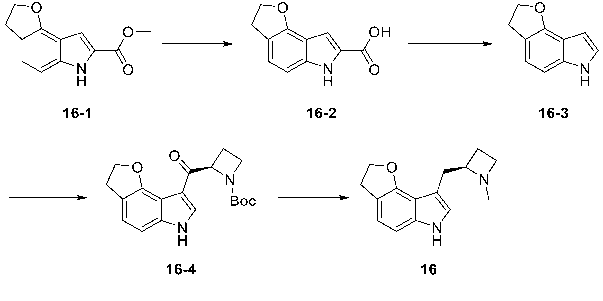

### Step 1

Compound **16-1** (4.18 g, 19.2 mmol) was dissolved in tetrahydrofuran (38 mL) and water (38 mL). Lithium hydroxide monohydrate (2.42 g, 57.72 mmol) was added, and the reaction mixture was stirred at 25°C for 16 hours. The reaction mixture was adjusted to pH 4 with 1 mol/L aqueous hydrochloric acid solution and extracted with ethyl acetate (100 mL × 4). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound 16-2. ¹H NMR (400 MHz, DMSO-d₆): δ 12.89 (s, 1H), 11.71 (s, 1H), 7.11 (d, J = 8.4 Hz, 1H), 6.92 (dd, J = 8.4, 0.8 Hz, 1H), 6.89 (d, J = 2.0 Hz, 1H), 4.64 (t, J = 8.8 Hz, 2H), 3.21 (t, J = 8.8 Hz, 2H). ESI-MS calculated for: [M+H]⁺ = 204.06, found: 204.0.

### Step 2

Compound **16-2** (4.00 g, 19.7 mmol) was dissolved in quinoline (32 mL). Copper(II) oxide (470 mg, 5.91 mmol) was added, and the reaction mixture was stirred at 200°C for 2 hours. The reaction mixture was cooled to room temperature, adjusted to pH 4 with 1 mol/L aqueous hydrochloric acid solution, and extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **16-3.** ¹H NMR (400 MHz, DMSO-d₆): δ 11.02 (s, 1H), 7.21 (s, 1H), 6.94 (d, J = 8.0 Hz, 1H), 6.88 (d, J = 8.0 Hz, 1H), 6.26 (s, 1H), 4.59 (t, J = 8.8 Hz, 2H), 3.20 (t, J = 8.8 Hz, 2H). ESI-MS calculated for: [M+H]⁺ = 160.07, found: 160.0.

### Step 3

(*R*)-N-*tert*-Butoxycarbonyl-azetidine-2-carboxylic acid (2.64 g, 13.1 mmol) was dissolved in dichloromethane (50 mL). Oxalyl chloride (1.35 mL, 15.7 mmol) was added dropwise at 0°C, and the reaction mixture was stirred at 0°C for 10 minutes. *N,N-*Dimethylformamide (96.0 mg, 1.39 mmol) was slowly added, and the reaction mixture was stirred at 25°C for 1 hour. The reaction mixture was concentrated under reduced pressure to obtain a crude intermediate. Compound **16-3** (1.50 g, 9.42 mmol) was dissolved in dichloromethane (15 mL). Under a nitrogen atmosphere, ethylmagnesium bromide (4.95 mL, 9.89 mmol, 2 mol/L) was slowly added dropwise at 0°C, and the mixture was stirred at 0°C for 0.5 hours. The crude intermediate was dissolved in dichloromethane (50 mL) and added dropwise to the reaction mixture at 0°C. The reaction mixture was stirred at 0°C for 0.5 hours. Saturated aqueous citric acid solution (100 mL) was added to the reaction mixture, and the mixture was extracted with dichloromethane (50 mL × 3). The organic phases were combined and washed with saturated brine (100 mL). The organic phase was dried over anhydrous sodium sulfate, filtered, and concentrated under reduced pressure. The crude product was purified by silica gel column chromatography (dichloromethane/ethyl acetate, 1/1, v/v) to obtain compound **16-4.** ESI-MS calculated for: [M+H]⁺ = 343.16, found: 343.2.

### Step 4

Compound **16-4** (165 mg, 0.48 mmol) was dissolved in tetrahydrofuran (4 mL). A solution of lithium aluminum hydride in tetrahydrofuran (2.88 mL, 7.20 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.28 mL), and then 15% aqueous sodium hydroxide solution (0.28 mL) and water (0.84 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-0.1% formic acid in water, gradient: 10-20%, retention time: 7.1-10.0 min, run time: 17 min) to obtain the monoformate salt of compound **16.** ¹H NMR (400 MHz, DMSO-*d*₆): δ 10.75 (s, 1H), 8.30 (s, 1H), 6.99 (s, 1H), 6.90 (d, J = 8.0 Hz, 1H), 6.80 (d, J = 8.0 Hz, 1H), 4.61 (t, J = 8.8 Hz, 2H), 3.60-3.56 (m, 1H), 3.47-3.43 (m, 1H), 3.17 (t, J = 8.8 Hz, 2H), 3.09-3.03 (m, 1H), 2.97-2.85 (m, 2H), 2.24 (s, 3H), 2.00-1.92 (m, 2H). ESI-MS calculated for: [M+H]⁺ = 243.14, found 243.0.

### Example 17

### Synthetic route:

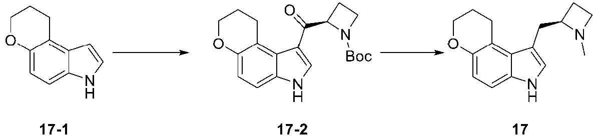

### Step 1

(*R*)-*N*-*tert-*Butoxycarbonyl-azetidine-2-carboxylic acid (1.32 g, 6.55 mmol) was dissolved in dichloromethane (20 mL). Oxalyl chloride (0.675 mL, 7.85 mmol) was added dropwise at 0°C, and the reaction mixture was stirred at 0°C for 10 minutes. *N,N-*Dimethylformamide (48.0 mg, 0.695 mmol) was slowly added, and the reaction mixture was stirred at 25°C for 1 hour. The reaction mixture was concentrated under reduced pressure to obtain a crude intermediate. Compound **17-1** (900 mg, 5.20 mmol) was dissolved in dichloromethane (15 mL). Under a nitrogen atmosphere, ethylmagnesium bromide (2.73 mL, 5.46 mmol, 2 mol/L) was slowly added dropwise at 0°C, and the mixture was stirred at 0°C for 0.5 hours. The crude intermediate was dissolved in dichloromethane (20 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (dichloromethane/ethyl acetate, 1/1, v/v) to obtain compound **17-2.** ESI-MS calculated for: [M+H]⁺ = 357.17, found: 356.9.

### Step 2

Compound **17-2** (320 mg, 0.90 mmol) was dissolved in tetrahydrofuran (10 mL). A solution of lithium aluminum hydride in tetrahydrofuran (3.60 mL, 9.00 mmol, 2.5 mol/L) was added dropwise at 0°C. Under a nitrogen atmosphere, the reaction mixture was stirred at 60°C for 16 hours. The reaction mixture was cooled to room temperature, quenched by the addition of ice water (0.36 mL), and then 15% aqueous sodium hydroxide solution (0.36 mL) and water (1.08 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 15-45%, retention time: 8.8-10.2 min, run time: 16 min) to obtain compound 17. ¹H NMR (400 MHz, DMSO-d₆): δ 10.56 (s, 1H), 7.00 (d, J = 8.4 Hz, 1H), 6.94 (d, J = 2.4 Hz, 1H), 6.48 (d, J = 8.4 Hz, 1H), 4.07-4.04 (m, 2H), 3.26-3.23 (m, 1H), 3.18-3.04 (m, 4H), 2.86-2.78 (m, 1H), 2.64-2.57 (m, 1H), 2.12 (s, 3H), 2.00-1.94 (m, 3H), 1.79-1.75 (m, 1H). ESI-MS calculated for: [M+H]⁺ = 257.16, found 257.1.

### Example 18

### Synthetic route:

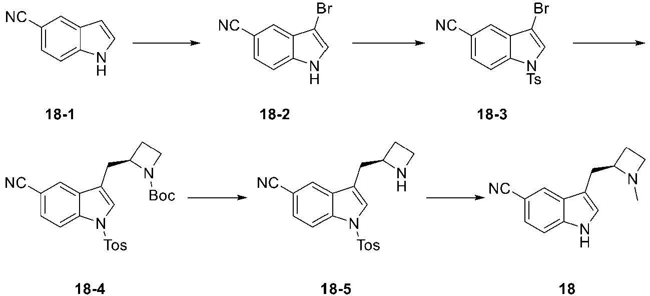

### Step 1

Compound **18-1** (4.00 g, 28.1 mmol) was dissolved in N,N-dimethylformamide (80 mL). Under a nitrogen atmosphere, N-bromosuccinimide (5.01 g, 28.1 mmol) was slowly added at 0°C, and the mixture was stirred at 25°C for 16 hours. Water (100 mL) was added to the reaction mixture, and the mixture was extracted with ethyl acetate (200 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **18-2.** ¹H NMR (400 MHz, DMSO-*d*₆): δ 12.04 (s, 1H), 7.91 (s, 1H), 7.79 (s, 1H), 7.61 (d, J = 8.4 Hz, 1H), 7.53 (d, J = 8.4 Hz, 1H).

### Step 2

Compound **18-2** (4.70 g, 21.3 mmol) was dissolved in N,N-dimethylformamide (90 mL). Under a nitrogen atmosphere, sodium hydride (1.02 g, 25.5 mmol, 60% purity) was slowly added at 0°C, and the mixture was stirred at 0°C for 0.5 hours. p-Toluenesulfonyl chloride (4.46 g, 23.4 mmol) was added, and the mixture was stirred at 25°C for 12 hours. Saturated aqueous ammonium chloride solution (100 mL) was added to the reaction mixture, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 4/1, v/v) to obtain compound **18-3.** ¹H NMR (400 MHz, DMSO-*d*₆): δ 8.38 (s, 1H), 8.16 (d, J = 8.4 Hz, 1H), 8.04 (d, J = 1.2 Hz, 1H), 7.98 (d, J = 8.4 Hz, 2H), 7.85 (dd, J = 8.4, 1.2 Hz, 1H), 7.43 (d, J = 8.4 Hz, 2H), 2.33 (s, 3H).

### Step 3

In a glove box filled with nitrogen, compound **18-3** (200 mg, 0.53 mmol), *(2R)-tert-*butyl 2-(bromomethyl)azetidine-1-carboxylate (199 mg, 0.80 mmol), anhydrous sodium carbonate (110 mg, 1.06 mmol), tris(trimethylsilyl)silane (197 mg, 0.80 mmol), nickel(II) chloride dimethoxyethane (5.8 mg, 0.027 mmol), 4,4'-di-tert-butyl-2,2'-dipyridine (7.1 mg, 0.027 mmol), and bis[2-(2,4-difluorophenyl)-5-trifluoromethylpyridine][2-2'-bi(4-tert-butylpyridine)]iridium(III) bis(hexafluorophosphate) (6.0 mg, 0.0053 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with dichloromethane (20 mL × 3). The organic phases were combined and washed with saturated brine (20 mL × 2). The organic phase was dried over anhydrous sodium sulfate, filtered, and concentrated under reduced pressure. The crude product was purified by silica gel column chromatography (petroleum ether/ethyl acetate, 2/1, v/v) to obtain compound **18-4.** ESI-MS calculated for: [M+H-56]⁺ = 410.17, found 410.0.

### Step 4

Compound **18-4** (160 mg, 0.34 mmol) was dissolved in 1,2-dichloromethane (5 mL). Trifluoroacetic acid (1.0 mL) was added to the reaction mixture, and the reaction mixture was stirred at 25°C for 1 hour. After completion of the reaction, the mixture was concentrated under reduced pressure to obtain compound **18-5.** ESI-MS calculated for: [M+H]⁺ = 366.12, found 366.4.

### Step 5

Compound **18-5** (125 mg, 0.34 mmol) was dissolved in methanol (5 mL). Diisopropylethylamine (132 mg, 1.02 mmol) and paraformaldehyde (55.2 mg, 0.68 mmol) were added to the reaction mixture, and the reaction mixture was stirred at 25°C for 0.5 hours. Sodium cyanoborohydride (32.1 mg, 0.51 mmol) was added, and the reaction mixture was stirred at 25°C for 2 hours. Saturated aqueous sodium bicarbonate solution (5 mL) was added to the reaction mixture, and the mixture was extracted with ethyl acetate (10 mL × 3). The organic phases were combined, dried over anhydrous sodium sulfate, filtered, and concentrated under reduced pressure. The crude product was dissolved in tetrahydrofuran (0.4 mL), ethanol (2.0 mL), and water (2.0 mL). Lithium hydroxide monohydrate (54.0 mg, 1.28 mmol) was added, and the reaction mixture was stirred at 70°C for 2 hours. The reaction mixture was cooled to room temperature and concentrated under reduced pressure. The crude product was purified by high-performance liquid chromatography (Waters-Agilent-C18-10 µm-19*250 mm, mobile phase: acetonitrile-10 mmol/L aqueous ammonium bicarbonate solution, gradient: 17-33%, retention time: 9.0-10.7 min, run time: 16 min) to obtain compound **18.** ¹H NMR (400 MHz, DMSO-d₆): δ 11.40 (s, 1H), 8.09 (s, 1H), 7.49 (d, J = 8.4 Hz, 1H), 7.39 (dd, J = 8.4, 1.2 Hz, 1H), 7.33 (s, 1H), 3.24-3.20 (m, 1H), 3.13-3.07 (m, 1H), 2.96-2.91 (m, 1H), 2.81-2.76 (m, 1H), 2.63-2.59 (m, 1H), 2.09 (s, 3H), 1.94-1.87 (m, 1H), 1.83-1.74 (m, 1H). ESI-MS calculated for: [M+H]⁺ = 226.13, found 226.0.

### Example 19

### Synthetic route:

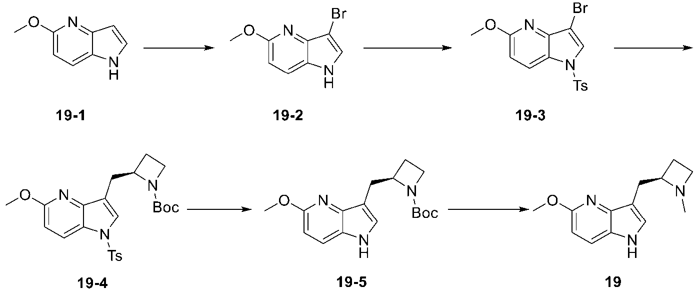

### Step 1

Compound **19-1** (7.01 g, 47.3 mmol) was dissolved in N,N-dimethylformamide (140 mL). Under a nitrogen atmosphere, N-bromosuccinimide (8.84 g, 49.7 mmol) was slowly added at 0°C, and the mixture was stirred at 25°C for 2 hours. Water (200 mL) was added to the reaction mixture, and the mixture was extracted with ethyl acetate (300 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **19-2.** ¹H NMR (400 MHz, DMSO-d₆): δ 11.53 (s, 1H), 7.74 (d, J = 8.8 Hz, 1H), 7.66 (s, 1H), 6.63 (d, J = 8.8 Hz, 1H), 3.89 (s, 3H).

### Step 2

Compound **19-2** (4.30 g, 18.9 mmol) was dissolved in N,N-dimethylformamide (40 mL). Under a nitrogen atmosphere, sodium hydride (909 mg, 22.7 mmol, 60% purity) was slowly added at 0°C, and the mixture was stirred at 0°C for 0.5 hours. p-Toluenesulfonyl chloride (4.33 g, 22.7 mmol) was added, and the mixture was stirred at 25°C for 0.5 hours. Saturated aqueous ammonium chloride solution (100 mL) was added to the reaction mixture, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 4/1, v/v) to obtain compound **19-3.** ¹H NMR (400 MHz, DMSO-d₆): δ 8.32 (s, 1H), 8.28 (d, J = 8.8 Hz, 1H), 7.92 (d, J = 8.4 Hz, 2H), 7.42 (d, J = 8.4 Hz, 2H), 6.88 (d, J = 8.8 Hz, 1H), 3.89 (s, 3H), 2.34 (s, 3H).

### Step 3

In a glove box filled with nitrogen, compound **19-3** (200 mg, 0.52 mmol), *(2R)-tert-*butyl 2-(bromomethyl)azetidine-1-carboxylate (195 mg, 0.78 mmol), anhydrous sodium carbonate (110 mg, 1.04 mmol), tris(trimethylsilyl)silane (194 mg, 0.78 mmol), nickel(II) chloride dimethoxyethane (5.7 mg, 0.026 mmol), 4,4'-di-tert-butyl-2,2'-dipyridine (7.0 mg, 0.026 mmol), and bis[2-(2,4-difluorophenyl)-5-trifluoromethylpyridine][2-2'-bi(4-tert-butylpyridine)]iridium(III) bis(hexafluorophosphate) (5.8 mg, 0.0052 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with dichloromethane (20 mL × 3). The organic phases were combined and washed with saturated brine (20 mL × 2). The organic phase was dried over anhydrous sodium sulfate, filtered, and concentrated under reduced pressure. The crude product was purified by silica gel column chromatography (petroleum ether/ethyl acetate, 2/1, v/v) to obtain compound **19-4.** ESI-MS calculated for: [M+H]⁺ = 472.18, found 472.0.

### Step 4

Compound **19-4** (230 mg, 0.49 mmol) was dissolved in anhydrous tetrahydrofuran (5 mL), anhydrous ethanol (5 mL), and water (2 mL). Sodium hydroxide (58.8 mg, 1.47 mmol) was added, and the reaction mixture was stirred at 60°C for 4 hours. Water (20 mL) was added to the reaction mixture, and the mixture was extracted with dichloromethane (20 mL × 3). The organic phases were combined and washed with saturated brine (20 mL). The organic phase was dried over anhydrous sodium sulfate, filtered, and concentrated under reduced pressure. The crude product was purified by silica gel column chromatography (dichloromethane/methanol, 10/1, v/v) to obtain compound **19-5.** ESI-MS calculated for: [M+H]⁺ = 318.17, found 318.5.

### Step 5

Compound **19-5** (70.0 mg, 0.22 mmol) was dissolved in tetrahydrofuran (3 mL). A solution of lithium aluminum hydride in tetrahydrofuran (0.44 mL, 1.10 mmol, 2.5 mol/L) was added dropwise at 0°C. Under a nitrogen atmosphere, the reaction mixture was stirred at 60°C for 2 hours. The reaction mixture was cooled to room temperature, quenched by the addition of ice water (0.05 mL), and then 15% aqueous sodium hydroxide solution (0.05 mL) and water (0.15 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 15-43%, retention time: 6.9-8.5 min, run time: 16 min) to obtain compound 19. ¹H NMR (400 MHz, DMSO-d₆): δ 10.81 (s, 1H), 7.61 (d, J = 8.4 Hz, 1H), 7.24 (d, J = 2.4 Hz, 1H), 6.49 (d, J = 8.4 Hz, 1H), 3.87 (s, 3H), 3.24-3.20 (m, 2H), 2.92-2.85 (m, 1H), 2.80-2.73 (m, 1H), 2.61-2.56 (m, 1H), 2.14 (s, 3H), 1.92-1.79 (m, 2H). ESI-MS calculated for: [M+H]⁺ = 232.14, found 232.2.

### Example 20

### Synthetic route:

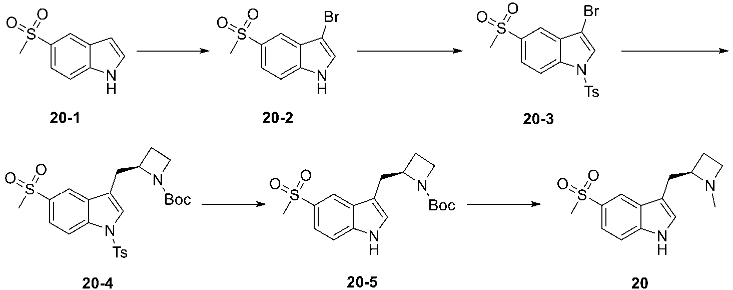

### Step 1

Compound **20-1** (2.00 g, 10.2 mmol) was dissolved in N,N-dimethylformamide (20 mL). Under a nitrogen atmosphere, N-bromosuccinimide (1.91 g, 10.8 mmol) was slowly added at 0°C, and the mixture was stirred at 25°C for 2 hours. Water (50 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 1/1, v/v) to obtain compound **20-2.** ¹H NMR (400 MHz, DMSO-d₆): δ 7.98 (s, 1H), 7.82 (s, 1H), 7.74-7.64 (m, 2H), 3.20 (s, 3H).

### Step 2

Compound **20-2** (2.40 g, 8.75 mmol) was dissolved in N,N-dimethylformamide (40 mL). Under a nitrogen atmosphere, sodium hydride (420 mg, 10.5 mmol, 60% purity) was slowly added at 0°C, and the mixture was stirred at 0°C for 0.5 hours. p-Toluenesulfonyl chloride (1.84 g, 9.63 mmol) was added, and the mixture was stirred at 25°C for 1 hour. Saturated aqueous ammonium chloride solution (100 mL) was added to the reaction mixture, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 4/1, v/v) to obtain compound **20-3.** ¹H NMR (400 MHz, CDCl₃): δ 8.16 (d, J = 8.8 Hz, 2H), 7.92 (dd, J = 8.8, 1.6 Hz, 1H), 7.82-7.75 (m, 3H), 7.29 (d, J = 8.8 Hz, 2H), 3.08 (s, 3H), 2.38 (s, 3H).

### Step 3

In a glove box filled with nitrogen, compound **20-3** (200 mg, 0.47 mmol), *(2R)-tert-*butyl 2-(bromomethyl)azetidine-1-carboxylate (175 mg, 0.70 mmol), anhydrous sodium carbonate (99.6 mg, 0.94 mmol), tris(trimethylsilyl)silane (175 mg, 0.70 mmol), nickel(II) chloride dimethoxyethane (5.2 mg, 0.024 mmol), 4,4'-di-tert-butyl-2,2'-dipyridine (6.3 mg, 0.024 mmol), and bis[2-(2,4-difluorophenyl)-5-trifluoromethylpyridine][2-2'-bi(4-tert-butylpyridine)]iridium(III) bis(hexafluorophosphate) (5.2 mg, 0.0047 mmol) were dissolved in ethylene glycol dimethyl ether (5 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with dichloromethane (20 mL × 3). The organic phases were combined and washed with saturated brine (20 mL × 2). The organic phase was dried over anhydrous sodium sulfate, filtered, and concentrated under reduced pressure. The crude product was purified by silica gel column chromatography (petroleum ether/ethyl acetate, 3/1, v/v) to obtain compound **20-4.** ESI-MS calculated for: [M+H-56]⁺ = 463.15, found 463.1.

### Step 4

Compound **20-4** (350 mg, 0.67 mmol) was dissolved in anhydrous tetrahydrofuran (1.5 mL), anhydrous ethanol (1.5 mL), and water (0.6 mL). Lithium hydroxide monohydrate (141 mg, 3.35 mmol) was added, and the reaction mixture was stirred at 60°C for 3 hours. Water (20 mL) was added to the reaction mixture, and the mixture was extracted with dichloromethane (20 mL × 3). The organic phases were combined and washed with saturated brine (20 mL). The organic phase was dried over anhydrous sodium sulfate, filtered, and concentrated under reduced pressure. The crude product was purified by silica gel column chromatography (petroleum ether/ethyl acetate, 1/1, v/v) to obtain compound **20-5.** ESI-MS calculated for: [M+H-56]⁺ = 309.15, found 309.0.

### Step 5

Compound **20-5** (70.0 mg, 0.22 mmol) was dissolved in tetrahydrofuran (3 mL). A solution of lithium aluminum hydride in tetrahydrofuran (0.44 mL, 1.10 mmol, 2.5 mol/L) was added dropwise at 0°C. Under a nitrogen atmosphere, the reaction mixture was stirred at 60°C for 2 hours. The reaction mixture was cooled to room temperature, quenched by the addition of ice water (0.05 mL), and then 15% aqueous sodium hydroxide solution (0.05 mL) and water (0.15 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 20-25%, retention time: 6.0-7.0 min, run time: 16 min) to obtain compound **20.** ¹H NMR (400 MHz, DMSO-d₆): δ 11.40 (s, 1H), 8.13 (d, J = 1.2 Hz, 1H), 7.59-7.52 (m, 2H), 7.36 (s, 1H), 3.27-3.22 (m, 1H), 3.17-3.11 (m, 4H), 2.99-2.94 (m, 1H), 2.86-2.79 (m, 1H), 2.64-2.58 (m, 1H), 2.09 (s, 3H), 1.96-1.91 (m, 1H), 1.81-1.76 (m, 1H). ESI-MS calculated for: [M+H]⁺ = 279.11, found 279.0.

### Example 21

### Synthetic route:

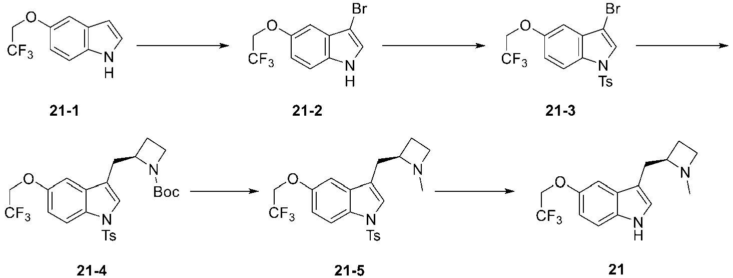

### Step 1

Compound **21-1** (1.50 g, 6.97 mmol) was dissolved in N,N-dimethylformamide (15 mL). Under a nitrogen atmosphere, N-bromosuccinimide (1.24 g, 6.97 mmol) was slowly added at 0°C, and the mixture was stirred at 25°C for 2 hours. Water (20 mL) was added to the reaction mixture, and the mixture was extracted with ethyl acetate (50 mL × 3). The organic phases were combined and washed with saturated brine (30 mL). The organic phase was dried over anhydrous sodium sulfate, filtered, and concentrated under reduced pressure. The crude product was purified by silica gel column chromatography (petroleum ether/ethyl acetate, 1/1, v/v) to obtain compound **21-2.** ESI-MS calculated for: [M+H]⁺ = 293.97 and 295.97, found 294.0 and 296.0.

### Step 2

Compound **21-2** (2.29 g, 6.62 mmol) was dissolved in N,N-dimethylformamide (20 mL). Under a nitrogen atmosphere, sodium hydride (320 mg, 7.94 mmol, 60% purity) was slowly added at 0°C, and the mixture was stirred at 0°C for 0.5 hours. p-Toluenesulfonyl chloride (1.51 g, 7.94 mmol) was added, and the mixture was stirred at 25°C for 3 hours. Saturated aqueous ammonium chloride solution (20 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **21-3.** ¹H NMR (400 MHz, DMSO-d₆): δ 8.13 (s, 1H), 7.93-7.88 (m, 3H), 7.40 (d, J = 8.0 Hz, 2H), 7.15 (dd, J = 8.8, 2.4 Hz, 1H), 7.08 (d, J = 2.4 Hz, 1H), 4.83 (q, J = 8.8 Hz, 2H), 2.32 (s, 3H).

### Step 3

In a glove box filled with nitrogen, compound **21-3** (350 mg, 0.78 mmol), *(2R)-tert-*butyl 2-(bromomethyl)azetidine-1-carboxylate (292 mg, 1.17 mmol), anhydrous sodium carbonate (166 mg, 1.56 mmol), tris(trimethylsilyl)silane (290 mg, 1.56 mmol), nickel(II) chloride dimethoxyethane (8.6 mg, 0.04 mmol), 4,4'-di-tert-butyl-2,2'-dipyridine (10.4 mg, 0.04 mmol), and bis[2-(2,4-difluorophenyl)-5-trifluoromethylpyridine][2-2'-bi(4-tert-butylpyridine)]iridium(III) bis(hexafluorophosphate) (8.8 mg, 0.0078 mmol) were dissolved in ethylene glycol dimethyl ether (10 mL). The reaction mixture was placed under irradiation with a 34 W blue LED (420 nm) and stirred at 25°C for 16 hours. After completion of the reaction, the blue light was turned off. Water (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (petroleum ether/ethyl acetate, 2/1, v/v) to obtain compound **21-4.** ESI-MS calculated for: [M+H]⁺ = 539.17, found 539.3.

### Step 4

Compound **21-4** (450 mg, 0.84 mmol) was dissolved in 1,2-dichloromethane (10 mL). Trifluoroacetic acid (4 mL) was added to the reaction mixture, and the reaction mixture was stirred at 25°C for 1 hour. After completion of the reaction, the mixture was concentrated under reduced pressure, and the residue was dissolved in 1,2-dichloroethane (10 mL). Triethylamine (249 mg, 2.46 mmol) and 37% aqueous formaldehyde solution (200 mg, 2.46 mmol) were added to the reaction mixture, and the reaction mixture was stirred at 25°C for 0.5 hours. Sodium triacetoxyborohydride (348 mg, 1.64 mmol) was added, and the reaction mixture was stirred at 25°C for 2 hours. Saturated aqueous sodium bicarbonate solution (30 mL) was added to the reaction mixture, and the mixture was extracted with dichloromethane (30 mL × 3). The organic phases were combined, dried over anhydrous sodium sulfate, filtered, and concentrated under reduced pressure. The crude product was purified by silica gel column chromatography (dichloromethane/methanol, 20/1, v/v) to obtain compound **21-5.** ESI-MS calculated for: [M+H]⁺ = 453.14, found 453.5.

### Step 5

Compound **21-5** (310 mg, 0.69 mmol) was dissolved in anhydrous tetrahydrofuran (3 mL), anhydrous ethanol (3 mL), and water (3 mL). Sodium hydroxide (138 mg, 3.45 mmol) was added, and the reaction mixture was stirred at 60°C for 4 hours. The reaction mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 45-55%, retention time: 5.5-6.8 min, run time: 16 min) to obtain compound 21. ¹H NMR (400 MHz, DMSO-*d*₆): δ 10.72 (s, 1H), 7.25 (d, J = 8.8 Hz, 1H), 7.16 (d, J = 2.4 Hz, 1H), 7.10 (d, J = 2.4 Hz, 1H), 6.80 (dd, J = 8.8, 2.4 Hz, 1H), 4.69 (q, J = 8.8 Hz, 2H), 3.24-3.20 (m, 1H), 3.15-3.08 (m, 1H), 2.91-2.83 (m, 1H), 2.76-2.69 (m, 1H), 2.62 -2.56 (m, 1H), 2.11 (s, 3H), 1.94-1.89 (m, 1H), 1.80-1.74 (m, 1H). ESI-MS calculated for: [M+H]⁺ = 299.13, found 299.1.

### Example 22

### Synthetic route:

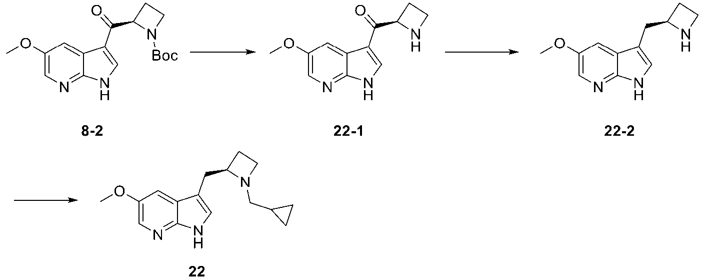

### Step 1

Compound **8-2** (2.90 g, 8.75 mmol) was dissolved in 1,2-dichloromethane (40 mL). Trifluoroacetic acid (10 mL) was added to the reaction mixture, and the reaction mixture was stirred at 25°C for 1 hour. After completion of the reaction, the mixture was concentrated under reduced pressure. The crude product was purified by high-performance liquid chromatography (Waters-spherical-C18-20 µm, 100A, 330 g, mobile phase: acetonitrile-10 mmol/L aqueous ammonium bicarbonate solution, gradient: 7-11%, retention time: 15-25 min, run time: 40 min) to obtain compound **22-1.** ¹H NMR (400 MHz, DMSO-*d*₆): δ 8.44 (s, 1H), 8.15 (d, J = 2.8 Hz, 1H), 7.99 (d, J = 2.8 Hz, 1H), 5.71-5.64 (m, 1H), 4.03-3.96 (m, 1H), 3.88 (s, 3H), 3.80-3.74 (m, 1H), 2.94-2.90 (m, 1H), 2.47-2.42 (m, 1H). ESI-MS calculated for: [M+H]⁺ = 232.10, found 232.0.

### Step 2

Compound **22-1** (2.20 g, 9.51 mmol) was dissolved in tetrahydrofuran (30 mL). A solution of lithium aluminum hydride in tetrahydrofuran (38.0 mL, 95.1 mmol, 2.5 mol/L) was added dropwise at 0°C. Under a nitrogen atmosphere, the reaction mixture was stirred at 60°C for 12 hours. The reaction mixture was cooled to room temperature, quenched by the addition of ice water (3.8 mL), and then 15% aqueous sodium hydroxide solution (3.8 mL) and water (11.4 mL) were added. The mixture was filtered, and the filtrate was concentrated under reduced pressure to obtain a crude product containing the target compound. The crude product was purified by high-performance liquid chromatography (Waters-spherical-C18-20 µm, 100A, 220 g, mobile phase: acetonitrile-10 mmol/L aqueous ammonium bicarbonate solution, gradient: 11-23%, retention time: 10-26 min, run time: 40 min) to obtain compound 22-2. ESI-MS calculated for: [M+H]⁺ = 218.12, found 218.1.

### Step 3

Compound **22-2** (200 mg, 0.92 mmol) was dissolved in methanol (5 mL). Diisopropylethylamine (360 mg, 2.76 mmol) and cyclopropanecarbaldehyde (190 mg, 2.76 mmol) were added to the reaction mixture, and the reaction mixture was stirred at 25°C for 0.5 hours. Sodium cyanoborohydride (58.0 mg, 0.92 mmol) was added, and the reaction mixture was stirred at 25°C for 2 hours. Saturated aqueous sodium bicarbonate solution (30 mL) was added to the reaction mixture, and the mixture was extracted with dichloromethane (30 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 25-45%, retention time: 6.5-8.4 min, run time: 16 min) to obtain compound **22.** ¹H NMR (400 MHz, DMSO-*d*₆): δ 11.15 (s, 1H), 7.92 (d, J = 1.6 Hz, 1H), 7.50 (s, 1H), 7.17 (s, 1H), 3.82 (s, 3H), 3.22-3.18 (m, 2H), 2.96-2.93 (m, 1H), 2.78-2.73 (m, 1H), 2.66-2.63 (m, 1H), 2.31-2.27 (m, 1H), 2.06-2.03 (m, 1H), 1.94-1.86 (m, 1H), 1.83-1.74 (m, 1H), 0.78-0.67 (m, 1H), 0.40-0.36 (m, 2H), 0.05-0.01 (m, 2H). ESI-MS calculated for: [M+H]⁺ = 272.17, found 272.1.

### Example 23

### Synthetic route:

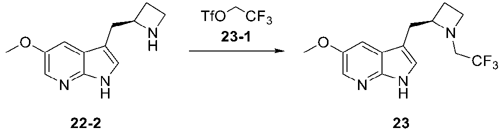

### Step 1

Compound **22-2** (110 mg, 0.51 mmol) was dissolved in tetrahydrofuran (2 mL). Diisopropylethylamine (200 mg, 1.53 mmol) and compound 23-1 (120 mg, 0.51 mmol) were added to the reaction mixture, and the reaction mixture was stirred at 60°C for 16 hours. Saturated aqueous sodium bicarbonate solution (5 mL) was added to the reaction mixture, and the mixture was extracted with dichloromethane (10 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 37-55%, retention time: 8.3-8.8 min, run time: 16 min) to obtain compound **23.** ¹H NMR (400 MHz, DMSO-*d*₆): δ 11.19 (s, 1H), 7.93 (d, J = 2.4 Hz, 1H), 7.57 (d, J = 2.4 Hz, 1H), 7.19 (d, J = 1.2 Hz, 1H), 3.82 (s, 3H), 3.56-3.50 (m, 1H), 3.40-3.38 (m, 1H), 3.24-3.17 (m, 1H), 3.09-2.88 (m, 3H), 2.80-2.75 (m, 1H), 2.01-1.94 (m, 1H), 1.93-1.85 (m, 1H). ESI-MS calculated for: [M+H]⁺ = 300.12, found 300.0.

### Example 24

### Synthetic route:

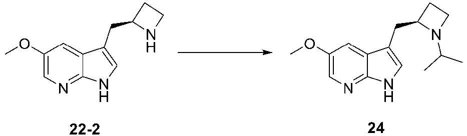

### Step 1

Compound **22-2** (200 mg, 0.92 mmol) was dissolved in acetonitrile (10 mL). Diisopropylethylamine (360 mg, 2.76 mmol) and 2-iodopropane (310 mg, 1.84 mmol) were added to the reaction mixture, and the reaction mixture was stirred at 70°C for 16 hours. Saturated aqueous sodium bicarbonate solution (5 mL) was added to the reaction mixture, and the mixture was extracted with dichloromethane (10 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 17-30%, retention time: 7.5-9.6 min, run time: 17 min) to obtain compound **24.** ¹H NMR (400 MHz, DMSO-*d*₆): δ 11.16 (s, 1H), 7.92 (d, J = 2.8 Hz, 1H), 7.48 (d, J = 2.8 Hz, 1H), 7.19 (s, 1H), 3.82 (s, 3H), 3.29-3.26 (m, 1H), 3.22-3.19 (m, 1H), 2.95-2.90 (m, 1H), 2.82-2.78 (m, 1H), 2.67-2.61 (m, 1H), 2.41-2.35 (m, 1H), 1.84-1.79 (m, 1H), 1.71-1.65 (m, 1H), 1.01 (d, J = 6.4 Hz, 3H), 0.84 (d, J = 6.4 Hz, 3H). ESI-MS calculated for: [M+H]⁺ = 260.17, found 260.1.

### Example 25

### Synthetic route:

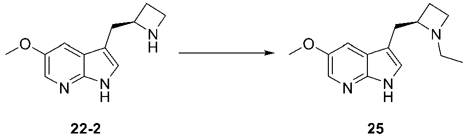

### Step 1

Compound **22-2** (200 mg, 0.92 mmol) was dissolved in methanol (5 mL). Triethylamine (280 mg, 2.76 mmol) and acetaldehyde (405 mg, 4.60 mmol) were added to the reaction mixture, and the reaction mixture was stirred at 25°C for 0.5 hours. Sodium cyanoborohydride (58.0 mg, 0.92 mmol) was added, and the reaction mixture was stirred at 25°C for 2 hours. Saturated aqueous sodium bicarbonate solution (30 mL) was added to the reaction mixture, and the mixture was extracted with dichloromethane (30 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 15-35%, retention time: 6.6-8.3 min, run time: 16 min) to obtain compound **25.** ¹H NMR (400 MHz, DMSO-*d*₆): δ 11.16 (s, 1H), 7.92 (d, J = 2.8 Hz, 1H), 7.51 (d, J = 2.8 Hz, 1H), 7.18 (d, J = 2.0 Hz, 1H), 3.82 (s, 3H), 3.25-3.17 (m, 2H), 2.91-2.87 (m, 1H), 2.78-2.74 (m, 1H), 2.56-2.53 (m, 1H), 2.48-2.45 (m, 1H), 2.19-2.14 (m, 1H), 1.93-1.89 (m, 1H), 1.84-1.75 (m, 1H), 0.85 (t, J = 7.2 Hz, 3H). ESI-MS calculated for: [M+H]⁺ = 246.15, found 246.0.

### Example 26

### Synthetic route:

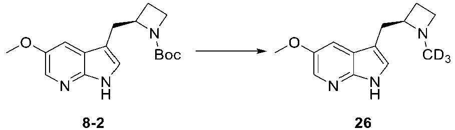

### Step 1

Compound **8-2** (142 mg, 0.45 mmol) was dissolved in tetrahydrofuran (6 mL). Lithium aluminum deuteride (189 mg, 4.50 mmol) was slowly added at 0°C. Under a nitrogen atmosphere, the reaction mixture was stirred at 60°C for 1 hour. The reaction mixture was cooled to room temperature, quenched by the addition of ice water (0.05 mL), and then 15% aqueous sodium hydroxide solution (0.05 mL) and water (0.1 mL) were added. The mixture was filtered, and the filtrate was concentrated under reduced pressure to obtain a crude product containing the target compound. The crude product was purified by high-performance liquid chromatography (Waters-Agilent-C18-10 µm-19*250 mm, mobile phase: acetonitrile-10 mmol/L aqueous ammonium bicarbonate solution, gradient: 10-30%, retention time: 7.8-9.8 min, run time: 16 min) to obtain compound **26.** ¹H NMR (400 MHz, DMSO-*d*₆): δ 11.16 (s, 1H), 7.92 (d, J = 2.8 Hz, 1H), 7.50 (d, J = 2.8 Hz, 1H), 7.18 (d, J = 2.0 Hz, 1H), 3.82 (s, 3H), 3.22-3.19 (m, 1H), 3.13-3.07 (m, 1H), 2.87-2.82 (m, 1H), 2.75-2.69 (m, 1H), 2.63-2.58 (m, 1H), 1.94-1.88 (m, 1H), 1.82-1.76 (m, 1H). ESI-MS calculated for: [M+H]⁺ = 235.16, found 235.1.

### Example 27

### Synthetic route:

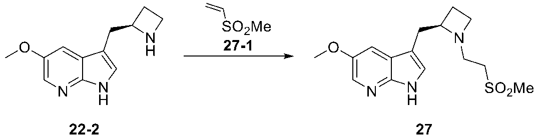

### Step 1

Compound **22-2** (100 mg, 0.46 mmol) was dissolved in ethanol (3 mL). Diisopropylethylamine (178 mg, 1.38 mmol) and compound **27-1** (146 mg, 1.38 mmol) were added to the reaction mixture, and the reaction mixture was stirred at 60°C for 2 hours. Saturated aqueous sodium bicarbonate solution (5 mL) was added to the reaction mixture, and the mixture was extracted with dichloromethane (10 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 23-33%, retention time: 6.5-7.3 min, run time: 16 min) to obtain compound 27. ¹H NMR (400 MHz, DMSO-*d*₆): δ 11.18 (s, 1H), 7.92 (d, J = 2.8 Hz, 1H), 7.53 (d, J = 2.8 Hz, 1H), 7.21 (d, J = 2.4 Hz, 1H), 3.82 (s, 3H), 3.32-3.29 (m, 2H), 3.14-3.02 (m, 2H), 3.01 (s, 3H), 2.94-2.87 (m, 2H), 2.77-2.66 (m, 2H), 2.57-2.53 (m, 1H), 1.97-1.90 (m, 1H), 1.82-1.74 (m, 1H). ESI-MS calculated for: [M+H]⁺ = 324.13, found 324.1.

### Example 28

### Synthetic route:

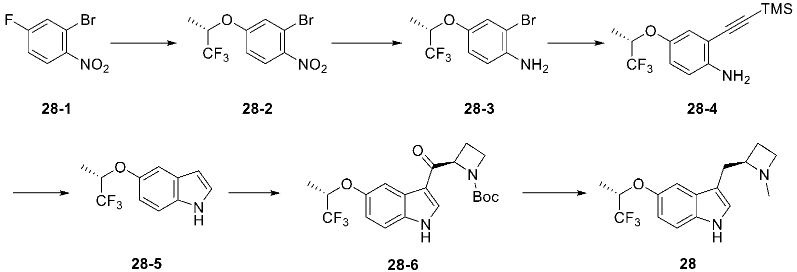

### Step 1

Compound (2S)-1,1,1-trifluoropropan-2-ol (7.41 g, 65.0 mmol) was dissolved in *N,N-*dimethylformamide (130 mL). Under a nitrogen atmosphere, sodium hydride (2.84 g, 70.9 mmol, 60% purity) was slowly added at 0°C, and the mixture was stirred at 0°C for 0.5 hours. Compound **28-1** (13.0 g, 59.1 mmol) was slowly added, and the mixture was stirred at 25°C for 0.5 hours. Saturated aqueous ammonium chloride solution (200 mL) was added to the reaction mixture, and the mixture was extracted with ethyl acetate (200 mL × 3). The organic phases were combined and washed with saturated brine (200 mL). The organic phase was dried over anhydrous sodium sulfate, filtered, and concentrated under reduced pressure. The crude product was purified by silica gel column chromatography (petroleum ether/ethyl acetate, 15/1, v/v) to obtain compound **28-2.** ¹H NMR (400 MHz, DMSO-*d*₆): δ 8.08 (dd, J = 8.8, 1.2 Hz, 1H), 7.68 (t, J = 2.8 Hz, 1H), 7.32 (dd, J = 8.8, 2.8 Hz, 1H), 5.58-5.51 (m, 1H), 1.45 (d, J = 6.4 Hz, 3H).

### Step 2

Compound **28-2** (7.80 g, 24.8 mmol) was dissolved in ethanol (66 mL) and water (13 mL). Iron powder (6.94 g, 124 mmol) and ammonium chloride (6.64 g, 124 mmol) were added, and the mixture was stirred at 80°C for 2 hours. The reaction mixture was cooled to room temperature and filtered. Water (150 mL) was added to the filtrate, and the mixture was extracted with ethyl acetate (150 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 15/1, v/v) to obtain compound **28-3.** ¹H NMR (400 MHz, DMSO-*d*₆): δ 7.14 (d, J = 2.8 Hz, 1H), 6.85 (dd, J = 8.8, 2.8 Hz, 1H), 6.75 (d, J = 8.8 Hz, 1H), 5.01 (s, 2H), 4.97-4.90 (m, 1H), 1.34 (d, J = 6.4 Hz, 3H).

### Step 3

Compound **28-3** (5.76 g, 20.3 mmol) was dissolved in triethylamine (50 mL). Under a nitrogen atmosphere, trimethylsilylacetylene (3.98 g, 40.6 mmol), bis(triphenylphosphine)palladium(II) dichloride (712 mg, 1.01 mmol), and copper(I) iodide (193 mg, 1.01 mmol) were added, and the mixture was stirred at 50°C for 16 hours. The reaction mixture was cooled to room temperature and filtered. Water (50 mL) was added to the filtrate, and the mixture was extracted with ethyl acetate (50 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 20/1, v/v) to obtain compound **28-4.** ESI-MS calculated for: [M+H]⁺ = 302.11, found 302.0.

### Step 4

Compound **28-4** (1.80 g, 5.97 mmol) was dissolved in N,N-dimethylformamide (27 mL). Under a nitrogen atmosphere, copper(I) iodide (2.27 g, 11.9 mmol) was added, and the mixture was stirred at 100°C for 2 hours. The reaction mixture was cooled to room temperature. Water (30 mL) was added, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 15/1, v/v) to obtain compound **28-5.** ¹H NMR (400 MHz, DMSO-*d*₆): δ 11.02 (s, 1H), 7.35-7.30 (m, 2H), 7.22 (d, J = 2.4 Hz, 1H), 6.82 (dd, J = 8.8, 2.4 Hz, 1H), 6.34 (dd, J = 7.2, 5.2 Hz, 1H), 5.04-4.97 (m, 1H), 1.40 (d, J = 6.4 Hz, 3H). ESI-MS calculated for: [M+H]⁺ = 230.07, found 230.0.

### Step 5

(*R*)-*N*-*tert*-Butoxycarbonyl-azetidine-2-carboxylic acid (1.32 g, 6.55 mmol) was dissolved in dichloromethane (20 mL). Oxalyl chloride (0.675 mL, 7.85 mmol) was added dropwise at 0°C, and the reaction mixture was stirred at 0°C for 10 minutes. *N,N-*Dimethylformamide (48.0 mg, 0.695 mmol) was slowly added, and the reaction mixture was stirred at 25°C for 1 hour. The reaction mixture was concentrated under reduced pressure to obtain a crude intermediate. Compound **28-5** (1.50 g, 6.54 mmol) was dissolved in dichloromethane (15 mL). Under a nitrogen atmosphere, ethylmagnesium bromide (3.44 mL, 6.87 mmol, 2 mol/L) was slowly added dropwise at 0°C, and the mixture was stirred at 0°C for 0.5 hours. The crude intermediate was dissolved in dichloromethane (20 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (dichloromethane/ethyl acetate, 1/1, v/v) to obtain compound **28-6.** ESI-MS calculated for: [M+H]⁺ = 413.16, found: 413.0.

### Step 6

Compound **28-6** (200 mg, 0.48 mmol) was dissolved in tetrahydrofuran (5 mL). A solution of lithium aluminum hydride in tetrahydrofuran (1.92 mL, 4.80 mmol, 2.5 mol/L) was added dropwise at 0°C. Under a nitrogen atmosphere, the reaction mixture was stirred at 60°C for 8 hours. The reaction mixture was cooled to room temperature, quenched by the addition of ice water (0.19 mL), and then 15% aqueous sodium hydroxide solution (0.19 mL) and water (0.57 mL) were added. The mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 40-60%, retention time: 6.6-8.3 min, run time: 17 min) to obtain compound **28.** ¹H NMR (400 MHz, DMSO-*d*₆): δ 10.72 (s, 1H), 7.24 (d, J = 8.8 Hz, 1H), 7.19 (d, J = 2.4 Hz, 1H), 7.10 (d, J = 2.4 Hz, 1H), 6.80 (dd, J = 8.8, 2.4 Hz, 1H), 5.09-4.98 (m, 1H), 3.24-3.19 (m, 1H), 3.12-3.06 (m, 1H), 2.90-2.82 (m, 1H), 2.76-2.69 (m, 1H), 2.62-2.57 (m, 1H), 2.11 (s, 3H), 1.96-1.89 (m, 1H), 1.82-1.74 (m, 1H), 1.41 (d, J = 6.4 Hz, 3H). ESI-MS calculated for: [M+H]⁺ = 313.14, found 313.0.

### Example 29

### Synthetic route:

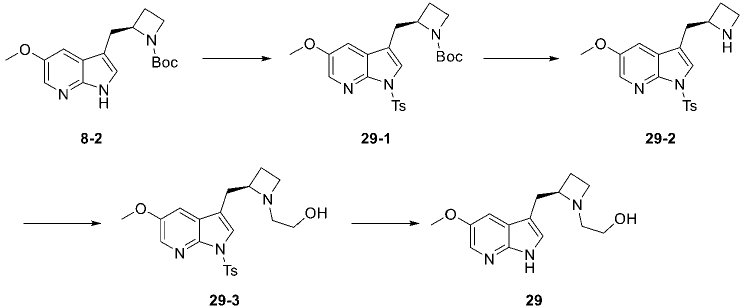

### Step 1

Compound **8-2** (1.00 g, 3.15 mmol) was dissolved in *N,N*-dimethylformamide (20 mL). Under a nitrogen atmosphere, sodium hydride (151 mg, 3.78 mmol, 60% purity) was slowly added at 0°C, and the mixture was stirred at 0°C for 0.5 hours. p-Toluenesulfonyl chloride (721 mg, 3.78 mmol) was added, and the mixture was stirred at 25°C for 3 hours. Saturated aqueous ammonium chloride solution (20 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **29-1.** ESI-MS calculated for: [M+H]⁺ = 472.18, found 472.0.

### Step 2

Compound **29-1** (100 mg, 0.91 mmol) was dissolved in anhydrous dichloromethane (10 mL). Trifluoroacetic acid (4 mL) was added, and the mixture was stirred at 25°C for 1 hour. The reaction mixture was concentrated under reduced pressure. Saturated aqueous sodium bicarbonate solution (20 mL) was added to the residue, and the mixture was extracted with dichloromethane (50 mL × 3). The organic phases were combined and washed with saturated brine (50 mL). The organic phase was dried over anhydrous sodium sulfate, filtered, and concentrated under reduced pressure to obtain compound **29-2.** ESI-MS calculated for: [M+H]⁺ = 372.13, found 372.1.

### Step 3

Compound **29-2** (200 mg, 0.27 mmol) was dissolved in N,N-dimethylformamide (4 mL). Bromoethanol (169 mg, 1.35 mmol) and potassium carbonate (112 mg, 0.81 mmol) were added to the reaction mixture, and the reaction mixture was stirred at 70°C for 6 hours. Saturated aqueous ammonium chloride solution (30 mL) was added to the reaction mixture, and the mixture was extracted with ethyl acetate (30 mL × 3). The organic phases were combined, washed with saturated brine (30 mL), dried over anhydrous sodium sulfate, filtered, and concentrated under reduced pressure. The crude product was purified by silica gel column chromatography (dichloromethane/methanol, 10/1, v/v) to obtain compound **29-3.** ESI-MS calculated for: [M+H]⁺ = 416.16, found 416.5.

### Step 4

Compound **29-3** (55.0 mg, 0.13 mmol) was dissolved in anhydrous ethanol (3 mL) and water (1 mL). Sodium hydroxide (26.0 mg, 0.65 mmol) was added, and the reaction mixture was stirred at 70°C for 6 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 28-38%, retention time: 9.5-10.5 min, run time: 17 min) to obtain compound 29. ¹H NMR (400 MHz, DMSO-*d*₆): δ 11.16 (s, 1H), 7.92 (d, J = 2.4 Hz, 1H), 7.52 (d, J = 2.4 Hz, 1H), 7.18 (d, J = 2.0 Hz, 1H), 4.42-4.36 (m, 1H), 3.82 (s, 3H), 3.38-3.35 (m, 2H), 3.30-3.27 (m, 2H), 2.96-2.91 (m, 1H), 2.79-2.73 (m, 2H), 2.57-2.54 (m, 1H), 2.35-2.30 (m, 1H), 1.95-1.90 (m, 1H), 1.84-1.78 (m, 1H). ESI-MS calculated for: [M+H]⁺ = 262.15, found 262.1.

### Example 30

### Synthetic route:

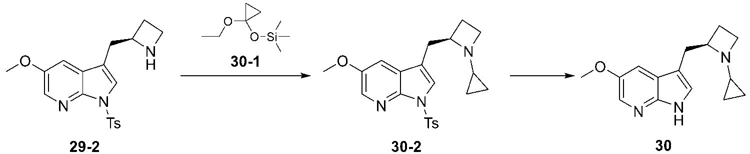

### Step 1

Compound **29-2** (180 mg, 0.48 mmol) was dissolved in 1,2-dichloroethane (15 mL). Compound **30-1** (167 mg, 0.96 mmol) and sodium triacetoxyborohydride (203 mg, 0.96 mmol) were added to the reaction mixture, and the reaction mixture was stirred at 60°C for 16 hours. Saturated aqueous sodium bicarbonate solution (30 mL) was added to the reaction mixture, and the mixture was extracted with dichloromethane (30 mL × 3). The organic phases were combined, washed with saturated brine (30 mL), dried over anhydrous sodium sulfate, filtered, and concentrated under reduced pressure. The crude product was purified by silica gel column chromatography (petroleum ether/ethyl acetate, 5/1, v/v) to obtain compound **30-2.** ESI-MS calculated for: [M+H]⁺ = 412.16, found 412.5.

### Step 2

Compound **30-2** (65.0 mg, 0.16 mol) was dissolved in anhydrous ethanol (3 mL) and water (1.2 mL). Lithium hydroxide monohydrate (67.1 mg, 1.60 mmol) was added, and the reaction mixture was stirred at 60°C for 4 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 28-38%, retention time: 9.5-10.5 min, run time: 17 min) to obtain compound **30.** ¹H NMR (400 MHz, DMSO-*d*₆): δ 11.16 (s, 1H), 7.92 (d, J = 2.8 Hz, 1H), 7.48 (d, J = 2.8 Hz, 1H), 7.18 (s, 1H), 3.82 (s, 3H), 3.55-3.49 (m, 1H), 3.20-3.16 (m, 1H), 2.96-2.85 (m, 2H), 2.75-2.68 (m, 1H), 1.92-1.85 (m, 1H), 1.84-1.79 (m, 1H), 1.76-1.68 (m, 1H), 0.34-0.23 (m, 3H), 0.15-0.11 (m, 1H). ESI-MS calculated for: [M+H]⁺ = 258.15, found 258.1.

### Example 31

### Synthetic route:

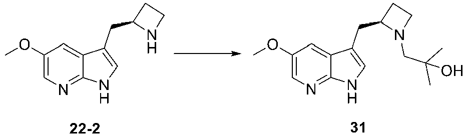

### Step 1

Compound **22-2** (200 mg, 0.92 mmol) was dissolved in ethylene glycol dimethyl ether (5 mL). Triethylamine (189 mg, 1.87 mmol) and methyloxirane (265 mg, 3.68 mmol) were added to the reaction mixture, and the reaction mixture was stirred at 70°C for 16 hours. The reaction mixture was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 18-28%, retention time: 9.3-11.3 min, run time: 17 min) to obtain compound **31.** ¹H NMR (400 MHz, DMSO-*d*₆): δ 11.15 (s, 1H), 7.91 (d, J = 2.8 Hz, 1H), 7.50 (d, J = 2.8 Hz, 1H), 7.17 (d, J = 2.4 Hz, 1H), 3.98-2.94 (m, 1H), 3.82 (s, 3H), 3.39-3.37 (m, 1H), 3.34-3.30 (m, 1H), 2.92-2.87 (m, 1H), 2.75-2.69 (m, 2H), 2.34 (d, J = 12.4 Hz, 1H), 2.18 (d, J = 12.4 Hz, 1H), 1.95-1.88 (m, 1H), 1.85-1.77 (m, 1H), 1.02 (s, 3H), 1.01 (s, 3H). ESI-MS calculated for: [M+H]⁺ = 290.18, found 290.1.

### Example 32

### Synthetic route:

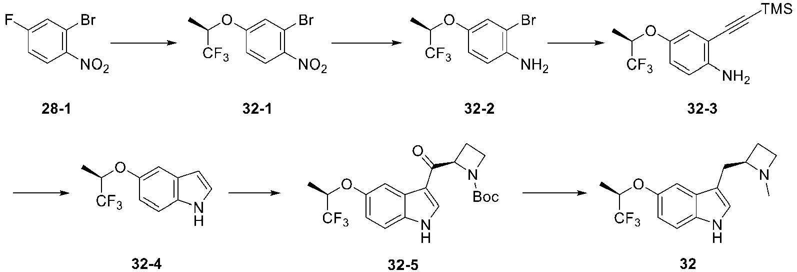

### Step 1

Compound (2R)-1,1,1-trifluoropropan-2-ol (9.70 g, 85.0 mmol) was dissolved in *N,N-*dimethylformamide (200 mL). Under a nitrogen atmosphere, sodium hydride (2.23 g, 92.7 mmol, 60% purity) was slowly added at 0°C, and the mixture was stirred at 0°C for 0.5 hours. Compound **28-1** (17.0 g, 77.3 mmol) was slowly added, and the mixture was stirred at 25°C for 0.5 hours. Saturated aqueous ammonium chloride solution (200 mL) was added to the reaction mixture, and the mixture was extracted with ethyl acetate (200 mL × 3). The organic phases were combined and washed with saturated brine (200 mL). The organic phase was dried over anhydrous sodium sulfate, filtered, and concentrated under reduced pressure. The crude product was purified by silica gel column chromatography (petroleum ether/ethyl acetate, 15/1, v/v) to obtain compound **32-1.** ¹H NMR (400 MHz, DMSO-*d*₆): δ 8.09 (d, J = 8.0 Hz, 1H), 7.69-7.68 (d, J = 2.8 Hz, 1H), 7.36-7.30 (m, 1H), 5.59-5.53 (m, 1H), 1.45 (d, J = 6.4 Hz, 3H).

### Step 2

Compound **32-1** (22.0 g, 70.1 mmol) was dissolved in ethanol (500 mL) and water (100 mL). Iron powder (19.6 g, 350 mmol) and ammonium chloride (18.7 g, 350 mmol) were added, and the mixture was stirred at 80°C for 6 hours. The reaction mixture was cooled to room temperature and filtered. Water (150 mL) was added to the filtrate, and the mixture was extracted with ethyl acetate (150 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 15/1, v/v) to obtain compound **32-2.** ¹H NMR (400 MHz, DMSO-*d*₆): δ 7.15 (d, *J* = 2.8 Hz, 1H), 6.86 (dd, J = 8.0, 2.8 Hz, 1H), 6.76 (d, J = 8.0 Hz, 1H), 5.02 (s, 2H), 4.96-4.91 (m, 1H), 1.35 (d, J = 6.4 Hz, 3H).

### Step 3

Compound **32-2** (19.0 g, 60.5 mmol) was dissolved in triethylamine (150 mL). Under a nitrogen atmosphere, trimethylsilylacetylene (11.9 g, 121 mmol), bis(triphenylphosphine)palladium(II) dichloride (2.12 g, 3.03 mmol), and copper(I) iodide (580 mg, 3.03 mmol) were added, and the mixture was stirred at 50°C for 16 hours. The reaction mixture was cooled to room temperature and filtered. Water (100 mL) was added to the filtrate, and the mixture was extracted with ethyl acetate (150 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 20/1, v/v) to obtain compound **32-3.** ¹H NMR (400 MHz, DMSO-*d*₆): δ 6.90 (d, J = 2.8 Hz, 1H), 6.84 (dd, J = 8.8, 2.8 Hz, 1H), 6.67 (d, J = 8.8 Hz, 1H), 5.07 (s, 2H), 4.95-4.86 (m, 1H), 1.33 (d, J = 6.4 Hz, 3H), 0.23 (s, 9H).

### Step 4

Compound **32-3** (8.90 g, 29.5 mmol) was dissolved in *N,N*-dimethylformamide (90 mL). Under a nitrogen atmosphere, copper(I) iodide (11.3 g, 59.1 mmol) was added, and the mixture was stirred at 100°C for 2 hours. The reaction mixture was cooled to room temperature. Water (100 mL) was added, and the mixture was extracted with ethyl acetate (200 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 15/1, v/v) to obtain compound **32-4.** ¹H NMR (400 MHz, DMSO-*d*₆): δ 11.01 (s, 1H), 7.35-7.29 (m, 2H), 7.22 (d, J = 2.8 Hz, 1H), 6.82 (dd, J = 8.8, 2.8 Hz, 1H), 6.35 (t, J = 2.0 Hz, 1H), 5.05-4.96 (m, 1H), 1.40 (d, J = 6.4 Hz, 3H). ESI-MS calculated for: [M+H]⁺ = 230.07, found 230.0.

### Step 5

(*R*)-*N*-*tert*-Butoxycarbonyl-azetidine-2-carboxylic acid (1.98 g, 9.83 mmol) was dissolved in dichloromethane (30 mL). Oxalyl chloride (0.683 mL, 11.8 mmol) was added dropwise at 0°C, and the reaction mixture was stirred at 0°C for 10 minutes. *N,N-*Dimethylformamide (72.0 mg, 1.04 mmol) was slowly added, and the reaction mixture was stirred at 25°C for 1 hour. The reaction mixture was concentrated under reduced pressure to obtain a crude intermediate. Compound **32-4** (2.00 g, 8.73 mmol) was dissolved in dichloromethane (15 mL). Under a nitrogen atmosphere, ethylmagnesium bromide (3.44 mL, 6.87 mmol, 2 mol/L) was slowly added dropwise at 0°C, and the mixture was stirred at 0°C for 0.5 hours. The crude intermediate was dissolved in dichloromethane (20 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (dichloromethane/ethyl acetate, 1/1, v/v) to obtain compound **32-5.** ESI-MS calculated for: [M+H]⁺ = 413.16, found: 413.0.

### Step 6

Compound **32-5** (144 mg, 0.35 mmol) was dissolved in tetrahydrofuran (7 mL). A solution of lithium aluminum hydride in tetrahydrofuran (1.40 mL, 3.50 mmol, 2.5 mol/L) was added dropwise at 0°C. Under a nitrogen atmosphere, the reaction mixture was stirred at 60°C for 8 hours. The reaction mixture was cooled to room temperature, quenched by the addition of ice water (0.14 mL), and then 15% aqueous sodium hydroxide solution (0.14 mL) and water (0.42 mL) were added. The mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 38-51%, retention time: 7.7-9.5 min, run time: 16 min) to obtain compound **32.** ¹H NMR (400 MHz, DMSO-*d*₆): δ 10.72 (s, 1H), 7.24 (d, J = 8.8 Hz, 1H), 7.19 (d, J = 2.4 Hz, 1H), 7.10 (d, J = 2.4 Hz, 1H), 6.80 (dd, J = 8.8, 2.4 Hz, 1H), 5.07-5.00 (m, 1H), 3.26-3.22 (m, 1H), 3.13-3.07 (m, 1H), 2.86-2.82 (m, 1H), 2.75-2.67 (m, 1H), 2.63-2.58 (m, 1H), 2.11 (s, 3H), 1.97-1.90 (m, 1H), 1.82-1.73 (m, 1H), 1.41 (d, J = 6.4 Hz, 3H). ESI-MS calculated for: [M+H]⁺ = 313.14, found 313.0.

### Example 33

### Synthetic route:

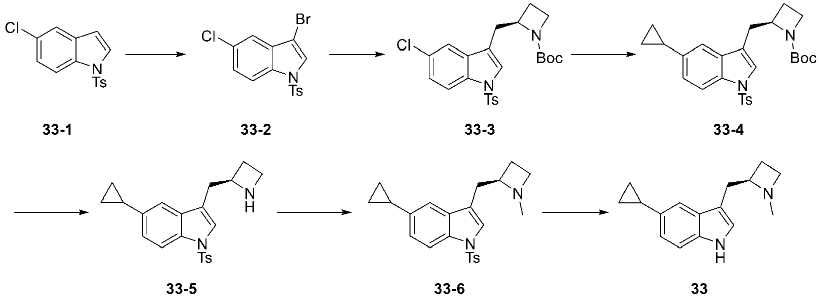

### Step 1

Compound **33-1** (8.86 g, 29.0 mmol) was dissolved in N,N-dimethylformamide (125 mL). Under a nitrogen atmosphere, liquid bromine (1.63 mL, 31.9 mmol) was slowly added at 0°C, and the mixture was stirred at 25°C for 1 hour. Aqueous sodium bicarbonate solution (140 mL) was added to the reaction mixture, and the mixture was extracted with ethyl acetate (300 mL × 3). The organic phases were combined and washed with saturated brine (300 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **33-2.** ¹H NMR (400 MHz, CDCl₃): δ 7.84 (d, J = 8.8 Hz, 1H), 7.68 (d, J = 8.8 Hz, 2H), 7.55 (s, 1H), 7.39 (d, J = 2.4 Hz, 1H), 7.25 (dd, J = 8.8, 2.4 Hz, 1H), 7.18 (d, J = 7.6 Hz, 2H), 2.29 (s, 3H).

### Step 2

In a glove box filled with nitrogen, compound **33-2** (500 mg, 1.30 mmol), (2*R*)-*tert-*butyl 2-(bromomethyl)azetidine-1-carboxylate (490 mg, 1.95 mmol), anhydrous sodium carbonate (276 mg, 2.60 mmol), tris(trimethylsilyl)silane (480 mg, 1.95 mmol), nickel(II) chloride dimethoxyethane (14.0 mg, 0.065 mmol), 4,4'-di-*tert*-butyl-2,2'-dipyridine (17.5 mg, 0.065 mmol), and bis[2-(2,4-difluorophenyl)-5-trifluoromethylpyridine][2-2'-bi(4-*tert-*butylpyridine)]iridium(III) bis(hexafluorophosphate) (15.0 mg, 0.013 mmol) were dissolved in *N,N*-dimethylacetamide (8 mL). The reaction mixture was placed under irradiation with a 34 W blue LED (420 nm) and stirred at 25°C for 16 hours. After completion of the reaction, the blue light was turned off. Water (50 mL) was added to the reaction mixture, and the mixture was extracted with dichloromethane (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (petroleum ether/ethyl acetate, 3/1, v/v) to obtain compound **33-3.** ¹H NMR (400 MHz, DMSO-*d*₆): δ 7.91 (d, J = 8.8 Hz, 1H), 7.82 (d, J = 8.4 Hz, 2H), 7.70 (s, 1H), 7.61 (d, J = 2.4 Hz, 1H), 7.39-7.34 (m, 3H), 4.43-4.38 (m, 1H), 3.65-3.61 (m, 1H), 3.34-3.30 (m, 2H), 3.01-2.98 (m, 1H), 2.31 (s, 3H), 2.17-2.10 (m, 1H), 1.83-1.77 (m, 1H), 1.34 (s, 9H). ESI-MS calculated for: [M+Na]⁺ = 497.14, found 497.1.

### Step 3

Compound **33-3** (335 mg, 0.71 mmol) was dissolved in 1,4-dioxane (10 mL) and water (2 mL). Cyclopropylboronic acid (91.5 mg, 1.06 mmol), potassium phosphate (452 mg, 2.13 mmol), and methanesulfonato(2-dicyclohexylphosphino-2',4',6'-triisopropyl-1,1'-biphenyl)(2'-amino-1,1'-biphenyl-2-yl)palladium(II) (60.1 mg, 0.071 mmol) were added. Under a nitrogen atmosphere, the mixture was stirred at 100°C for 16 hours. The reaction mixture was cooled to room temperature. Water (30 mL) was added, and the mixture was extracted with ethyl acetate (100 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (petroleum ether/ethyl acetate, 5/1, v/v) to obtain compound **33-4.** ESI-MS calculated for: [M+H-56]⁺ = 425.21, found 425.1.

### Step 4

Compound **33-4** (270 mg, 0.56 mmol) was dissolved in anhydrous dichloromethane (10 mL). Trifluoroacetic acid (2 mL) was added, and the mixture was stirred at 25°C for 1 hour. The reaction mixture was concentrated under reduced pressure. Saturated aqueous sodium bicarbonate solution (20 mL) was added to the residue, and the mixture was extracted with dichloromethane (50 mL × 3). The organic phases were combined and washed with saturated brine (50 mL). The organic phase was dried over anhydrous sodium sulfate, filtered, and concentrated under reduced pressure to obtain compound **33-5.** ESI-MS calculated for: [M+H]⁺ = 381.16, found 381.1.

### Step 5

Compound **33-5** (210 mg, 0.55 mmol) was dissolved in 1,2-dichloroethane (5 mL). Triethylamine (167 mg, 1.65 mmol) and 37% formaldehyde solution (134 mg, 165 mmol) were added to the reaction mixture, and the reaction mixture was stirred at 25°C for 0.5 hours. Sodium triacetoxyborohydride (233 mg, 1.10 mmol) was added, and the reaction mixture was stirred at 25°C for 2 hours. Saturated aqueous sodium bicarbonate solution (30 mL) was added to the reaction mixture, and the mixture was extracted with ethyl acetate (30 mL × 3). The organic phases were combined and dried over anhydrous sodium sulfate. After filtration, the filtrate was concentrated under vacuum. The crude product was purified by silica gel column chromatography (dichloromethane/methanol, 15/1, v/v) to obtain compound **33-6.** ESI-MS calculated for: [M+H]⁺ = 395.17, found 395.2.

### Step 6

Compound **33-6** (125 mg, 0.32 mmol) was dissolved in anhydrous methanol (3 mL) and water (1 mL). Potassium hydroxide (180 mg, 3.20 mmol) was added, and the reaction mixture was stirred at 80°C for 2 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 33-50%, retention time: 8.0-10.3 min, run time: 18 min) to obtain compound **33.** ¹H NMR (400 MHz, DMSO-*d*₆): δ 10.61 (s, 1H), 7.22 (s, 1H), 7.18 (d, J = 8.4 Hz, 1H), 7.03 (d, J = 2.0 Hz, 1H), 6.77 (dd, J = 8.4, 2.0 Hz, 1H), 3.32-3.30 (m, 1H), 3.25-3.17 (m, 1H), 3.10-3.03 (m, 1H), 2.74-2.69 (m, 1H), 2.62-2.57 (m, 1H), 2.11 (s, 3H), 1.99-1.95 (m, 1H), 1.92-1.87 (m, 1H), 1.82-1.72 (m, 1H), 0.93-0.85 (m, 2H), 0.66-0.58 (m, 2H). ESI-MS calculated for: [M+H]⁺ = 241.16, found 241.1.

### Example 34

### Synthetic route:

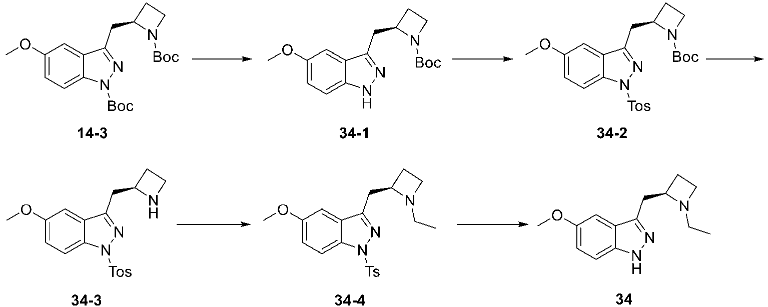

### Step 1

Compound **14-3** (1.68 g, 4.02 mmol) was dissolved in tetrahydrofuran (10 mL), ethanol (10 mL), and water (4 mL). Lithium hydroxide monohydrate (843 mg, 20.1 mmol) was added to the reaction system, and the mixture was stirred at 25°C for 2 hours. Water (40 mL) was added to the reaction mixture, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **34-1.** ¹H NMR (400 MHz, DMSO-*d*₆): δ 12.61 (s, 1H), 7.37 (d, J = 8.8 Hz, 1H), 7.09 (s, 1H), 6.98 (dd, J = 8.8, 2.4 Hz, 1H), 4.51-4.47 (m, 1H), 3.78 (s, 3H), 3.72-3.66 (m, 1H), 3.65-3.57 (m, 1H), 3.38-3.31 (m, 1H), 3.21-3.18 (m, 1H), 2.18-2.12 (m, 1H), 1.99-1.94 (m, 1H), 1.38 (s, 9H). ESI-MS calculated for: [M+H]⁺ = 318.17, found 318.1.

### Step 2

Compound **34-1** (945 mg, 2.98 mmol) was dissolved in *N,N*-dimethylformamide (10 mL). Under a nitrogen atmosphere, sodium hydride (143 mg, 3.58 mmol, 60% purity) was slowly added at 0°C, and the mixture was stirred at 0°C for 0.5 hours. *p*-Toluenesulfonyl chloride (682 mg, 3.58 mmol) was added, and the mixture was stirred at 25°C for 2 hours. Aqueous ammonium chloride solution (50 mL) was added to the reaction mixture, and the mixture was extracted with ethyl acetate (50 mL × 3). The organic phases were combined and washed with saturated brine (50 mL × 3). The organic phase was dried over anhydrous sodium sulfate, filtered, and concentrated under reduced pressure. The crude product was purified by silica gel column chromatography (petroleum ether/ethyl acetate, 10/1, v/v) to obtain compound **34-2.** ESI-MS calculated for: [M+H]⁺ = 472.18, found 472.1.

### Step 3

Compound **34-2** (1.20 g, 2.54 mmol) was dissolved in 1,2-dichloromethane (10 mL). Trifluoroacetic acid (5 mL, 67.3 mmol) was slowly added at 0°C, and the mixture was stirred at 25°C for 2 hours. Saturated aqueous sodium bicarbonate solution (100 mL) was added to the reaction mixture, and the mixture was extracted with dichloromethane (100 mL × 3). The organic phases were combined and washed with saturated brine (100 mL). The organic phase was dried over anhydrous sodium sulfate, filtered, and concentrated under reduced pressure to obtain compound **34-3.** ESI-MS calculated for: [M+H]⁺ = 372.13, found 372.1.

### Step 4

Compound **34-3** (300 mg, 0.81 mmol) was dissolved in methanol (5 mL). *N,N-*Diisopropylethylamine (310 mg, 2.43 mmol) and acetaldehyde (143 mg, 1.62 mmol) were added, and the mixture was stirred at 25°C for 0.5 hours. Sodium cyanoborohydride (51.4 mg, 0.82 mmol) was added, and the mixture was stirred at 25°C for 2 hours. Saturated aqueous sodium bicarbonate solution (50 mL) was added to the reaction mixture, and the mixture was extracted with ethyl acetate (50 mL × 3). The organic phases were combined and washed with saturated brine (50 mL × 3). The organic phase was dried over anhydrous sodium sulfate, filtered, and concentrated under reduced pressure. The crude product was purified by silica gel column chromatography (dichloromethane/methanol, 20/1, v/v) to obtain compound **34-4.** ESI-MS calculated for: [M+H]⁺ = 400.16, found 400.0.

### Step 5

Compound **34-4** (170 mg, 0.43 mmol) was dissolved in tetrahydrofuran (2 mL), methanol (2 mL), and water (0.8 mL). Lithium hydroxide monohydrate (90.2 mg, 2.15 mmol) was added, and the reaction mixture was stirred at 25°C for 12 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 28-48%, retention time: 7.5-9.5 min, run time: 17 min) to obtain compound **34.** ¹H NMR (400 MHz, DMSO-*d*₆): δ 12.49 (s, 1H), 7.34 (d, J = 8.8 Hz, 1H), 7.14 (d, J = 2.0 Hz, 1H), 6.95 (dd, J = 8.8, 2.0 Hz, 1H), 3.78 (s, 3H), 3.30-3.24 (m, 2H), 3.12-3.07 (m, 1H), 3.00-2.94 (m, 1H), 2.58-2.53 (m, 1H), 2.49-2.46 (m, 1H), 2.19-2.13 (m, 1H), 1.93-1.81 (m, 2H), 0.84 (t, J = 7.2 Hz, 3H). ESI-MS calculated for: [M+H]⁺ = 246.15, found 246.1.

### Example 35

### Synthetic route:

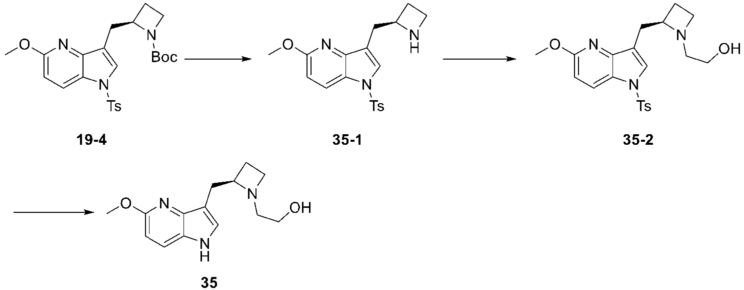

### Step 1

Compound **19-4** (250 mg, 0.53 mmol) was dissolved in 1,2-dichloromethane (10 mL). Trifluoroacetic acid (3.5 mL, 47.1 mmol) was slowly added at 0°C, and the mixture was stirred at 25°C for 2 hours. Saturated aqueous sodium bicarbonate solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure to obtain compound **35-1.** ESI-MS calculated for: [M+H]⁺ = 372.13, found 372.4.

### Step 2

Compound **35-1** (216 mg, 0.58 mmol) was dissolved in N,N-dimethylformamide (3.5 mL). Potassium carbonate (240 mg, 1.74 mmol), potassium iodide (9.6 mg, 0.058 mmol), and 2-bromoethanol (217 mg, 1.74 mmol) were added, and the mixture was stirred at 70°C for 2 hours. Water (20 mL) was added to the reaction mixture, and the mixture was extracted with ethyl acetate (20 mL × 3). The organic phases were combined and washed with saturated brine (20 mL × 3). The organic phase was dried over anhydrous sodium sulfate, filtered, and concentrated under reduced pressure. The crude product was purified by silica gel column chromatography (dichloromethane/methanol, 10/1, v/v) to obtain compound **35-2.** ESI-MS calculated for: [M+H]⁺ = 416.16, found 416.4.

### Step 3

Compound **35-2** (185 mg, 0.45 mmol) was dissolved in ethanol (2 mL) and water (0.5 mL). Sodium hydroxide (54.0 mg, 1.35 mmol) was added, and the reaction mixture was stirred at 60°C for 4 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-0.04% aqueous ammonia + 7.5 mmol/L aqueous ammonium bicarbonate solution, gradient: 20-25%, retention time: 6.2-8.0 min, run time: 16 min) to obtain compound **35.** ¹H NMR (400 MHz, DMSO-*d*₆): δ 10.81 (s, 1H), 7.61 (d, J = 8.8 Hz, 1H), 7.25 (s, 1H), 6.49 (d, J = 8.8 Hz, 1H), 4.33-4.29 (m, 1H), 3.86 (s, 3H), 3.41-3.36 (m, 2H), 3.29-3.25 (m, 2H), 2.95-2.89 (m, 1H), 2.79-2.75 (m, 1H), 2.70-2.65 (m, 1H), 2.60-2.55 (m, 1H), 2.35-2.29 (m, 1H), 1.92-1.81 (m, 2H). ESI-MS calculated for: [M+H]⁺ = 262.15, found 262.0.

### Example 36

### Synthetic route:

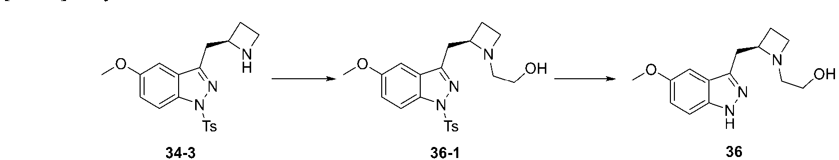

### Step 1

Compound **34-3** (250 mg, 0.67 mmol) was dissolved in *N,N*-dimethylformamide (5 mL). Potassium carbonate (278 mg, 2.01 mmol) and 2-bromoethanol (251 mg, 2.01 mmol) were added, and the mixture was stirred at 25°C for 3 hours. Water (20 mL) was added to the reaction mixture, and the mixture was extracted with ethyl acetate (20 mL × 3). The organic phases were combined and washed with saturated brine (20 mL × 3). The organic phase was dried over anhydrous sodium sulfate, filtered, and concentrated under reduced pressure. The crude product was purified by silica gel column chromatography (dichloromethane/methanol, 10/1, v/v) to obtain compound **36-1.** ESI-MS calculated for: [M+H]⁺ = 416.16, found 416.5.

### Step 2

Compound **36-1** (110 mg, 0.26 mmol) was dissolved in tetrahydrofuran (1 mL), methanol (1 mL), and water (1 mL). Lithium hydroxide monohydrate (54.6 mg, 1.37 mmol) was added, and the reaction mixture was stirred at 25°C for 16 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 17-27%, retention time: 7.8-8.7 min, run time: 17 min) to obtain compound **36.** ¹H NMR (400 MHz, DMSO-*d*₆): δ 12.51 (s, 1H), 7.35 (d, J = 8.8 Hz, 1H), 7.15 (s, 1H), 6.96 (d, J = 8.8 Hz, 1H), 4.44-4.39 (m, 1H), 3.79 (s, 3H), 3.48-3.43 (m, 1H), 3.39-3.35 (m, 2H), 3.17-3.09 (m, 1H), 2.92-2.88 (m, 1H), 2.85-2.70 (m, 1H), 2.63-2.52 (m, 2H), 2.40-2.29 (m, 1H), 1.98-1.85 (m, 2H). ESI-MS calculated for: [M+H]⁺ = 262.15, found 262.0.

### Example 37

### Synthetic route:

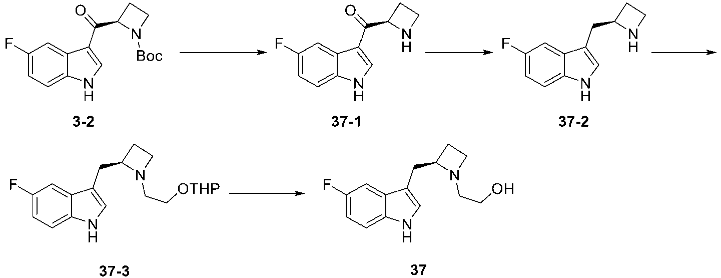

### Step 1

Compound **3-2** (2.12 g, 6.66 mmol) was dissolved in 1,2-dichloromethane (40 mL). Trifluoroacetic acid (10 mL) was slowly added at 0°C, and the mixture was stirred at 25°C for 1 hour. The reaction mixture was concentrated under reduced pressure to obtain a crude product containing the target compound. The crude product was purified by high-performance liquid chromatography (Waters-spherical-C18-20 µm, 100A, 330 g, mobile phase: acetonitrile-0.5% formic acid in water, gradient: 11-20%, retention time: 16-27 min, run time: 40 min) to obtain compound **37-1.** ¹H NMR (400 MHz, DMSO-*d*₆): δ 12.51 (s, 1H), 8.43 (s, 1H), 7.86 (dd, J = 8.8, 2.4 Hz, 1H), 7.57 (dd, J = 8.80, 4.4 Hz, 1H), 7.18-7.13 (m, 1H), 5.79-5.75 (m, 1H), 4.11-4.04 (m, 1H), 3.84-3.78 (m, 1H), 2.98-2.93 (m, 1H), 2.50-2.43 (m, 1H). ESI-MS calculated for: [M+H]⁺ = 219.09, found 219.0.

### Step 2

Compound **37-1** (2.40 g, 11.0 mmol) was dissolved in tetrahydrofuran (90 mL). A solution of lithium aluminum hydride in tetrahydrofuran (44 mL, 110 mmol, 2.5 mol/L) was added dropwise at 0°C. Under a nitrogen atmosphere, the reaction mixture was stirred at 60°C for 3 hours. The reaction mixture was cooled to room temperature, quenched by the addition of ice water (4.1 mL), and then 15% aqueous sodium hydroxide solution (4.1 mL) and water (12.3 mL) were added. The mixture was filtered, and the filtrate was concentrated under reduced pressure to obtain a crude product containing the target compound. The crude product was purified by high-performance liquid chromatography (Waters-spherical-C18-20 µm, 100A, 220 g, mobile phase: acetonitrile-10 mmol/L aqueous ammonium bicarbonate solution, gradient: 15-25%, retention time: 20-35 min, run time: 50 min) to obtain compound **37-2.** ESI-MS calculated for: [M+H]⁺ = 205.11, found 205.0.

### Step 3

Compound **37-2** (350 mg, 1.71 mmol) was dissolved in N,N-dimethylformamide (5 mL) and acetonitrile (5 mL). Potassium carbonate (709 mg, 5.13 mmol) and 2-(2-bromoethoxy)tetrahydropyran (1.07 g, 5.13 mmol) were added, and the mixture was stirred at 25°C for 16 hours. Water (20 mL) was added to the reaction mixture, and the mixture was extracted with ethyl acetate (20 mL × 3). The organic phases were combined and washed with saturated brine (20 mL × 3). The organic phase was dried over anhydrous sodium sulfate, filtered, and concentrated under reduced pressure. The crude product was purified by silica gel column chromatography (dichloromethane/methanol, 10/1, v/v) to obtain compound **37-3.** ESI-MS calculated for: [M+H]⁺ = 333.19, found 333.0.

### Step 4

Compound **37-3** (150 mg, 0.45 mol) was dissolved in tetrahydrofuran (5 mL). An aqueous hydrochloric acid solution (0.90 mL, 0.90 mmol, 1 mol/L) was added, and the reaction mixture was stirred at 25°C for 3 hours. The reaction mixture was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 20-30%, retention time: 8.8-10.3 min, run time: 17 min) to obtain compound **37.** ¹H NMR (400 MHz, DMSO-*d*₆): δ 10.87 (s, 1H), 7.31-7.24 (m, 2H), 7.18 (s, 1H), 6.90-6.85 (m, 1H), 4.34 (s, 1H), 3.28-3.21 (m, 4H), 2.93-2.88 (m, 1H), 2.78-2.62 (m, 2H), 2.57-2.53 (m, 1H), 2.36-2.25 (m, 1H), 1.95-1.87 (m, 1H), 1.82-1.71 (m, 1H). ESI-MS calculated for: [M+H]⁺ = 249.13, found 249.0.

### Example 38

### Synthetic route:

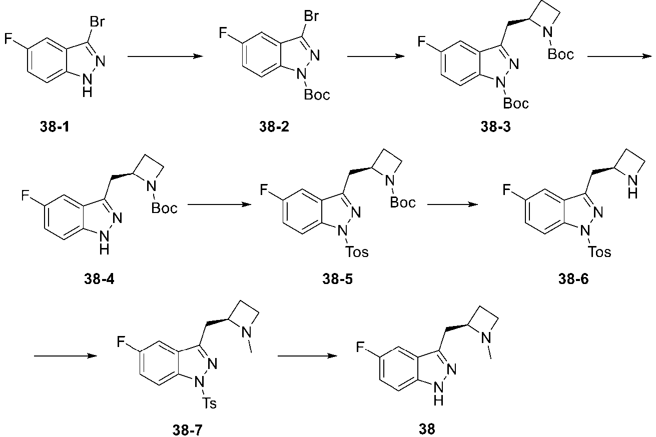

### Step 1

Compound **38-1** (2.00 g, 9.30 mmol) was dissolved in 1,2-dichloromethane (30 mL). Triethylamine (1.88 g, 18.6 mmol), di-tert-butyl dicarbonate (3.04 g, 14.0 mmol), and 4-dimethylaminopyridine (114 mg, 0.93 mmol) were added, and the mixture was stirred at 25°C for 4 hours. Saturated aqueous ammonium chloride solution (40 mL) was added to the reaction mixture, and the mixture was extracted with dichloromethane (30 mL × 3). The organic phases were combined and washed with saturated brine (60 mL). The organic phase was dried over anhydrous sodium sulfate, filtered, and concentrated under reduced pressure. The crude product was purified by silica gel column chromatography (petroleum ether/dichloromethane, 5/1, v/v) to obtain compound **38-2.** ¹H NMR (400 MHz, DMSO-*d*₆): δ 8.13 (dd, J = 9.2, 4.0 Hz, 1H), 7.65-7.60 (m, 1H), 7.57 (dd, J = 8.0, 2.4 Hz, 1H), 1.65 (s, 9H).

### Step 2

In a glove box filled with nitrogen, compound **38-2** (400 mg, 1.60 mmol), (2*R*)-*tert-*butyl 2-(bromomethyl)azetidine-1-carboxylate (500 mg, 1.59 mmol), anhydrous sodium carbonate (339 mg, 3.20 mmol), tris(trimethylsilyl)silane (398 mg, 1.60 mmol), nickel(II) chloride dimethoxyethane (17.6 mg, 0.080 mmol), 4,4'-di-*tert*-butyl-2,2'-dipyridine (21.5 mg, 0.080 mmol), and bis[2-(2,4-difluorophenyl)-5-trifluoromethylpyridine][2-2'-bi(4-*tert-*butylpyridine)]iridium(III) bis(hexafluorophosphate) (18.0 mg, 0.016 mmol) were dissolved in ethylene glycol dimethyl ether (5 mL). The reaction mixture was placed under irradiation with a 34 W blue LED (420 nm) and stirred at 25°C for 16 hours. After completion of the reaction, the blue light was turned off. Water (50 mL) was added to the reaction mixture, and the mixture was extracted with ethyl acetate (50 mL × 3). The organic phases were combined and washed with saturated brine (50 mL × 2). The organic phase was dried over anhydrous sodium sulfate, filtered, and concentrated under reduced pressure. The crude product was purified by silica gel column chromatography (petroleum ether/ethyl acetate, 3/1, v/v) to obtain compound **38-3.** ESI-MS calculated for: [M+H]⁺ = 406.21, found 406.1.

### Step 3

Compound .**38-3** (880 mg, 2.17 mmol) was dissolved in tetrahydrofuran (5 mL), ethanol (5 mL), and water (2 mL). Lithium hydroxide monohydrate (455 mg, 10.9 mmol) was added, and the reaction mixture was stirred at 25°C for 2 hours. Water (50 mL) was added to the reaction mixture, and the mixture was extracted with ethyl acetate (50 mL × 3). The organic phases were combined, dried over anhydrous sodium sulfate, filtered, and concentrated under reduced pressure. The crude product was purified by silica gel column chromatography (dichloromethane/methanol, 20/1, v/v) to obtain compound **38-4.** ¹H NMR (400 MHz, DMSO-*d*₆): δ 12.89 (s, 1H), 7.51-7.43 (m, 2H), 7.23-7.18 (m, 1H), 4.54-4.46 (m, 1H), 3.70-3.64 (m, 1H), 3.55-3.46 (m, 1H), 3.29-3.22 (m, 2H), 2.20-2.13 (m, 1H), 1.98-1.91 (m, 1H), 1.35 (s, 9H). ESI-MS calculated for: [M+H]⁺ = 306.15, found 306.5.

### Step 4

Compound **38-4** (560 mg, 1.83 mmol) was dissolved in *N,N*-dimethylformamide (10 mL). Under a nitrogen atmosphere, sodium hydride (87.8 mg, 2.20 mmol, 60% purity) was slowly added at 0°C, and the mixture was stirred at 0°C for 0.5 hours. *p*-Toluenesulfonyl chloride (419 mg, 2.20 mmol) was added, and the mixture was stirred at 25°C for 2 hours. Saturated aqueous ammonium chloride solution (50 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **38-5.** ¹H NMR (400 MHz, DMSO-*d*₆): δ 8.10 (dd, J = 9.2, 4.0 Hz, 1H), 7.77 (d, J = 8.4 Hz, 2H), 7.64-7.59 (m, 1H), 7.57-7.51 (m, 1H), 7.37 (d, J = 8.4 Hz, 2H), 4.52-4.48 (m, 1H), 3.64-3.59 (m, 1H), 3.29-3.18 (m, 3H), 2.31 (s, 3H), 2.18-2.13 (m, 1H), 1.78-1.72 (m, 1H), 1.24 (s, 9H). ESI-MS calculated for: [M+H]⁺ = 460.16, found 460.2.

### Step 5

Compound **38-5** (630 mg, 1.37 mmol) was dissolved in 1,2-dichloromethane (6 mL). Trifluoroacetic acid (2.0 mL, 26.9 mmol) was slowly added at 0°C, and the mixture was stirred at 25°C for 2 hours. Saturated aqueous sodium bicarbonate solution (30 mL) was added to the reaction mixture, and the mixture was extracted with dichloromethane (30 mL × 3). The organic phases were combined and washed with saturated brine (30 mL). The organic phase was dried over anhydrous sodium sulfate, filtered, and concentrated under reduced pressure to obtain compound **38-6.** ESI-MS calculated for: [M+H]⁺ = 360.11, found 360.0.

### Step 6

Compound **38-6** (200 mg, 0.56 mmol) was dissolved in methanol (5 mL). *N,N-*Diisopropylethylamine (220 mg, 1.68 mmol) and paraformaldehyde (18.5 mg, 0.62 mmol) were added, and the mixture was stirred at 25°C for 0.5 hours. Sodium cyanoborohydride (35.5 mg, 0.57 mmol) was added, and the mixture was stirred at 25°C for 2 hours. Saturated aqueous sodium bicarbonate solution (30 mL) was added to the reaction mixture, and the mixture was extracted with ethyl acetate (30 mL × 3). The organic phases were combined, dried over anhydrous sodium sulfate, filtered, and concentrated under reduced pressure. The crude product was purified by silica gel column chromatography (dichloromethane/methanol, 20/1, v/v) to obtain compound **38-7.** ESI-MS calculated for: [M+H]⁺ = 374.13, found 374.0.

### Step 7

Compound **38-7** (150 mg, 0.40 mol) was dissolved in tetrahydrofuran (1 mL), methanol (1 mL), and water (0.4 mL). Lithium hydroxide monohydrate (83.9 mg, 2.00 mmol) was added, and the reaction mixture was stirred at 25°C for 16 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 20-30%, retention time: 8.3-9.1 min, run time: 17 min) to obtain compound **38.** ¹H NMR (400 MHz, DMSO-*d*₆): δ 12.78 (s, 1H), 7.52 (dd, J = 9.2, 2.4 Hz, 1H), 7.47 (dd, J = 9.2, 4.4 Hz, 1H), 7.21-7.17 (m, 1H), 3.27-3.20 (m, 2H), 3.10-3.05 (m, 1H), 3.00-2.96 (m, 1H), 2.63-2.58 (m, 1H), 2.08 (s, 3H), 1.94-1.83 (m, 2H). ESI-MS calculated for: [M+H]⁺ = 220.12, found 220.0.

### Example 39

### Synthetic route:

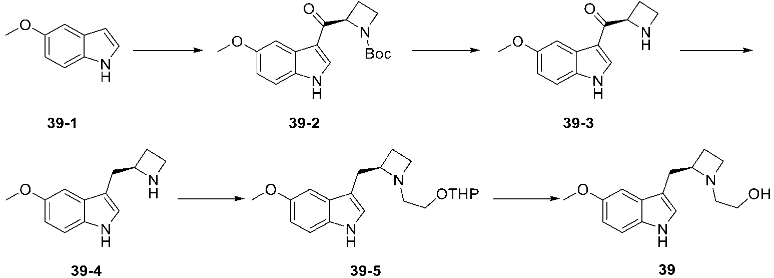

### Step 1

(*R*)-*N*-*tert*-Butoxycarbonyl-azetidine-2-carboxylic acid (1.98 g, 9.83 mmol) was dissolved in dichloromethane (30 mL). Oxalyl chloride (0.683 mL, 11.8 mmol) was added dropwise at 0°C, and the reaction mixture was stirred at 0°C for 10 minutes. *N,N-*Dimethylformamide (72.0 mg, 1.04 mmol) was slowly added, and the reaction mixture was stirred at 25°C for 1 hour. The reaction mixture was concentrated under reduced pressure to obtain a crude intermediate. Compound **39-1** (1.00 g, 6.79 mmol) was dissolved in dichloromethane (10 mL). Under a nitrogen atmosphere, ethylmagnesium bromide (3.23 mL, 6.45 mmol, 2 mol/L) was slowly added dropwise at 0°C, and the mixture was stirred at 0°C for 0.5 hours. The crude intermediate was dissolved in dichloromethane (20 mL) and added dropwise to the reaction mixture at 0°C. The reaction mixture was stirred at 0°C for 0.5 hours. Saturated aqueous citric acid solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (dichloromethane/ethyl acetate, 1/1, v/v) to obtain compound **39-2.** ESI-MS calculated for: [M+H]⁺ = 331.16, found: 331.0.

### Step 2

Compound **39-2** (2.99 g, 9.05 mmol) was dissolved in 1,2-dichloromethane (40 mL). Trifluoroacetic acid (10 mL) was slowly added at 0°C, and the mixture was stirred at 25°C for 1 hour. The reaction mixture was concentrated under reduced pressure to obtain a crude product containing the target compound. The crude product was purified by high-performance liquid chromatography (Waters-spherical-C18-20 µm, 100A, 330 g, mobile phase: acetonitrile-0.5% formic acid in water, gradient: 11-20%, retention time: 16-27 min, run time: 40 min) to obtain compound **39-3.** ¹H NMR (400 MHz, DMSO-*d*₆): δ 12.33 (s, 1H), 8.30 (d, J = 3.2 Hz, 1H), 7.69 (d, J = 2.4 Hz, 1H), 7.45 (d, J = 8.8 Hz, 1H), 6.92 (dd, J = 8.8, 2.4 Hz, 1H), 5.81-5.75 (m, 1H), 4.11-4.06 (m, 1H), 3.86-3.79 (m, 4H), 2.97-2.91 (m, 1H), 2.51-2.45 (m, 1H). ESI-MS calculated for: [M+H]⁺ = 231.11, found 231.0.

### Step 3

Compound **39-3** (3.00 g, 13.0 mmol) was dissolved in tetrahydrofuran (90 mL). A solution of lithium aluminum hydride in tetrahydrofuran (52.1 mL, 130 mmol, 2.5 mol/L) was added dropwise at 0°C. Under a nitrogen atmosphere, the reaction mixture was stirred at 60°C for 3 hours. The reaction mixture was cooled to room temperature, quenched by the addition of ice water (5.2 mL), and then 15% aqueous sodium hydroxide solution (5.2 mL) and water (15.6 mL) were added. The mixture was filtered, and the filtrate was concentrated under reduced pressure to obtain a crude product containing the target compound. The crude product was purified by high-performance liquid chromatography (Waters-spherical-C18-20 µm, 100A, 220 g, mobile phase: acetonitrile-10 mmol/L aqueous ammonium bicarbonate solution, gradient: 15-25%, retention time: 20-35 min, run time: 50 min) to obtain compound **39-4.** ESI-MS calculated for: [M+H]⁺ = 217.13, found 217.0.

### Step 4

Compound **39-4** (200 mg, 0.92 mmol) was dissolved in *N,N*-dimethylformamide (12 mL) and acetonitrile (12 mL). Potassium carbonate (381 mg, 2.76 mmol) and 2-(2-bromoethoxy)tetrahydropyran (577 mg, 2.76 mmol) were added, and the mixture was stirred at 25°C for 24 hours. Water (50 mL) was added to the reaction mixture, and the mixture was extracted with ethyl acetate (50 mL × 3). The organic phases were combined and washed with saturated brine (50 mL × 3). The organic phase was dried over anhydrous sodium sulfate, filtered, and concentrated under reduced pressure. The crude product was purified by silica gel column chromatography (petroleum ether/ethyl acetate, 1/1, v/v) to obtain compound **39-5.** ESI-MS calculated for: [M+H]⁺ = 345.21, found 345.1.

### Step 5

Compound **39-5** (150 mg, 0.44 mol) was dissolved in tetrahydrofuran (5 mL). An aqueous hydrochloric acid solution (0.88 mL, 0.88 mmol, 1 mol/L) was added, and the reaction mixture was stirred at 25°C for 3 hours. The reaction mixture was concentrated under reduced pressure to obtain a crude product containing the target compound. The crude product was purified by high-performance liquid chromatography (Waters-XBridge-C18-10 µm-19*250 mm, mobile phase: acetonitrile-0.04% aqueous ammonia +7.5 mmol/L aqueous ammonium bicarbonate solution, gradient: 30-40%, retention time: 6.0-7.8 min, run time: 17 min) to obtain compound **39.** ¹H NMR (400 MHz, DMSO-*d*₆): δ 10.59 (s, 1H), 7.19 (d, J = 8.8 Hz, 1H), 7.04 (d, J = 2.4 Hz, 1H), 6.99 (d, J = 2.4 Hz, 1H), 6.69 (dd, J = 8.8, 2.4 Hz, 1H), 4.33 (s, 1H), 3.75 (s, 3H), 3.36-3.32 (m, 2H), 3.29-3.25 (m, 2H), 2.94-2.86 (m, 1H), 2.72-2.65 (m, 2H), 2.58-2.54 (m, 1H), 2.33-2.29 (m, 1H), 1.95-1.89 (m, 1H), 1.85-1.75 (m, 1H). ESI-MS calculated for: [M+H]⁺ = 261.16, found 261.1.

### Example 40

### Synthetic route:

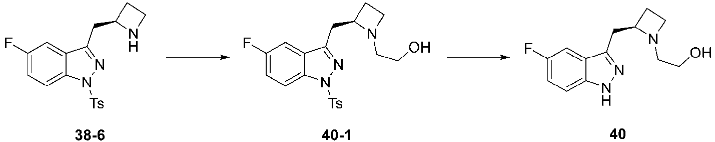

### Step 1

Compound **38-6** (400 mg, 1.11 mmol) was dissolved in *N,N 7*-dimethylformamide (5 mL). Potassium carbonate (460 mg, 3.33 mmol), 2-bromoethanol (416 mg, 3.33 mmol), and potassium iodide (18.4 mg, 0.11 mmol) were added, and the mixture was stirred at 70°C for 2 hours. Water (30 mL) was added to the reaction mixture, and the mixture was extracted with ethyl acetate (30 mL × 3). The organic phases were combined and washed with saturated brine (30 mL × 3). The organic phase was dried over anhydrous sodium sulfate, filtered, and concentrated under reduced pressure. The crude product was purified by silica gel column chromatography (dichloromethane/methanol, 10/1, v/v) to obtain compound **40-1.** ESI-MS calculated for: [M+H]⁺ = 404.14, found 404.0.

### Step 2

Compound **40-1** (220 mg, 0.55 mol) was dissolved in tetrahydrofuran (1 mL), methanol (1 mL), and water (0.4 mL). Lithium hydroxide monohydrate (115 mg, 2.75 mmol) was added, and the reaction mixture was stirred at 25°C for 16 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-0.1% formic acid in water, gradient: 5%-10%, retention time: 5.9-7.7 min, run time: 17 min) to obtain the monoformate salt of compound **40.** ¹H NMR (400 MHz, DMSO-*d*₆): δ 12.80 (s, 1H), 8.21 (s, 1H), 7.54 (dd, J = 9.2, 2.4 Hz, 1H), 7.48 (dd, J = 9.2, 4.4 Hz, 1H), 7.23-7.19 (m, 1H), 3.63-3.58 (m, 2H), 3.38-3.32 (m, 2H), 3.23-3.17 (m, 1H), 3.10-3.06 (m, 1H), 2.94-2.87 (m, 1H), 2.65-2.59 (m, 1H), 2.50-2.44 (m, 1H), 1.98-1.85 (m, 2H). ESI-MS calculated for: [M+H]⁺ = 250.13, found 250.0.

### Example 41

### Synthetic route:

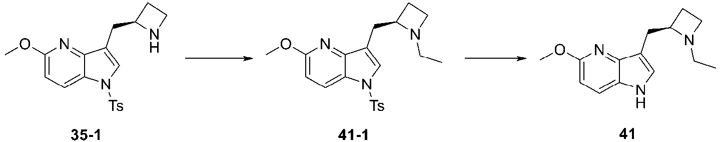

### Step 1

Compound **35-1** (250 mg, 0.67 mmol) was dissolved in methanol (5 mL). Diisopropylethylamine (260 mg, 2.01 mmol) and acetaldehyde (118 mg, 1.34 mmol) were added, and the mixture was stirred at 25°C for 0.5 hours. Sodium cyanoborohydride (42.5 mg, 0.68 mmol) was added, and the mixture was stirred at 25°C for 2 hours. Saturated aqueous sodium bicarbonate solution (20 mL) was added to the reaction mixture, and the mixture was extracted with ethyl acetate (20 mL × 3). The organic phases were combined and washed with saturated brine (20 mL). The organic phase was dried over anhydrous sodium sulfate, filtered, and concentrated under reduced pressure. The crude product was purified by silica gel column chromatography (dichloromethane/methanol, 10/1, v/v) to obtain compound **41-1.** ESI-MS calculated for: [M+H]⁺ = 400.16, found 400.6.

### Step 2

Compound **41-1** (160 mg, 0.40 mol) was dissolved in tetrahydrofuran (1.5 mL), methanol (1.5 mL), and water (0.6 mL). Lithium hydroxide monohydrate (83.9 mg, 2.00 mmol) was added, and the reaction mixture was stirred at 25°C for 16 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 17-30%, retention time: 7.2-9.0 min, run time: 17 min) to obtain compound **41.** ¹H NMR (400 MHz, DMSO-*d*₆): δ 10.81 (s, 1H), 7.61 (d, J = 8.8 Hz, 1H), 7.24 (d, J = 2.0 Hz, 1H), 6.50 (d, J = 8.4 Hz, 1H), 3.86 (s, 3H), 3.31-3.22 (m, 3H), 2.98-2.93 (m, 1H), 2.78-2.73 (m, 1H), 2.59-2.53 (m, 1H), 2.26-2.17 (m, 1H), 1.97-1.73 (m, 2H), 0.91 (t, J = 7.2 Hz, 3H). ESI-MS calculated for: [M+H]⁺ = 246.15, found 246.0.

### Example 42

### Synthetic route:

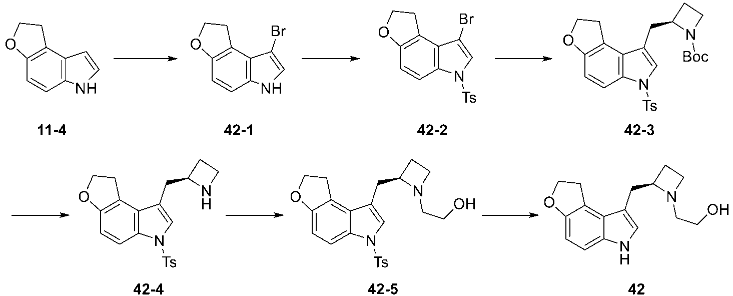

### Step 1

Compound **11-4** (1.60 g, 10.1 mmol) was dissolved in *N,N*-dimethylformamide (20 mL). Under a nitrogen atmosphere, *N*-bromosuccinimide (1.88 g, 10.6 mmol) was slowly added at 0°C, and the mixture was stirred at 25°C for 1 hour. Aqueous sodium bicarbonate solution (100 mL) was added to the reaction mixture, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **42-1.** ¹H NMR (400 MHz, DMSO-*d*₆): δ 11.26 (s, 1H), 7.45 (d, J = 2.4 Hz, 1H), 7.15 (d, J = 8.8 Hz, 1H), 6.68 (d, J = 8.8 Hz, 1H), 4.55 (t, J = 8.8 Hz, 2H), 3.51 (t, J = 8.8 Hz, 2H).

### Step 2

Compound **42-1** (2.19 g, 9.20 mmol) was dissolved in *N,N*-dimethylformamide (35 mL). Under a nitrogen atmosphere, sodium hydride (442 mg, 11.0 mmol, 60% purity) was slowly added at 0°C, and the mixture was stirred at 0°C for 0.5 hours. *p*-Toluenesulfonyl chloride (3.51 g, 18.4 mmol) was added, and the mixture was stirred at 25°C for 1 hour. Aqueous ammonium chloride solution (50 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/dichloromethane, 4/1, v/v) to obtain compound **42-2.** ¹H NMR (400 MHz, DMSO-*d*₆): δ 8.04 (s, 1H), 7.85 (d, J = 8.8 Hz, 2H), 7.72 (d, J = 8.8 Hz, 1H), 7.39 (d, J = 8.0 Hz, 2H), 6.88 (d, J = 8.8 Hz, 1H), 4.57 (t, J = 8.8 Hz, 2H), 3.49 (t, J = 8.8 Hz, 2H), 2.32 (s, 3H).

### Step 3

In a glove box filled with nitrogen, compound **42-2** (300 mg, 0.76 mmol), *(2R)-tert-*butyl 2-(bromomethyl)azetidine-1-carboxylate (285 mg, 1.14 mmol), anhydrous sodium carbonate (161 mg, 1.52 mmol), tris(trimethylsilyl)silane (283 mg, 1.14 mmol), nickel(II) chloride dimethoxyethane (8.35 mg, 0.038 mmol), 4,4'-di-*tert*-butyl-2,2'-dipyridine (10.2 mg, 0.038 mmol), and bis[2-(2,4-difluorophenyl)-5-trifluoromethylpyridine][2-2'-bi(4-tert-butylpyridine)]iridium(III) bis(hexafluorophosphate) (8.53 mg, 0.008 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (30 mL) was added to the reaction mixture, and the mixture was extracted with ethyl acetate (30 mL × 3). The organic phases were combined and washed with saturated brine (30 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **42-3.** ¹H NMR (400 MHz, DMSO-*d*₆): δ 7.74 (d, J = 8.4 Hz, 2H), 7.64 (d, J = 8.8 Hz, 1H), 7.56 (s, 1H), 7.34 (d, J = 8.0 Hz, 2H), 6.78 (d, J = 8.8 Hz, 1H), 4.55 (t, J = 8.8 Hz, 2H), 4.36-4.31 (m, 1H), 3.73-3.67 (m, 1H), 3.63-3.59 (m, 1H), 3.48-3.37 (m, 2H), 3.24-3.16 (m, 1H), 2.92-2.86 (m, 1H), 2.30 (s, 3H), 2.13-2.05 (m, 1H), 1.89-1.83 (m, 1H), 1.35 (s, 9H). ESI-MS calculated for: [M+H-100]⁺ = 383.19, found 383.1.

### Step 4

Compound **42-3** (377 mg, 0.78 mmol) was dissolved in dichloromethane (12 mL). Trifluoroacetic acid (4 mL) was slowly added at 0°C, and the mixture was stirred at 25°C for 2 hours. The reaction mixture was concentrated under reduced pressure. Saturated aqueous sodium bicarbonate solution (20 mL) was added to the residue, and the mixture was extracted with ethyl acetate (20 mL × 3). The organic phases were combined and washed with saturated brine (50 mL). The organic phase was dried over anhydrous sodium sulfate, filtered, and concentrated under reduced pressure to obtain compound **42-4.** ESI-MS calculated for: [M+H]⁺ = 383.14, found 383.0.

### Step 5

Compound **42-4** (317 mg, 0.83 mmol) was dissolved in *N,N*-dimethylformamide (2 mL). Potassium carbonate (344 mg, 2.49 mmol), 2-bromoethanol (311 mg, 2.49 mmol), and potassium iodide (13.8 mg, 0.083 mmol) were added, and the mixture was stirred at 70°C for 2 hours. Water (20 mL) was added to the reaction mixture, and the mixture was extracted with ethyl acetate (20 mL × 3). The organic phases were combined and washed with saturated brine (20 mL × 3). The organic phase was dried over anhydrous sodium sulfate, filtered, and concentrated under reduced pressure. The crude product was purified by silica gel column chromatography (dichloromethane/methanol, 10/1, v/v) to obtain compound **42-5.** ESI-MS calculated for: [M+H]⁺ = 427.16, found 427.0.

### Step 6

Compound **42-5** (150 mg, 0.35 mmol) was dissolved in anhydrous methanol (1.5 mL) and water (1.5 mL). Potassium hydroxide (196 mg, 3.50 mmol) was added, and the reaction mixture was stirred at 60°C for 4 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-0.04% aqueous ammonia solution +7.5 mol/L aqueous ammonium bicarbonate solution, gradient: 25-35%, retention time: 7.0-8.3 min, run time: 16 min) to obtain compound **42.** ¹H NMR (400 MHz, DMSO-*d*₆): δ 10.59 (s, 1H), 7.03 (d, J = 8.4 Hz, 2H), 6.56 (d, J = 8.4 Hz, 1H), 4.51 (t, J = 8.8 Hz, 2H), 4.32 (t, J = 5.2 Hz, 1H), 3.46 (t, J = 8.8 Hz, 2H), 3.27-3.19 (m, 4H), 3.03-2.95 (m, 1H), 2.75-2.63 (m, 2H), 2.59-2.52 (m, 1H), 2.33-2.25 (m, 1H), 1.96-1.89 (m, 1H), 1.82-1.71 (m, 1H). ESI-MS calculated for: [M+H]⁺ = 273.15, found 273.0.

### Example 43

### Synthetic route:

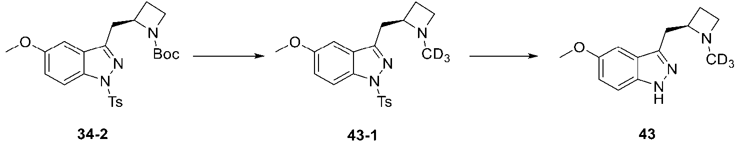

### Step 1

Compound **34-2** (167 mg, 0.35 mmol) was dissolved in tetrahydrofuran (16 mL). Lithium aluminum deuteride (118 mg, 2.80 mmol) was added at 0°C. Under a nitrogen atmosphere, the reaction mixture was stirred at 60°C for 1 hour. The reaction mixture was cooled to room temperature, quenched by the addition of ice water (1 mL), and then 15% aqueous sodium hydroxide solution (1 mL) and water (3 mL) were added. The mixture was filtered, and the filtrate was concentrated under reduced pressure to obtain compound **43-1.** ESI-MS calculated for: [M+H]⁺ = 389.16, found 389.1.

### Step 2

Compound **43-1** (130 mg, 0.33 mmol) was dissolved in tetrahydrofuran (3 mL), ethanol (3 mL), and water (1 mL). Lithium hydroxide monohydrate (69.2 mg, 1.65 mmol) was added, and the reaction mixture was stirred at 25°C for 16 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-0.1% formic acid in water, gradient: 8-18%, retention time: 7.0-9.5 min, run time: 17 min) to obtain the monoformate salt of compound **43.** ¹H NMR (400 MHz, DMSO-*d*₆): δ 12.51 (s, 1H), 8.24 (s, 1H), 7.35 (d, J = 8.8 Hz, 1H), 7.14 (d, J = 2.0 Hz, 1H), 6.96 (dd, J = 8.8, 2.0 Hz, 1H), 3.78 (s, 3H), 3.29-3.25 (m, 2H), 3.13-3.06 (m, 1H), 3.02-2.96 (m, 1H), 2.74-2.69 (m, 1H), 1.98-1.85 (m, 2H). ESI-MS calculated for: [M+H]⁺ = 235.16, found 235.0.

### Example 44

### Synthetic route:

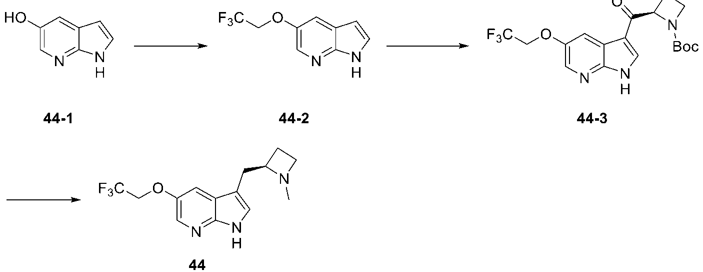

### Step 1

Compound **44-1** (5.00 g, 37.3 mmol) was dissolved in acetonitrile (50 mL). Potassium carbonate (7.73 g, 55.9 mmol) and 2,2,2-trifluoroethyl trifluoromethanesulfonate (13.0 g, 55.9 mmol) were slowly added, and the mixture was stirred at 60°C for 2 hours. Water (80 mL) was added to the reaction mixture, and the mixture was extracted with ethyl acetate (80 mL × 3). The organic phases were combined and washed with saturated brine (80 mL). The organic phase was dried over anhydrous sodium sulfate, filtered, and concentrated under reduced pressure. The crude product was purified by silica gel column chromatography (petroleum ether/ethyl acetate, 5/1, v/v) to obtain compound **44-2.** ¹H NMR (400 MHz, DMSO-*d*₆): δ 11.59 (s, 1H), 8.05 (d, J = 2.8 Hz, 1H), 7.69 (d, J = 2.8 Hz, 1H), 7.48 (d, J = 3.2 Hz, 1H), 6.39 (d, J = 3.2 Hz, 1H), 4.79 (q, J = 9.2 Hz, 2H). ESI-MS calculated for: [M+H]⁺ = 217.05, found 217.1.

### Step 2

(*R*)-*N*-*tert*-Butoxycarbonyl-azetidine-2-carboxylic acid (1.98 g, 9.83 mmol) was dissolved in dichloromethane (30 mL). Oxalyl chloride (0.683 mL, 11.8 mmol) was added dropwise at 0°C, and the reaction mixture was stirred at 0°C for 10 minutes. *N,N-*Dimethylformamide (72.0 mg, 1.04 mmol) was slowly added, and the reaction mixture was stirred at 25°C for 1 hour. The reaction mixture was concentrated under reduced pressure to obtain a crude intermediate. Compound **44-2** (2.00 g, 9.25 mmol) was dissolved in dichloromethane (20 mL). Under a nitrogen atmosphere, ethylmagnesium bromide (4.86 mL, 9.71 mmol, 2 mol/L) was slowly added dropwise at 0°C, and the mixture was stirred at 0°C for 0.5 hours. The crude intermediate was dissolved in dichloromethane (20 mL) and added dropwise to the reaction mixture at 0°C. The reaction mixture was stirred at 0°C for 0.5 hours. Saturated aqueous citric acid solution (30 mL) was added to the reaction mixture, and the mixture was extracted with dichloromethane (30 mL × 3). The organic phases were combined and washed with saturated brine (50 mL). The organic phase was dried over anhydrous sodium sulfate, filtered, and concentrated under reduced pressure. The crude product was purified by silica gel column chromatography (dichloromethane/ethyl acetate, 1/1, v/v) to obtain compound **44-3.** ESI-MS calculated for: [M+H]⁺ = 400.14, found: 400.0.

### Step 3

Compound **44-3** (230 mg, 0.58 mmol) was dissolved in tetrahydrofuran (3 mL). A solution of lithium aluminum hydride in tetrahydrofuran (2.32 mL, 5.80 mmol, 2.5 mol/L) was added dropwise at 0°C. Under a nitrogen atmosphere, the reaction mixture was stirred at 60°C for 8 hours. The reaction mixture was cooled to room temperature, quenched by the addition of ice water (0.23 mL), and then 15% aqueous sodium hydroxide solution (0.23 mL) and water (0.69 mL) were added. The mixture was filtered, and the filtrate was concentrated under reduced pressure. The crude product was purified by high-performance liquid chromatography (Waters-SunFire-C18-10 µm-19*250 mm, mobile phase: acetonitrile-10 mmol/L aqueous ammonium bicarbonate solution, gradient: 25-35%, retention time: 8.6-10.0 min, run time: 16 min) to obtain compound **44.** ¹H NMR (400 MHz, DMSO-*d*₆): δ 11.30 (s, 1H), 8.01 (d, J = 2.8 Hz, 1H), 7.71 (d, J = 2.8 Hz, 1H), 7.25 (d, J = 2.4 Hz, 1H), 4.80 (q, J = 9.2 Hz, 2H), 3.27-3.24 (m, 1H), 3.14-3.10 (m, 1H), 2.90-2.84 (m, 1H), 2.78-2.69 (m, 1H), 2.64-2.57 (m, 1H), 2.10 (s, 3H), 1.96-1.88 (m, 1H), 1.85-1.74 (m, 1H). ESI-MS calculated for: [M+H]⁺ = 300.12, found 300.0.

### Example 45

### Synthetic route:

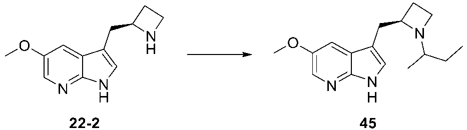

### Step 1

Compound **22-2** (250 mg, 1.15 mmol) was dissolved in methanol (5 mL). Diisopropylethylamine (450 mg, 3.45 mmol) and 2-butanone (415 mg, 5.75 mmol) were added to the reaction mixture, and the mixture was stirred at 25°C for 0.5 hours. Sodium cyanoborohydride (108 mg, 1.72 mmol) was added, and the reaction mixture was stirred at 25°C for 2 hours. Saturated aqueous sodium bicarbonate solution (30 mL) was added to the reaction mixture, and the mixture was extracted with dichloromethane (30 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-0.5% aqueous ammonia solution, gradient: 30-50%, retention time: 8.5-10.0 min, run time: 17 min) to obtain compound **45.** ¹H NMR (400 MHz, DMSO-*d*₆): δ 11.17 (s, 1H), 7.92 (d, J = 2.0 Hz, 1H), 7.48 (d, J = 2.0 Hz, 1H), 7.20 (s, 1H), 3.82 (s, 3H), 3.27-3.15 (m, 2H), 3.00-2.81 (m, 1H), 2.82-2.74 (m, 1H), 2.68-2.61 (m, 1H), 2.24-2.15 (m, 1H), 1.84-1.76 (m, 1H), 1.72-1.66 (m, 1H), 1.48-1.31 (m, 1H), 1.26-1.04 (m, 1H), 1.02-0.80 (m, 6H). ESI-MS calculated for: [M+H]⁺ = 274.18, found 274.0.

### Example 46

### Synthetic route:

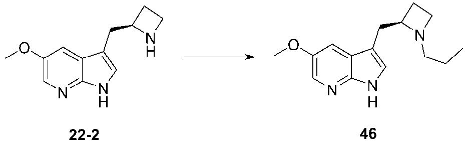

### Step 1

Compound **22-2** (248 mg, 1.14 mmol) was dissolved in methanol (5 mL). Diisopropylethylamine (440 mg, 3.42 mmol) and propionaldehyde (415 mg, 5.75 mmol) were added to the reaction mixture, and the mixture was stirred at 25°C for 0.5 hours. Sodium cyanoborohydride (108 mg, 1.72 mmol) was added, and the reaction mixture was stirred at 25°C for 2 hours. Saturated aqueous sodium bicarbonate solution (30 mL) was added to the reaction mixture, and the mixture was extracted with dichloromethane (30 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L ammonium bicarbonate + 0.04% aqueous ammonia solution, gradient: 26-36%, retention time: 8.7-10.8 min, run time: 17 min) to obtain compound **46.** ¹H NMR (400 MHz, DMSO-*d*₆): δ 11.16 (s, 1H), 7.92 (d, J = 2.0 Hz, 1H), 7.51 (d, J = 2.0 Hz, 1H), 7.18 (s, 1H), 3.82 (s, 3H), 3.25-3.17 (m, 2H), 2.91-2.86 (m, 1H), 2.77-2.72 (m, 1H), 2.60-2.54 (m, 1H), 2.43-2.36 (m, 1H), 2.17-2.05 (m, 1H), 1.93-1.88 (m, 1H), 1.82-1.75 (m, 1H), 1.30-1.25 (m, 2H), 0.81 (t, J = 7.2 Hz, 3H). ESI-MS calculated for: [M+H]⁺ = 260.17, found 260.1.

### Example 47

### Synthetic route:

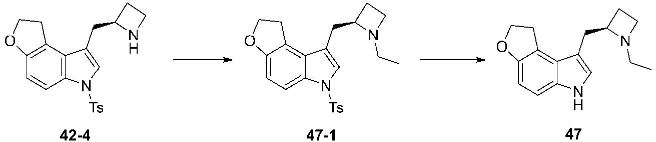

### Step 1

Compound **42-4** (205 mg, 0.54 mmol) was dissolved in *N,N*-dimethylformamide (5 mL). *N,N*-Diisopropylethylamine (210 mg, 1.62 mmol) and acetaldehyde (48.1 mg, 1.08 mmol) were added, and the mixture was stirred at 25°C for 0.5 hours. Sodium cyanoborohydride (34.3 mg, 0.55 mmol) was added, and the mixture was stirred at 25°C for 2 hours. Saturated aqueous sodium bicarbonate solution (20 mL) was added to the reaction mixture, and the mixture was extracted with ethyl acetate (20 mL × 3). The organic phases were combined and washed with saturated brine (20 mL × 3). The organic phase was dried over anhydrous sodium sulfate, filtered, and concentrated under reduced pressure. The crude product was purified by silica gel column chromatography (dichloromethane/methanol, 20/1, v/v) to obtain compound **47-1.** ESI-MS calculated for: [M+H]⁺ = 411.17, found 411.0.

### Step 2

Compound **47-1** (90.0 mg, 0.22 mmol) was dissolved in anhydrous methanol (1.5 mL) and water (1.5 mL). Potassium hydroxide (123 mg, 2.20 mmol) was added, and the reaction mixture was stirred at 60°C for 4 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 20-35%, retention time: 8.0-9.7 min, run time: 17 min) to obtain compound **47.** ¹H NMR (400 MHz, DMSO-*d*₆): δ 10.59 (s, 1H), 7.08-6.98 (m, 2H), 6.56 (d, J = 8.4 Hz, 1H), 4.51 (t, J = 8.8 Hz, 2H), 3.46 (t, J = 8.8 Hz, 2H), 3.26-3.21 (m, 1H), 3.15-3.08 (m, 1H), 3.04-2.96 (m, 1H), 2.75-2.70 (m, 1H), 2.58-2.52 (m, 1H), 2.50-2.43 (m, 1H), 2.21-2.12 (m, 1H), 1.93-1.86 (m, 1H), 1.78-1.69 (m, 1H), 0.86 (t, J = 7.2 Hz, 3H). ESI-MS calculated for: [M+H]⁺ = 257.16, found 257.0.

### Example 48

### Synthetic route:

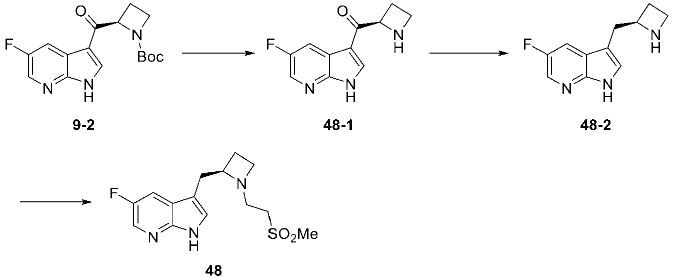

### Step 1

Compound **9-2** (5.00 g, 15.7 mmol) was dissolved in 1,2-dichloromethane (50 mL). Trifluoroacetic acid (17 mL) was slowly added at 0°C, and the mixture was stirred at 25°C for 1 hour. The reaction mixture was concentrated under reduced pressure to obtain a crude product containing the target compound. The crude product was purified by high-performance liquid chromatography (Waters-spherical-C18-20 µm, 100A, 330 g, mobile phase: acetonitrile-0.5% formic acid in water, gradient: 11-20%, retention time: 16-27 min, run time: 40 min) to obtain compound **48-1.** ¹H NMR (400 MHz, DMSO-*d*₆): δ 13.11 (s, 1H), 9.21 (s, 1H), 8.62 (s, 1H), 8.44-8.42 (m, 1H), 8.25 (dd, J = 9.2, 2.8 Hz, 1H), 5.82 (t, J = 8.0 Hz, 1H), 4.12-4.06 (m, 1H), 3.85-3.80 (m, 1H), 2.99-2.94 (m, 1H), 2.56-2.53 (m, 1H). ESI-MS calculated for: [M+H]⁺ = 220.08, found 220.0.

### Step 2

Compound **48-1** (4.80 g, 21.9 mmol) was dissolved in tetrahydrofuran (90 mL). A solution of lithium aluminum hydride in tetrahydrofuran (87.6 mL, 219 mmol, 2.5 mol/L) was added dropwise at 0°C. Under a nitrogen atmosphere, the reaction mixture was stirred at 60°C for 6 hours. The reaction mixture was cooled to room temperature, quenched by the addition of ice water (8.4 mL), and then 15% aqueous sodium hydroxide solution (8.4 mL) and water (25.2 mL) were added. The mixture was filtered, and the filtrate was concentrated under reduced pressure. The crude product was purified by high-performance liquid chromatography (Waters-spherical-C18-20 µm, 100A, 220 g, mobile phase: acetonitrile-10 mmol/L aqueous ammonium bicarbonate solution, gradient: 7.8-25%, retention time: 15-35 min, run time: 50 min) to obtain compound **48-2.** ESI-MS calculated for: [M+H]⁺ = 206.10, found 206.1.

### Step 3

Compound **48-2** (114 mg, 0.56 mmol) was dissolved in ethanol (3.0 mL). Diisopropylethylamine (217 mg, 1.68 mmol) and methyl vinyl sulfone (178 mg, 1.68 mmol) were added, and the mixture was stirred at 25°C for 16 hours. The reaction mixture was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 25-35%, retention time: 7.4-8.2 min, run time: 17 min) to obtain compound **48.** ¹H NMR (400 MHz, DMSO-*d*₆): δ 11.51 (s, 1H), 8.15-8.13 (m, 1H), 7.89 (dd, J = 9.2, 2.8 Hz, 1H), 7.36 (d, J = 2.4 Hz, 1H), 3.29-3.26 (m, 2H), 3.13-3.04 (m, 2H), 3.01 (s, 3H), 2.96-2.85 (m, 2H), 2.77-2.66 (m, 2H), 2.61-2.54 (m, 1H), 1.94-1.87 (m, 1H), 1.81-1.75 (m, 1H). ESI-MS calculated for: [M+H]⁺ = 312.11, found 312.0.

### Example 49

### Synthetic route:

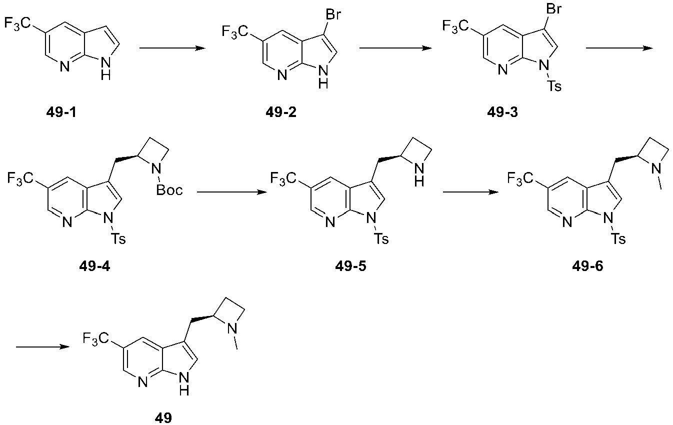

### Step 1

Compound **49-1** (2.50 g, 13.4 mmol) was dissolved in *N,N*-dimethylformamide (25 mL). Under a nitrogen atmosphere, *N*-bromosuccinimide (2.51 g, 14.1 mmol) was slowly added at 0°C, and the mixture was stirred at 25°C for 1 hour. Aqueous sodium bicarbonate solution (100 mL) was added to the reaction mixture, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **49-2.** ESI-MS calculated for: [M+H]⁺ = 264.95 and 266.95, found 264.9 and 266.9.

### Step 2

Compound **49-2** (3.00 g, 11.3 mmol) was dissolved in *N,N*-dimethylformamide (30 mL). Under a nitrogen atmosphere, sodium hydride (543 mg, 13.6 mmol, 60% purity) was slowly added at 0°C, and the mixture was stirred at 0°C for 0.5 hours. *p*-Toluenesulfonyl chloride (4.32 g, 22.6 mmol) was added, and the mixture was stirred at 25°C for 0.5 hours. Aqueous ammonium chloride solution (50 mL) was added to the reaction mixture, and the mixture was extracted with ethyl acetate (50 mL × 3). The organic phases were combined and washed with saturated brine (50 mL × 3). The organic phase was dried over anhydrous sodium sulfate, filtered, and concentrated under reduced pressure. The crude product was purified by silica gel column chromatography (petroleum ether/dichloromethane, 4/1, v/v) to obtain compound **49-3.** ¹H NMR (400 MHz, DMSO-*d*₆): δ 8.86 (s, 1H), 8.45 (s, 1H), 8.34 (s, 1H), 8.05 (d, J = 8.4 Hz, 2H), 7.46 (d, J = 8.4 Hz, 2H), 2.36 (s, 3H).

### Step 3

In a glove box filled with nitrogen, compound **49-3** (200 mg, 0.48 mmol), *(2R)-tert-*butyl 2-(bromomethyl)azetidine-1-carboxylate (180 mg, 0.72 mmol), anhydrous sodium carbonate (110 mg, 0.96 mmol), tris(trimethylsilyl)silane (179 mg, 0.72 mmol), nickel(II) chloride dimethoxyethane (5.27 mg, 0.024 mmol), 4,4'-di-*tert*-butyl-2,2'-dipyridine (6.44 mg, 0.024 mmol), and bis[2-(2,4-difluorophenyl)-5-trifluoromethylpyridine][2-2'-bi(4-tert-butylpyridine)]iridium(III) bis(hexafluorophosphate) (5.39 mg, 0.005 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (30 mL) was added to the reaction mixture, and the mixture was extracted with ethyl acetate (30 mL × 3). The organic phases were combined and washed with saturated brine (30 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **49-4.** ESI-MS calculated for: [M+H]⁺ = 510.16, found 510.1.

### Step 4

Compound **49-4** (310 mg, 0.61 mmol) was dissolved in dichloromethane (5 mL). Trifluoroacetic acid (1 mL) was slowly added at 0°C, and the mixture was stirred at 25°C for 2 hours. The reaction mixture was concentrated under reduced pressure. Saturated aqueous sodium bicarbonate solution (20 mL) was added to the residue, and the mixture was extracted with ethyl acetate (20 mL × 3). The organic phases were combined and washed with saturated brine (20 mL). The organic phase was dried over anhydrous sodium sulfate, filtered, and concentrated under reduced pressure to obtain compound **49-5.** ESI-MS calculated for: [M+H]⁺ = 410.11, found 410.0.

### Step 5

Compound **49-5** (290 mg, 0.71 mmol) was dissolved in methanol (5 mL). *N,N-*Diisopropylethylamine (280 mg, 2.13 mmol) and paraformaldehyde (23.5 mg, 0.78 mmol) were added, and the mixture was stirred at 25°C for 0.5 hours. Sodium cyanoborohydride (45.1 mg, 0.72 mmol) was added, and the mixture was stirred at 25°C for 2 hours. Water (20 mL) was added to the reaction mixture, and the mixture was extracted with ethyl acetate (20 mL × 3). The organic phase was dried over anhydrous sodium sulfate, filtered, and concentrated under reduced pressure. The crude product was purified by silica gel column chromatography (dichloromethane/methanol, 20/1, v/v) to obtain compound **49-6.** ESI-MS calculated for: [M+H]⁺ = 424.12, found 424.1.

### Step 6

Compound **49-6** (80.0 mg, 0.19 mmol) was dissolved in tetrahydrofuran (0.5 mL), methanol (0.5 mL), and water (0.5 mL). Sodium hydroxide (38.0 mg, 0.95 mmol) was added, and the reaction mixture was stirred at 25°C for 16 hours. The reaction mixture was filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 20-35%, retention time: 8.0-9.7 min, run time: 17 min) to obtain compound **49.** ¹H NMR (400 MHz, DMSO-*d*₆): δ 11.98 (s, 1H), 8.51 (d, J = 2.0 Hz, 1H), 8.36 (d, J = 2.0 Hz, 1H), 7.46 (s, 1H), 3.23-3.20 (m, 1H), 3.15-3.09 (m, 1H), 2.96-2.90 (m, 1H), 2.84-2.78 (m, 1H), 2.63-2.56 (m, 1H), 2.07 (s, 3H), 1.95-1.87 (m, 1H), 1.81-1.74 (m, 1H). ESI-MS calculated for: [M+H]⁺ = 270.11, found 269.9.

### Example 50

### Synthetic route:

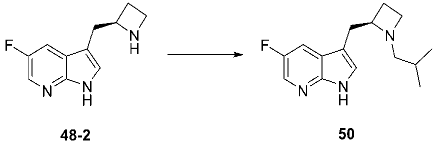

### Step 1

Compound **48-2** (150 mg, 0.58 mmol) was dissolved in methanol (1.5 mL). Diisopropylethylamine (225 mg, 1.74 mmol) and isobutyraldehyde (125 mg, 1.74 mmol) were added to the reaction mixture, and the mixture was stirred at 25°C for 0.5 hours. Sodium cyanoborohydride (36.5 mg, 0.58 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 30-45%, retention time: 8.6-10.8 min, run time: 17 min) to obtain compound **50.** ¹H NMR (400 MHz, DMSO-*d*₆): δ 11.48 (s, 1H), 8.14-8.13 (m, 1H), 7.84-7.81 (m, 1H), 7.33 (d, J = 2.4 Hz, 1H), 3.25-3.17 (m, 2H), 2.88-2.82 (m, 1H), 2.77-2.70 (m, 1H), 2.60-2.54 (m, 1H), 2.20-2.14 (m, 1H), 2.03-1.98 (m, 1H), 1.90-1.85 (m, 1H), 1.79-1.73 (m, 1H), 1.53-1.47 (m, 1H), 0.82-0.77 (m, 6H). ESI-MS calculated for: [M+H]⁺ = 262.16, found 262.1.

### Example 51

### Synthetic route:

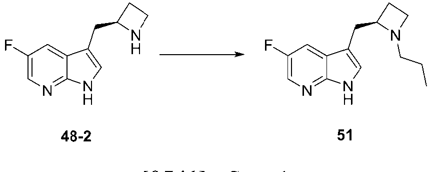

### Step 1

Compound **48-2** (150 mg, 0.58 mmol) was dissolved in methanol (1.5 mL). Diisopropylethylamine (225 mg, 1.74 mmol) and propionaldehyde (101 mg, 1.74 mmol) were added to the reaction mixture, and the mixture was stirred at 25°C for 0.5 hours. Sodium cyanoborohydride (36.5 mg, 0.58 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 25-35%, retention time: 7.7-9.8 min, run time: 17 min) to obtain compound **51.** ¹H NMR (400 MHz, DMSO-*d*₆): δ 11.49 (s, 1H), 8.15-8.13 (m, 1H), 7.83 (dd, J = 9.6, 2.4 Hz, 1H), 7.33 (d, J = 2.4 Hz, 1H), 3.25-3.17 (m, 2H), 2.91-2.85 (m, 1H), 2.78-2.71 (m, 1H), 2.60-2.53 (m, 1H), 2.41-2.34 (m, 1H), 2.14-2.08 (m, 1H), 1.92-1.85 (m, 1H), 1.78-1.72 (m, 1H), 1.30-1.24 (m, 2H), 0.81 (t, J = 7.2 Hz, 3H). ESI-MS calculated for: [M+H]⁺ = 248.15, found 248.0.

### Example 52

### Synthetic route:

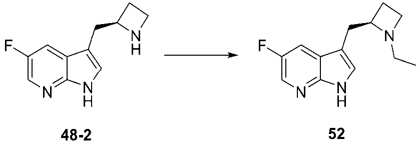

### Step 1

Compound **48-2** (150 mg, 0.58 mmol) was dissolved in methanol (2 mL). Diisopropylethylamine (225 mg, 1.74 mmol) and acetaldehyde (76.7 mg, 1.74 mmol) were added to the reaction mixture, and the mixture was stirred at 25°C for 0.5 hours. Sodium cyanoborohydride (36.5 mg, 0.58 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 20-30%, retention time: 7.4-9.2 min, run time: 17 min) to obtain compound **52.** ¹H NMR (400 MHz, DMSO-*d*₆): δ 11.48 (s, 1H), 8.14-8.13 (m, 1H), 7.83 (dd, J = 9.6, 2.4 Hz, 1H), 7.33 (d, J = 2.4 Hz, 1H), 3.24-3.16 (m, 2H), 2.91-2.86 (m, 1H), 2.78-2.72 (m, 1H), 2.58-2.52 (m, 1H), 2.48-2.41 (m, 1H), 2.17-2.13 (m, 1H), 1.90-1.86 (m, 1H), 1.79-1.72 (m, 1H), 0.84 (t, J = 7.2 Hz, 3H). ESI-MS calculated for: [M+H]⁺ = 234.13, found 234.0.

### Example 53

### Synthetic route:

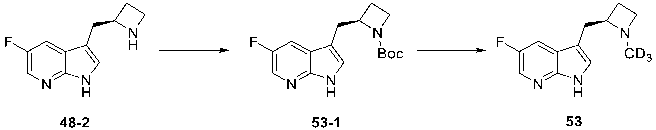

### Step 1

Compound **48-2** (800 mg, 3.12 mmol) was dissolved in 1,2-dichloromethane (8 mL). Triethylamine (950 mg, 9.36 mmol) and di-*tert*-butyl dicarbonate (820 mg, 3.74 mmol) were added, and the mixture was stirred at 25°C for 1 hour. Saturated aqueous ammonium chloride solution (40 mL) was added to the reaction mixture, and the mixture was extracted with dichloromethane (30 mL × 3). The organic phases were combined and washed with saturated brine (60 mL). The organic phase was dried over anhydrous sodium sulfate, filtered, and concentrated under reduced pressure. The crude product was purified by silica gel column chromatography (petroleum ether/dichloromethane, 3/1, v/v) to obtain compound **53-1.** ¹H NMR (400 MHz, DMSO-*d*₆): δ 8.56 (s, 1H), 8.18-8.17 (m, 1H), 7.63 (d, J = 7.6 Hz, 1H), 7.18 (d, J = 2.4 Hz, 1H), 4.52-4.47 (m, 1H), 3.80-3.75 (m, 1H), 3.62-3.58 (m, 1H), 3.23-3.15 (m, 2H), 2.19-2.14 (m, 1H), 1.90-1.82 (m, 1H), 1.46 (s, 9H). ESI-MS calculated for: [M+H]⁺ = 306.15, found 306.0.

### Step 2

Compound **53-1** (90.0 mg, 0.25 mmol) was dissolved in tetrahydrofuran (9 mL). Lithium aluminum deuteride (84.0 mg, 2.00 mmol) was added portionwise at 0°C. Under a nitrogen atmosphere, the reaction mixture was stirred at 60°C for 1 hour. The reaction mixture was cooled to room temperature, quenched by the addition of ice water (0.8 mL), and then 15% aqueous sodium hydroxide solution (0.8 mL) and water (2.4 mL) were added. The mixture was filtered, and the filtrate was concentrated under reduced pressure. The crude product was purified by high-performance liquid chromatography (Waters-XBridge-C18-10 µm-19*250 mm, mobile phase: acetonitrile-10 mmol/L aqueous ammonium bicarbonate solution, gradient: 15-25%, retention time: 8.6-9.9 min, run time: 17 min) to obtain compound **53.** ¹H NMR (400 MHz, DMSO-*d*₆): δ 11.49 (s, 1H), 8.15-8.14 (m, 1H), 7.83 (dd, J = 9.6, 2.4 Hz, 1H), 7.33 (d, J = 2.4 Hz, 1H), 3.22-3.19 (m, 1H), 3.12-3.07 (m, 1H), 2.88-2.83 (m, 1H), 2.76-2.71 (m, 1H), 2.63-2.57 (m, 1H), 1.94-1.87 (m, 1H), 1.81-1.74 (m, 1H). ESI-MS calculated for: [M+H]⁺ = 223.14, found 223.0.

### Example 54

### Synthetic route:

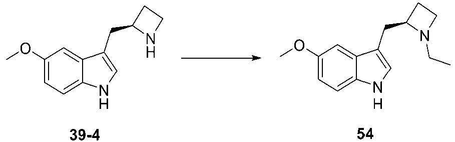

### Step 1

Compound **39-4** (150 mg, 0.69 mmol) was dissolved in methanol (1.5 mL). Diisopropylethylamine (268 mg, 2.07 mmol) and acetaldehyde (91.2 mg, 2.07 mmol) were added to the reaction mixture, and the mixture was stirred at 25°C for 0.5 hours. Sodium cyanoborohydride (43.4 mg, 0.69 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 28-38%, retention time: 8.5-9.7 min, run time: 17 min) to obtain compound **54.** ¹H NMR (400 MHz, DMSO-*d*₆): δ 10.59 (s, 1H), 7.19 (d, J = 8.8 Hz, 1H), 7.04 (d, J = 2.4 Hz, 1H), 6.98 (d, J = 2.4 Hz, 1H), 6.69 (dd, J = 8.8, 2.4 Hz, 1H), 3.75 (s, 3H), 3.25-3.16 (m, 2H), 2.92-2.87 (m, 1H), 2.75-2.70 (m, 1H), 2.58-2.51 (m, 2H), 2.18-2.14 (m, 1H), 1.93-1.90 (m, 1H), 1.78-1.74 (m, 1H), 0.87 (t, J = 7.2 Hz, 3H). ESI-MS calculated for: [M+H]⁺ = 245.16, found 245.1.

### Example 55

### Synthetic route:

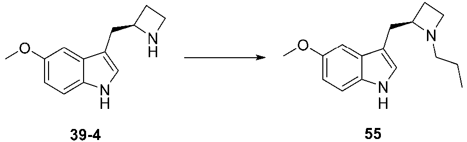

### Step 1

Compound **39-4** (150 mg, 0.69 mmol) was dissolved in methanol (1.5 mL). Diisopropylethylamine (268 mg, 2.07 mmol) and propionaldehyde (120 mg, 2.07 mmol) were added to the reaction mixture, and the mixture was stirred at 25°C for 0.5 hours. Sodium cyanoborohydride (43.4 mg, 0.69 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 32-42%, retention time: 8.0-9.0 min, run time: 17 min) to obtain compound **55.** ¹H NMR (400 MHz, DMSO-*d*₆): δ 10.59 (s, 1H), 7.20 (d, J = 8.8 Hz, 1H), 7.04 (d, J = 2.4 Hz, 1H), 6.98 (d, J = 2.4 Hz, 1H), 6.69 (dd, J = 8.8, 2.4 Hz, 1H), 3.75 (s, 3H), 3.25-3.16 (m, 2H), 2.91-2.87 (m, 1H), 2.75-2.70 (m, 1H), 2.58-2.51 (m, 1H), 2.49-2.40 (m, 1H), 2.16-2.07 (m, 1H), 1.92-1.90 (m, 1H), 1.79-1.75 (m, 1H), 1.32-1.26 (m, 2H), 0.82 (t, J = 7.2 Hz, 3H). ESI-MS calculated for: [M+H]⁺ = 259.17, found 259.1.

### Example 56

### Synthetic route:

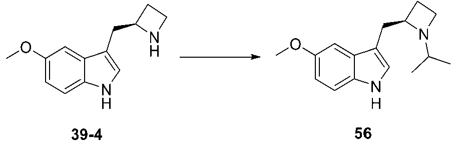

### Step 1

Compound **39-4** (100 mg, 0.46 mmol) was dissolved in acetonitrile (3 mL). Potassium carbonate (191 mg, 1.38 mmol) and iodopropane (157 mg, 0.92 mmol) were added to the reaction mixture, and the mixture was stirred at 70°C for 2 hours. The reaction mixture was cooled to room temperature. Water (20 mL) was added, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 25-40%, retention time: 7.3-8.9 min, run time: 17 min) to obtain compound **56.** ¹H NMR (400 MHz, DMSO-*d*₆): δ 10.60 (s, 1H), 7.20 (d, J = 8.8 Hz, 1H), 7.05 (d, J = 2.4 Hz, 1H), 6.95 (d, J = 2.4 Hz, 1H), 6.70 (dd, J = 8.8, 2.4 Hz, 1H), 3.75 (s, 3H), 3.29-3.18 (m, 2H), 2.96-2.91 (m, 1H), 2.80-2.73 (m, 1H), 2.65-2.59 (m, 1H), 2.40-2.36 (m, 1H), 1.85-1.82 (m, 1H), 1.70-1.68 (m, 1H), 1.02 (d, J = 6.4 Hz, 3H), 0.85 (d, J = 6.4 Hz, 3H). ESI-MS calculated for: [M+H]⁺ = 259.17, found 259.1.

### Example 57

### Synthetic route:

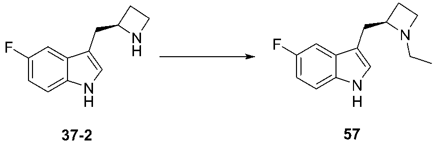

### Step 1

Compound **37-2** (150 mg, 0.73 mmol) was dissolved in methanol (3 mL). Diisopropylethylamine (377 mg, 2.92 mmol) and acetaldehyde (161 mg, 3.65 mmol) were added to the reaction mixture, and the mixture was stirred at 25°C for 0.5 hours. Sodium cyanoborohydride (45.9 mg, 0.73 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 23-35%, retention time: 6.9-8.2 min, run time: 17 min) to obtain compound **57.** ¹H NMR (400 MHz, DMSO-*d*₆): δ 10.87 (s, 1H), 7.31-7.28 (m, 1H), 7.24-7.21 (m, 1H), 7.17 (d, J = 4.0 Hz, 1H), 6.90-6.85 (m, 1H), 3.25-3.21 (m, 1H), 3.18-3.15 (m, 1H), 2.92-2.87 (m, 1H), 2.75-2.70 (m, 1H), 2.57-2.52 (m, 1H), 2.48-2.44 (m, 1H), 2.18-2.13 (m, 1H), 1.92-1.86 (m, 1H), 1.79-1.72 (m, 1H), 0.86 (t, J = 6.4 Hz, 3H). ESI-MS calculated for: [M+H]⁺ = 233.14, found 233.0.

### Example 58

### Synthetic route:

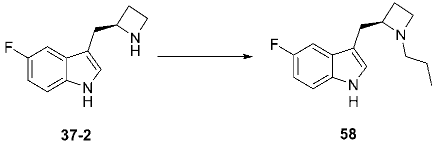

### Step 1

Compound **37-2** (150 mg, 0.73 mmol) was dissolved in methanol (3 mL). Diisopropylethylamine (377 mg, 2.92 mmol) and propionaldehyde (127 mg, 2.19 mmol) were added to the reaction mixture, and the mixture was stirred at 25°C for 0.5 hours. Sodium cyanoborohydride (45.9 mg, 0.73 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 32-42%, retention time: 8.0-9.0 min, run time: 17 min) to obtain compound **58.** ¹H NMR (400 MHz, DMSO-*d*₆): δ 10.88 (s, 1H), 7.31-7.28 (m, 1H), 7.29-7.24 (m, 1H), 7.17 (d, J = 4.0 Hz, 1H), 6.90-6.85 (m, 1H), 3.25-3.21 (m, 1H), 3.17-3.13 (m, 1H), 2.91-2.86 (m, 1H), 2.75-2.70 (m, 1H), 2.57-2.53 (m, 1H), 2.42-2.37 (m, 1H), 2.14-2.09 (m, 1H), 1.90-1.87 (m, 1H), 1.77-1.73 (m, 1H), 1.30-1.23 (m, 2H), 0.82 (t, J = 6.4 Hz, 3H). ESI-MS calculated for: [M+H]⁺ = 247.15, found 247.0.

### Example 59

### Synthetic route:

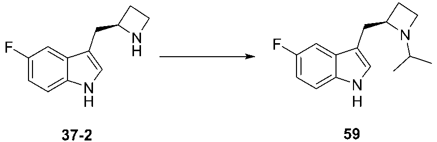

### Step 1

Compound **37-2** (120 mg, 0.59 mmol) was dissolved in acetonitrile (3 mL). Potassium carbonate (245 mg, 1.77 mmol) and 2-iodopropane (201 mg, 1.18 mmol) were added to the reaction mixture, and the mixture was stirred at 70°C for 4 hours. The reaction mixture was cooled to room temperature. Water (20 mL) was added, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 15-40%, retention time: 7.8-9.4 min, run time: 17 min) to obtain compound **59.** ¹H NMR (400 MHz, DMSO-*d*₆): δ 10.89 (s, 1H), 7.30 (dd, J = 8.8, 4.0 Hz, 1H), 7.23-7.19 (m, 2H), 6.91-6.86 (m, 1H), 3.32-3.25 (m, 1H), 3.24-3.21 (m, 1H), 2.96-2.93 (m, 1H), 2.77-2.71 (m, 1H), 2.64-2.61 (m, 1H), 2.39-2.35 (m, 1H), 1.83-1.79 (m, 1H), 1.70-1.66 (m, 1H), 1.01 (d, J = 6.4 Hz, 3H), 0.84 (d, J = 6.4 Hz, 3H). ESI-MS calculated for: [M+H]⁺ = 247.15, found 247.0.

### Example 60

### Synthetic route:

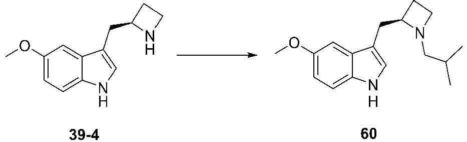

### Step 1

Compound **39-4** (100 mg, 0.46 mmol) was dissolved in methanol (1 mL). Diisopropylethylamine (178 mg, 1.38 mmol) and isobutyraldehyde (99.5 mg, 1.38 mmol) were added to the reaction mixture, and the mixture was stirred at 25°C for 0.5 hours. Sodium cyanoborohydride (28.9 mg, 0.46 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 35-45%, retention time: 7.7-9.6 min, run time: 17 min) to obtain compound **60.** ¹H NMR (400 MHz, DMSO-*d*₆): δ 10.59 (s, 1H), 7.20 (d, J = 8.8 Hz, 1H), 7.05 (d, J = 2.4 Hz, 1H), 6.95 (d, J = 2.4 Hz, 1H), 6.69 (dd, J = 8.8, 2.4 Hz, 1H), 3.75 (s, 3H), 3.32-3.24 (m, 1H), 3.21-3.17 (m, 1H), 2.90-2.85 (m, 1H), 2.75-2.67 (m, 1H), 2.59-2.52 (m, 1H), 2.26-2.21 (m, 1H), 2.04-2.00 (m, 1H), 1.91-1.87 (m, 1H), 1.81-1.76 (m, 1H), 1.57-1.51 (m, 1H), 0.85-0.82 (m, 6H). ESI-MS calculated for: [M+H]⁺ = 273.19, found 273.1.

### Example 61

### Synthetic route:

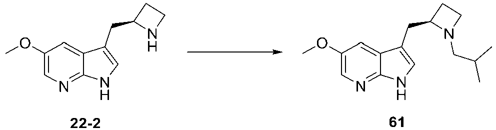

### Step 1

Compound **22-2** (100 mg, 0.46 mmol) was dissolved in methanol (2 mL). Diisopropylethylamine (178 mg, 1.38 mmol) and isobutyraldehyde (99.5 mg, 1.38 mmol) were added to the reaction mixture, and the mixture was stirred at 25°C for 0.5 hours. Sodium cyanoborohydride (28.9 mg, 0.46 mmol) was added, and the reaction mixture was stirred at 25°C for 2 hours. Saturated aqueous sodium bicarbonate solution (30 mL) was added to the reaction mixture, and the mixture was extracted with dichloromethane (30 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 35-45%, retention time: 5.0-7.3 min, run time: 16 min) to obtain compound **61.** ¹H NMR (400 MHz, DMSO-*d*₆): δ 11.15 (s, 1H), 7.92 (d, J = 2.4 Hz, 1H), 7.50 (d, J = 2.4 Hz, 1H), 7.17 (d, J = 2.0 Hz, 1H), 3.82 (s, 3H), 3.27-3.19 (m, 2H), 2.88-2.83 (m, 1H), 2.78-2.73 (m, 1H), 2.60-2.55 (m, 1H), 2.20-2.17 (m, 1H), 2.04-2.00 (m, 1H), 1.92-1.88 (m, 1H), 1.82-1.75 (m, 1H), 1.56-1.46 (m, 1H), 0.84-0.77 (m, 6H). ESI-MS calculated for: [M+H]⁺ = 274.18, found 274.0.

### Example 62

### Synthetic route:

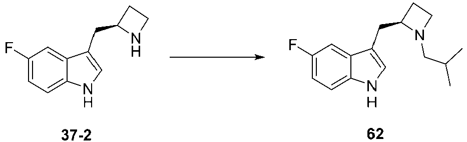

### Step 1

Compound **37-2** (150 mg, 0.73 mmol) was dissolved in methanol (3 mL). Diisopropylethylamine (377 mg, 2.92 mmol) and isobutyraldehyde (158 mg, 2.19 mmol) were added to the reaction mixture, and the mixture was stirred at 25°C for 0.5 hours. Sodium cyanoborohydride (45.9 mg, 0.73 mmol) was added, and the reaction mixture was stirred at 25°C for 2 hours. Saturated aqueous sodium bicarbonate solution (20 mL) was added to the reaction mixture, and the mixture was extracted with dichloromethane (20 mL × 3). The organic phases were combined, dried over anhydrous sodium sulfate, filtered, and the filtrate was concentrated under reduced pressure to obtain a crude product containing the target compound. The crude product was purified by high-performance liquid chromatography (Waters-XBridge-C18-10 µm-19*250 mm, mobile phase: acetonitrile-10 mmol/L aqueous ammonium bicarbonate solution, gradient: 44-60%, retention time: 6.7-9.4 min, run time: 17 min) to obtain compound **62.** ¹H NMR (400 MHz, DMSO-*d*₆): δ 10.87 (s, 1H), 7.30 (dd, J = 8.8, 4.4 Hz, 1H), 7.25 (dd, J = 9.8, 2.4 Hz, 1H), 7.17 (d, J = 2.4 Hz, 1H), 6.87 (td, J = 9.2, 2.4 Hz, 1H), 3.26-3.23 (m, 1H), 3.18-3.14 (m, 1H), 2.89-2.84 (m, 1H), 2.75-2.69 (m, 1H), 2.58-2.54 (m, 1H), 2.23-2.18 (m, 1H), 2.03-1.98 (m, 1H), 1.90-1.87 (m, 1H), 1.79-1.74 (m, 1H), 1.54-1.50 (m, 1H), 0.83-0.80 (m, 6H). ESI-MS calculated for: [M+H]⁺ = 261.17, found 261.1.

### Example 63

### Synthetic route:

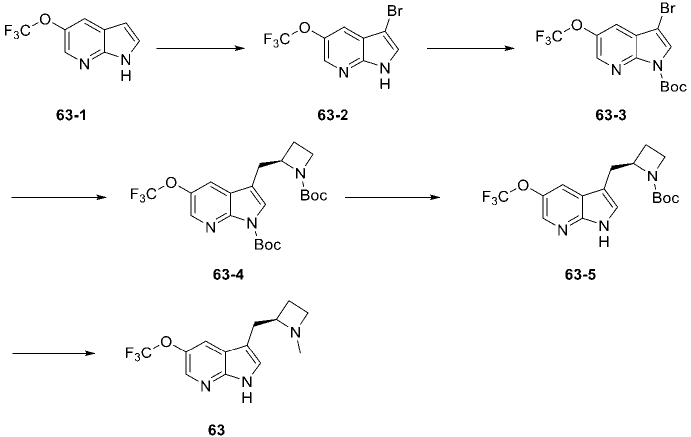

### Step 1

Compound **63-1** (1.00 g, 4.95 mmol) was dissolved in N,N-dimethylformamide (25 mL). Under a nitrogen atmosphere, *N*-bromosuccinimide (925 mg, 5.20 mmol) was slowly added at 0°C, and the mixture was stirred at 25°C for 1 hour. Aqueous sodium bicarbonate solution (50 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **63-2.** ESI-MS calculated for: [M+H]⁺ = 280.95 and 282.95, found 280.8 and 282.8.

### Step 2

Compound **63-2** (1.66 g, 5.91 mmol) was dissolved in dichloromethane (15 mL). *N*,*N*-Diisopropylethylamine (1.15 g, 8.87 mmol), di-*tert*-butyl dicarbonate (1.93 g, 8.87 mmol), and 4-dimethylaminopyridine (220 mg, 1.77 mmol) were added, and the mixture was stirred at 25°C for 2 hours. Aqueous ammonium chloride solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/dichloromethane, 5/1, v/v) to obtain compound **63-3.** ¹H NMR (400 MHz, DMSO-*d*₆): δ 8.56 (d, J = 2.4 Hz, 1H), 8.20 (s, 1H), 8.02 (d, J = 1.6 Hz, 1H), 1.61 (s, 9H).

### Step 3

In a glove box filled with nitrogen, compound **63-3** (200 mg, 0.52 mmol), (2*R*)*-tert-*butyl 2-(bromomethyl)azetidine-1-carboxylate (260 mg, 1.04 mmol), anhydrous sodium carbonate (110 mg, 1.04 mmol), tris(trimethylsilyl)silane (259 mg, 1.04 mmol), nickel(II) chloride dimethoxyethane (5.71 mg, 0.026 mmol), 4,4'-di-*tert*-butyl-2,2'-dipyridine (6.98 mg, 0.026 mmol), and bis[2-(2,4-difluorophenyl)-5-trifluoromethylpyridine][2-2'-bi(4-*tert-*butylpyridine)]iridium(III) bis(hexafluorophosphate) (5.83 mg, 0.0052 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with ethyl acetate (20 mL × 3). The organic phases were combined and washed with saturated brine (20 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **63-4.** ESI-MS calculated for: [M+H]⁺ = 472.20, found 472.2.

### Step 4

Compound **63-4** (731 mg, 1.55 mmol) was dissolved in tetrahydrofuran (5 mL), ethanol (5 mL), and water (2 mL). Lithium hydroxide monohydrate (325 mg, 7.75 mmol) was added, and the reaction mixture was stirred at 60°C for 2 hours. Water (20 mL) was added, and the mixture was extracted with ethyl acetate (20 mL × 3). The organic phases were combined and washed with saturated brine (20 mL). The organic phase was dried over anhydrous sodium sulfate, filtered, and concentrated under reduced pressure. The crude product was purified by silica gel column chromatography (dichloromethane/methanol, 20/1, v/v) to obtain compound **63-5.** ESI-MS calculated for: [M+H]⁺ = 372.15, found 372.1.

### Step 5

Compound **63-5** (210 mg, 0.57 mmol) was dissolved in tetrahydrofuran (5 mL). A solution of lithium aluminum hydride in tetrahydrofuran (2.28 mL, 5.70 mmol, 2.5 mol/L) was added dropwise at 0°C. Under a nitrogen atmosphere, the reaction mixture was stirred at 60°C for 2 hours. The reaction mixture was cooled to room temperature, quenched by the addition of ice water (0.25 mL), and then 15% aqueous sodium hydroxide solution (0.25 mL) and water (0.75 mL) were added. The mixture was filtered, and the filtrate was concentrated under reduced pressure. The crude product was purified by high-performance liquid chromatography (Waters-SunFire-C18-10 µm-19*250 mm, mobile phase: acetonitrile-10 mmol/L aqueous ammonium bicarbonate solution, gradient: 27-42%, retention time: 8.2-9.8 min, run time: 16 min) to obtain compound **63.** ¹H NMR (400 MHz, DMSO-*d*₆): δ 11.72 (s, 1H), 8.21 (d, J = 2.4 Hz, 1H), 8.04 (d, J = 1.2 Hz, 1H), 7.41 (s, 1H), 3.24-3.18 (m, 1H), 3.12-3.08 (m, 1H), 2.91-2.86 (m, 1H), 2.80-2.76 (m, 1H), 2.61-2.57 (m, 1H), 2.07 (s, 3H), 1.93-1.88 (m, 1H), 1.79-1.72 (m, 1H). ESI-MS calculated for: [M+H]⁺ = 286.11, found 285.9.

### Example 64

### Synthetic route:

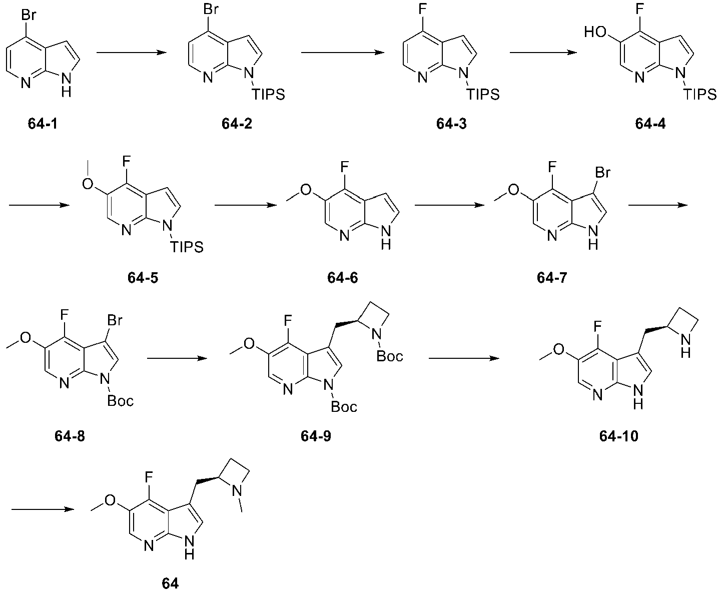

### Step 1

Sodium hydride (9.50 g, 238 mmol, 60% purity) was dissolved in *N,N-*dimethylformamide (380 mL). Under a nitrogen atmosphere, compound **64-1** (36.0 g, 183 mmol) was slowly added at 0°C, and the mixture was stirred at 0°C for 0.5 hours. Triisopropylsilyl chloride (52.8 g, 274 mmol) was added, and the mixture was stirred at 70°C for 3 hours. The reaction mixture was cooled to room temperature. Aqueous ammonium chloride solution (500 mL) was added, and the mixture was extracted with ethyl acetate (500 mL × 3). The organic phases were combined and washed with saturated brine (500 mL × 3). The organic phase was dried over anhydrous sodium sulfate, filtered, and concentrated under reduced pressure. The crude product was purified by silica gel column chromatography (petroleum ether/ethyl acetate, 100/1, v/v) to obtain compound **64-2.** ¹H NMR (400 MHz, DMSO-*d*₆): δ 8.10 (d, J = 5.2 Hz, 1H), 7.60 (d, J = 3.6 Hz, 1H), 7.37 (d, J = 5.2 Hz, 1H), 6.60 (d, J = 3.6 Hz, 1H), 1.90-1.83 (m, 3H), 1.05 (d, J = 6.8 Hz, 18H).

### Step 2

Compound **64-2** (5.00 g, 14.2 mmol) was dissolved in tetrahydrofuran (50 mL). Under a nitrogen atmosphere, n-butyllithium (11.3 mL, 28.3 mmol, 2.5 mol/L solution in *n-*hexane) was slowly added dropwise at -78°C, and the mixture was stirred at -78°C for 0.5 hours. *N*-Fluorobenzenesulfonimide (4.91 g, 15.6 mmol) was added, and the mixture was stirred at 25°C for 1 hour. Aqueous ammonium chloride solution (100 mL) was added to the reaction mixture, and the mixture was extracted with ethyl acetate (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 100/1, v/v) to obtain compound **64-3.** ¹H NMR (400 MHz, CDCl₃): δ 8.18 (dd, J = 8.4, 5.2 Hz, 1H), 7.26 (d, J = 3.6 Hz, 1H), 6.76 (dd, J = 9.6, 5.2 Hz, 1H), 6.63 (d, J = 3.6 Hz, 1H), 1.89-1.81 (m, 3H), 1.12 (d, J = 6.8 Hz, 18H).

### Step 3

Compound **64-3** (2.30 g, 7.86 mmol) was dissolved in tetrahydrofuran (50 mL). Under a nitrogen atmosphere, *sec*-butyllithium (13.3 mL, 17.3 mmol, 1.3 mol/L solution in *n-*hexane) was slowly added dropwise at -78°C, and the mixture was stirred at -78°C for 1 hour. (1*R*)-(-)-10-Camphorsulfonyloxaziridine (4.51 g, 19.7 mmol) was added, and the mixture was stirred at 25°C for 1 hour. The reaction mixture was added dropwise to an aqueous ammonium chloride solution (50 mL) and extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 20/1, v/v) to obtain compound **64-4.** ¹H NMR (400 MHz, CDCl₃): δ 8.06 (d, J = 12.0 Hz, 1H), 7.23 (d, J = 4.0 Hz, 1H), 6.56 (d, J = 4.0 Hz, 1H), 4.89 (s, 1H), 1.87-1.76 (m, 3H), 1.11 (d, J = 6.8 Hz, 18H). ESI-MS calculated for: [M+H]⁺ = 309.17, found 309.0.

### Step 4

Compound **64-4** (1.20 g, 3.89 mmol) and triphenylphosphine (1.33 g, 5.06 mmol) were dissolved in tetrahydrofuran (40 mL). Under a nitrogen atmosphere, diisopropyl azodicarboxylate (1.02 g, 5.06 mmol) was slowly added dropwise at 0°C, and the mixture was stirred at 0°C for 0.5 hours. Methanol (1.17 g, 36.6 mmol) was added, and the mixture was stirred at 25°C for 1 hour. The reaction mixture was added dropwise to an aqueous ammonium chloride solution (50 mL) and extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 1/0, v/v) to obtain compound **64-5.** ESI-MS calculated for: [M+H]⁺ = 323.19, found 323.0.

### Step 5

Compound **64-5** (800 mg, 2.48 mmol) was dissolved in tetrahydrofuran (20 mL). Under a nitrogen atmosphere, tetrabutylammonium fluoride (4.96 mL, 17.3 mmol, 1.0 mol/L solution in tetrahydrofuran) was slowly added dropwise at 0°C, and the mixture was stirred at 25°C for 1.5 hours. Water (50 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **64-6.** ¹H NMR (400 MHz, DMSO-*d*₆): δ 11.80 (s, 1H), 8.18 (d, J = 12.0 Hz, 1H), 7.49-7.47 (m, 1H), 6.47-6.45 (m, 1H), 3.91 (s, 3H). ESI-MS calculated for: [M+H]⁺ = 167.05, found 167.0.

### Step 6

Compound **64-6** (3.80 g, 22.9 mmol) was dissolved in *N*,*N*-dimethylformamide (50 mL). Under a nitrogen atmosphere, *N*-bromosuccinimide (4.07 g, 22.9 mmol) was slowly added at 0°C, and the mixture was stirred at 25°C for 1 hour. Aqueous sodium bicarbonate solution (50 mL) was added to the reaction mixture, and the mixture was extracted with ethyl acetate (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **64-7.** ¹H NMR (400 MHz, DMSO-*d*₆): δ 12.18 (s, 1H), 8.26 (d, J = 9.6 Hz, 1H), 7.69 (d, J = 2.8 Hz, 1H), 3.93 (s, 3H).

### Step 7

Compound **64-7** (5.16 g, 21.1 mmol) was dissolved in dichloromethane (60 mL). Under a nitrogen atmosphere, *N*,*N*-diisopropylethylamine (4.08 g, 31.6 mmol), di-*tert*-butyl dicarbonate (6.89 g, 31.6 mmol), and 4-dimethylaminopyridine (772 mg, 6.32 mmol) were slowly added at 0°C, and the mixture was stirred at 25°C for 2 hours. Water (50 mL) was added to the reaction mixture, and the mixture was extracted with dichloromethane (100 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/dichloromethane, 5/1, v/v) to obtain compound **64-8.** ¹H NMR (400 MHz, DMSO-*d*₆): δ 8.41 (d, J = 9.6 Hz, 1H), 7.97 (s, 1H), 3.98 (s, 3H), 1.60 (s, 9H).

### Step 8

In a glove box filled with nitrogen, compound **64-8** (304 mg, 0.88 mmol), (2*R*)*-tert-*butyl 2-(bromomethyl)azetidine-1-carboxylate (220 mg, 0.88 mmol), anhydrous sodium carbonate (187 mg, 1.76 mmol), tris(trimethylsilyl)silane (219 mg, 0.88 mmol), nickel(II) chloride dimethoxyethane (9.67 mg, 0.044 mmol), 4,4'-di-*tert*-butyl-2,2'-dipyridine (11.8 mg, 0.044 mmol), and bis[2-(2,4-difluorophenyl)-5-trifluoromethylpyridine][2-2'-bi(4-*tert-*butylpyridine)]iridium(III) bis(hexafluorophosphate) (9.87 mg, 0.0088 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with ethyl acetate (20 mL × 3). The organic phases were combined and washed with saturated brine (20 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **64-9.** ESI-MS calculated for: [M+H]⁺ = 436.22, found 436.1.

### Step 9

Compound **64-9** (350 mg, 0.80 mmol) was dissolved in dichloromethane (5 mL). Trifluoroacetic acid (0.6 mL) was slowly added at 0°C, and the mixture was stirred at 25°C for 1 hour. The reaction mixture was concentrated under reduced pressure. Saturated aqueous sodium bicarbonate solution (20 mL) was added to the residue, and the mixture was extracted with dichloromethane (20 mL × 3). The organic phases were combined and washed with saturated brine (20 mL). The organic phase was dried over anhydrous sodium sulfate, filtered, and concentrated under reduced pressure to obtain compound **64-10.** ESI-MS calculated for: [M+H]⁺ = 236.11, found 236.0.

### Step 10

Compound **64-10** (110 mg, 0.55 mmol) was dissolved in methanol (2 mL). *N,N-*Diisopropylethylamine (180 mg, 1.41 mmol) and 37% aqueous formaldehyde solution (15.5 mg, 0.52 mmol) were added, and the mixture was stirred at 25°C for 0.5 hours. Sodium cyanoborohydride (29.8 mg, 0.47 mmol) was added, and the mixture was stirred at 25°C for 2 hours. The reaction mixture was filtered, and the filtrate was concentrated under reduced pressure to obtain a crude product containing the target compound. The crude product was purified by high-performance liquid chromatography (Waters-SunFire-C18-10 µm-19*250 mm, mobile phase: acetonitrile-10 mmol/L aqueous ammonium bicarbonate solution, gradient: 10-40%, retention time: 8.4-9.8 min, run time: 17 min) to obtain compound **64.** ¹H NMR (400 MHz, DMSO-*d*₆): δ 11.47 (s, 1H), 8.13 (d, J = 9.6 Hz, 1H), 7.22 (s, 1H), 3.90 (s, 3H), 3.22-3.19 (m, 1H), 3.12-3.07 (m, 1H), 2.93-2.89 (m, 1H), 2.79-2.74 (m, 1H), 2.59-2.56 (m, 1H), 2.09 (s, 3H), 1.92-1.86 (m, 1H), 1.78-1.72 (m, 1H). ESI-MS calculated for: [M+H]⁺ = 250.13, found 250.0.

### Example 65

### Synthetic route:

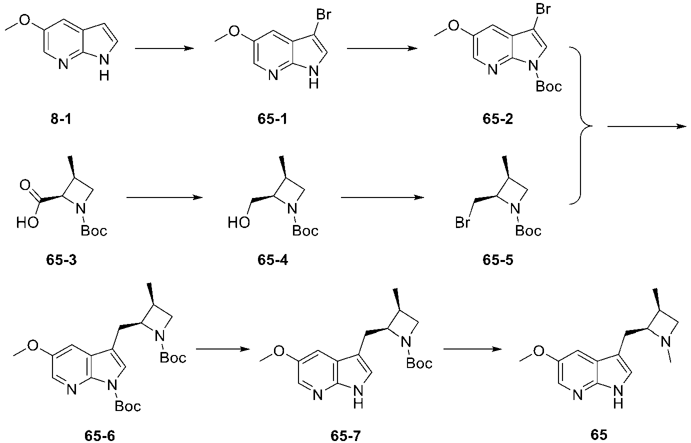

### Step 1

Compound **8-1** (5.00 g, 33.8 mmol) was dissolved in *N*,*N*-dimethylformamide (40 mL). Under a nitrogen atmosphere, *N*-bromosuccinimide (6.55 g, 36.8 mmol) was slowly added at 0°C, and the mixture was stirred at 0°C for 2 hours. Water (100 mL) was added to the reaction mixture, and the mixture was extracted with ethyl acetate (150 mL × 3). The organic phases were combined and washed with saturated brine (150 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **65-1.** ¹H NMR (400 MHz, DMSO-*d*₆): δ 11.96 (s, 1H), 8.07 (d, J = 4.0 Hz, 1H), 7.69 (d, J = 4.0 Hz, 1H), 7.36 (s, 1H), 3.89 (s, 3H).

### Step 2

Compound **65-1** (6.27 g, 27.6 mmol) was dissolved in dichloromethane (90 mL). Under a nitrogen atmosphere, *N*,*N*-diisopropylethylamine (7.13 g, 55.2 mmol), di-*tert*-butyl dicarbonate (9.03 g, 41.4 mmol), and 4-dimethylaminopyridine (340 mg, 2.76 mmol) were slowly added at 0°C, and the mixture was stirred at 25°C for 2 hours. Saturated aqueous ammonium chloride solution (100 mL) was added to the reaction mixture, and the mixture was extracted with dichloromethane (100 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/dichloromethane, 1/1, v/v) to obtain compound **65-2.** ¹H NMR (400 MHz, DMSO-*d*₆): δ 8.18 (d, J = 4.0 Hz, 1H), 7.98 (d, J = 4.00 Hz, 1H), 7.40 (s, 1H), 3.90 (s, 3H), 1.60 (s, 9H).

### Step 3

Compound **65-3** (1.70 g, 7.90 mmol) was dissolved in isopropyl acetate (5 mL). Under a nitrogen atmosphere, sodium borohydride (480 mg, 12.6 mmol) and boron trifluoride diethyl etherate (2.24 g, 15.8 mmol) were added at 0°C, and the mixture was stirred at 0°C for 3 hours. A 0.5 mol/L aqueous sodium hydroxide solution (12 mL) was added to the reaction mixture, and the mixture was stirred at 50°C for 0.5 hours. The reaction mixture was cooled to room temperature and extracted with ethyl acetate (50 mL × 3). The organic phases were combined and washed with saturated brine (100 mL). The organic phase was dried over anhydrous sodium sulfate, filtered, and concentrated under reduced pressure to obtain compound **65-4.** ¹H NMR (400 MHz, DMSO-*d*₆): δ 4.11-4.06 (m, 1H), 3.87-3.71 (m, 1H), 3.66-3.61 (m, 2H), 3.27-3.21 (m, 1H), 2.73-2.62 (m, 1H), 1.36 (s, 9H), 1.13 (d, J = 7.2 Hz, 3H).

### Step 4

Compound **65-4** (1.20 g, 5.96 mmol) was dissolved in acetonitrile (12 mL). Under a nitrogen atmosphere, triphenylphosphine (1.72 g, 6.56 mmol) was added, followed by the dropwise addition of a solution of carbon tetrabromide (2.17 g, 6.56 mmol) in acetonitrile (4 mL) at 0°C, and the mixture was stirred at 25°C for 12 hours. Saturated aqueous ammonium chloride solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **65-5.** ¹H NMR (400 MHz, CDCl₃): δ 4.45-4.39 (m, 1H), 3.97-3.92 (m, 1H), 3.71-3.65 (m, 1H), 3.48-3.43 (m, 1H), 3.34-3.31 (m, 1H), 2.79-2.71 (m, 1H), 1.44 (s, 9H), 1.27 (d, J = 7.2 Hz, 3H).

### Step 5

In a glove box filled with nitrogen, compound **65-2** (203 mg, 0.62 mmol), **65-5** (163 mg, 0.62 mmol), anhydrous sodium carbonate (131 mg, 1.24 mmol), tris(trimethylsilyl)silane (154 mg, 0.62 mmol), nickel(II) chloride dimethoxyethane (6.81 mg, 0.031 mmol), 4,4'-di-*tert-*butyl-2,2'-dipyridine (8.32 mg, 0.031 mmol), and bis[2-(2,4-difluorophenyl)-5-trifluoromethylpyridine][2-2'-bi(4-*tert*-butylpyridine)]iridium(III) bis(hexafluorophosphate) (6.96 mg, 0.0062 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with ethyl acetate (20 mL × 3). The organic phases were combined and washed with saturated brine (20 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **64-6.** ESI-MS calculated for: [M+H]⁺ = 432.24, found 432.2.

### Step 6

Compound **65-6** (680 mg, 1.58 mmol) was dissolved in tetrahydrofuran (5 mL), ethanol (5 mL), and water (2 mL). Lithium hydroxide monohydrate (331 mg, 7.90 mmol) was added, and the reaction mixture was stirred at 25°C for 2 hours. Water (20 mL) was added, and the mixture was extracted with ethyl acetate (20 mL × 3). The organic phase was dried over anhydrous sodium sulfate, filtered, and concentrated under reduced pressure. The crude product was purified by silica gel column chromatography (dichloromethane/methanol, 20/1, v/v) to obtain compound **65-7.** ¹H NMR (400 MHz, DMSO-*d*₆): δ 11.22 (s, 1H), 7.94 (d, J = 2.8 Hz, 1H), 7.49 (d, J = 2.8 Hz, 1H), 7.19 (s, 1H), 4.56-4.50 (m, 1H), 3.92-3.88 (m, 1H), 3.82 (s, 3H), 3.32-3.30 (m, 1H), 3.11-3.03 (m, 2H), 2.69-2.64 (m, 1H), 1.35 (s, 9H), 1.08 (d, J = 7.2 Hz, 3H). ESI-MS calculated for: [M+H]⁺ = 332.19, found 332.1.

### Step 7

Compound **65-7** (215 mg, 0.65 mmol) was dissolved in tetrahydrofuran (5 mL). A solution of lithium aluminum hydride in tetrahydrofuran (2.6 mL, 6.50 mmol, 2.5 mol/L) was slowly added dropwise at 0°C. Under a nitrogen atmosphere, the reaction mixture was stirred at 60°C for 2 hours. The reaction mixture was cooled to room temperature, quenched by the addition of ice water (0.3 mL), and then 15% aqueous sodium hydroxide solution (0.3 mL) and water (0.9 mL) were added. The mixture was filtered, and the filtrate was concentrated under reduced pressure to obtain a crude product containing the target compound. The crude product was purified by high-performance liquid chromatography (Waters-SunFire-C18-10 µm-19*250 mm, mobile phase: acetonitrile-0.05% aqueous ammonia, gradient: 13-23%, retention time: 9.0-11.0 min, run time: 16 min) to obtain compound **65.** ¹H NMR (400 MHz, CD₃OD): δ 7.93 (d, J = 2.8 Hz, 1H), 7.61 (d, J = 2.8 Hz, 1H), 7.20 (s, 1H), 3.90 (s, 3H), 3.69-2.66 (m, 1H), 3.15-3.13 (m, 2H), 2.97-2.94 (m, 2H), 2.60-2.54 (m, 1H), 2.19 (s, 3H), 1.28 (d, J = 7.2 Hz, 3H). ESI-MS calculated for: [M+H]⁺ = 246.15, found 246.0.

### Example 66

### Synthetic route:

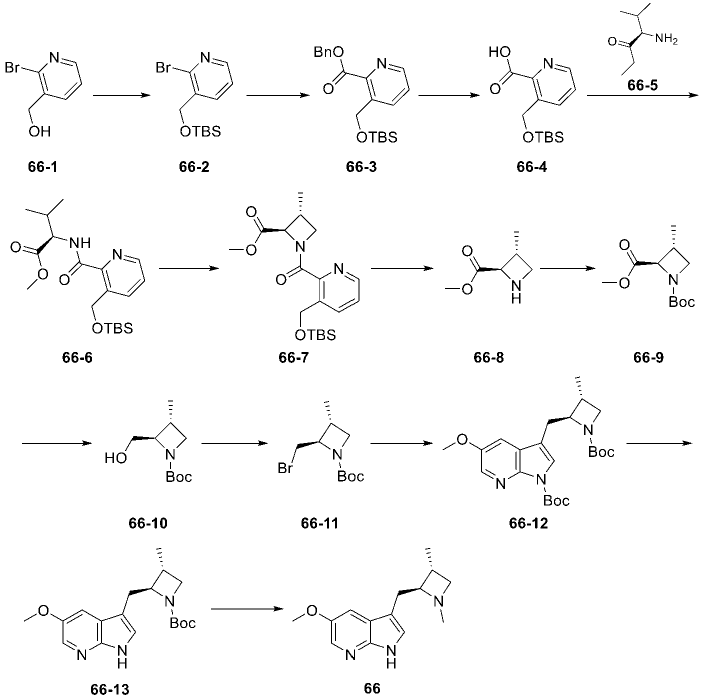

### Step 1

Compound **66-1** (25.0 g, 133 mmol) was dissolved in dichloromethane (250 mL). Under a nitrogen atmosphere, imidazole (27.2 g, 399 mmol) and *tert*-butyldimethylsilyl chloride (26.1 g, 173 mmol) were added at 0°C, and the mixture was stirred at 25°C for 12 hours. Saturated aqueous ammonium chloride solution (200 mL) was added to the reaction mixture, and the mixture was extracted with dichloromethane (200 mL). The organic phases were combined and washed with saturated brine (200 mL × 3). The organic phase was dried over anhydrous sodium sulfate, filtered, and concentrated under reduced pressure. The crude product was purified by silica gel column chromatography (petroleum ether/ethyl acetate, 10/1, v/v) to obtain compound **66-2.** ¹H NMR (400 MHz, CDCl₃): δ 8.26-8.25 (m, 1H), 7.86-7.84 (m, 1H), 7.32-7.29 (m, 1H), 4.69 (s, 2H), 0.96 (s, 9H), 0.14 (s, 6H).

### Step 2

Compound **66-2** (5.00 g, 16.5 mmol) was dissolved in N,N-dimethylformamide (35 mL). Under a nitrogen atmosphere, [1,1'-bis(diphenylphosphino)ferrocene]dichloropalladium(II) (1.21 g, 1.65 mmol), benzyl alcohol (8.94 g, 82.7 mmol), and triethylamine (8.37 g, 82.7 mmol) were added. The system was purged three times with carbon monoxide gas and stirred at 100°C for 12 hours under a carbon monoxide atmosphere. Water (150 mL) was added to the reaction mixture, and the mixture was extracted with ethyl acetate (150 mL × 3). The organic phases were combined and washed with saturated brine (150 mL × 3). The organic phase was dried over anhydrous sodium sulfate, filtered, and concentrated under reduced pressure. The crude product was purified by silica gel column chromatography (petroleum ether/ethyl acetate, 10/1, v/v) to obtain compound **66-3.** ¹H NMR (400 MHz, CDCl₃): δ 8.64-8.61 (m, 1H), 8.19 (d, J = 8.0 Hz, 1H), 7.51-7.48 (m, 2H), 7.37-7.32 (m, 4H), 5.44 (s, 2H), 5.07 (s, 2H), 0.95 (s, 9H), 0.10 (s, 6H). ESI-MS calculated for: [M+H]⁺ = 358.18, found 358.0.

### Step 3

Compound **66-3** (6.00 g, 16.8 mmol) was dissolved in ethyl acetate (80 mL). Under an argon atmosphere, 10% wet palladium on carbon (700 mg) was added. The system was purged three times with hydrogen and stirred at 25°C for 12 hours under a hydrogen atmosphere. The reaction mixture was filtered through diatomite, and the filter cake was washed with ethyl acetate (50 mL × 3). The filtrate was concentrated under reduced pressure to obtain compound **66-4.** ESI-MS calculated for: [M+H]⁺ = 268.13, found 268.0.

### Step 4

Compound **66-4** (4.40 g, 16.5 mmol) was dissolved in dichloromethane (50 mL). Under a nitrogen atmosphere, diisopropylethylamine (7.44 g, 57.6 mmol), 2-(7-azabenzotriazole)-*N*,*N*,*N'*,*N'*-tetramethyluronium hexafluorophosphate (7.82 g, 20.6 mmol), and compound **65-5** (2.62 g, 15.6 mmol) were added, and the mixture was stirred at 25°C for 2 hours. Water (100 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 20/1, v/v) to obtain compound **66-6.** ¹H NMR (400 MHz, CDCl₃): δ 8.72-8.69 (m, 1H), 8.48-8.46 (m, 1H), 8.27-8.25 (m, 1H), 7.47 (dd, J = 8.0, 4.4 Hz, 1H), 5.28 (s, 2H), 4.64 (dd, J = 9.2, 5.6 Hz, 1H), 3.76 (s, 3H), 2.33-2.23 (m, 1H), 1.02 (d, J = 6.8 Hz, 6H), 0.96 (s, 9H), 0.13 (s, 6H). ESI-MS calculated for: [M+H]⁺ = 381.21, found 381.1.

### Step 5

Compound **66-6** (600 mg, 1.58 mmol) was dissolved in toluene (15 mL). Under a nitrogen atmosphere, (diacetoxyiodo)benzene (1.27 g, 3.95 mmol), palladium acetate (17.7 mg, 0.079 mmol), and glacial acetic acid (200 mg, 3.33 mmol) were added, and the mixture was stirred in a microwave reactor at 110°C for 2 hours. The reaction mixture was cooled to room temperature. Water (20 mL) was added, and the mixture was extracted with ethyl acetate (20 mL × 3). The organic phases were combined and washed with saturated brine (20 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **66-7.** ESI-MS calculated for: [M+H]⁺ = 379.20, found 379.1.

### Step 6

Compound **66-7** (2.60 g, 6.87 mmol) was dissolved in dichloromethane (20 mL). A solution of hydrochloric acid in dioxane (20 mL, 80.0 mmol, 4.0 mol/L) was added, and the mixture was stirred at 25°C for 2 hours. The reaction mixture was concentrated under reduced pressure to obtain the monohydrochloride salt of compound **66-8.** ESI-MS calculated for: [M+H]⁺ = 130.08, found 130.1.

### Step 7

The monohydrochloride salt of compound **66-8** (1.40 g, 8.45 mmol) was dissolved in tetrahydrofuran (8 mL) and water (2 mL). Sodium carbonate (4.60 g, 43.4 mmol) and di-*tert-*butyl dicarbonate (4.73 g, 21.7 mmol) were added, and the mixture was stirred at 25°C for 4 hours. Water (30 mL) was added to the reaction mixture, and the mixture was extracted with ethyl acetate (60 mL × 3). The organic phases were combined and washed with saturated brine (30 mL). The organic phase was dried over anhydrous sodium sulfate, filtered, and concentrated under reduced pressure. The crude product was purified by silica gel column chromatography (petroleum ether/ethyl acetate, 10/1, v/v) to obtain compound **66-9.** ¹H NMR (400 MHz, CDCl₃): δ 4.18 (d, J = 4.8 Hz, 1H), 4.10 (t, J = 8.0 Hz, 1H), 3.76 (s, 3H), 3.44 (dd, J = 8.0, 5.2 Hz, 1H), 2.56-2.45 (m, 1H), 1.42 (s, 9H), 1.32 (d, J = 6.8 Hz, 3H).

### Step 8

Compound **66-9** (1.40 g, 6.11 mmol) was dissolved in methanol (20 mL). Under a nitrogen atmosphere, sodium borohydride (2.31 g, 61.1 mmol) was added in portions at 0°C, and the mixture was stirred at 0°C for 1 hour, then at 25°C for 3 hours. Water (100 mL) was added to the reaction mixture, and the mixture was extracted with ethyl acetate (50 mL × 3). The organic phases were combined and washed with saturated brine (100 mL). The organic phase was dried over anhydrous sodium sulfate, filtered, and concentrated under reduced pressure. The crude product was purified by silica gel column chromatography (petroleum ether/ethyl acetate, 4/1, v/v) to obtain compound **66-10.** ¹H NMR (400 MHz, CDCl₃): δ 4.00-3.96 (m, 1H), 3.88 (t, J = 8.4 Hz, 1H), 3.76-3.67 (m, 2H), 3.44 (dd, J = 8.4, 6.8 Hz, 1H), 2.35-2.25 (m, 1H), 1.45 (s, 9H), 1.18 (d, J = 6.8 Hz, 3H).

### Step 9

Compound **66-10** (1.10 g, 5.47 mmol) was dissolved in acetonitrile (12 mL). Under a nitrogen atmosphere, triphenylphosphine (1.58 g, 6.02 mmol) was added, followed by the dropwise addition of a solution of carbon tetrabromide (2.00 g, 6.02 mmol) in acetonitrile (4 mL) at 0°C, and the mixture was stirred at 25°C for 12 hours. Saturated aqueous ammonium chloride solution (50 mL) was added to the reaction mixture, and the mixture was extracted with dichloromethane (50 mL × 3). The organic phases were combined and washed with saturated brine (50 mL). The organic phase was dried over anhydrous sodium sulfate, filtered, and concentrated under reduced pressure. The crude product was purified by silica gel column chromatography (petroleum ether/ethyl acetate, 20/1, v/v) to obtain compound **66-11.** ¹H NMR (400 MHz, CDCl₃): δ 3.94-3.89 (m, 2H), 3.69 (dd, J = 9.6, 3.2 Hz, 1H), 3.52 (dd, J = 9.6, 8.4 Hz, 1H), 3.35 (dd, J = 8.0, 5.6 Hz, 1H), 2.46-2.36 (m, 1H), 1.44 (s, 9H), 1.25 (d, J = 6.8 Hz, 3H).

### Step 10

In a glove box filled with nitrogen, compound **65-2** (203 mg, 0.62 mmol), **66-11** (163 mg, 0.62 mmol), anhydrous sodium carbonate (131 mg, 1.24 mmol), tris(trimethylsilyl)silane (154 mg, 0.62 mmol), nickel(II) chloride dimethoxyethane (6.81 mg, 0.031 mmol), 4,4'-di-*tert-*butyl-2,2'-dipyridine (8.32 mg, 0.031 mmol), and bis[2-(2,4-difluorophenyl)-5-trifluoromethylpyridine][2-2'-bi(4-*tert*-butylpyridine)]iridium(III) bis(hexafluorophosphate) (6.96 mg, 0.0062 mmol) were dissolved in ethylene glycol dimethyl ether (4 mL). The reaction mixture was placed under irradiation with a 34 W blue LED (420 nm) and stirred at 25°C for 16 hours. After completion of the reaction, the blue light was turned off. Water (20 mL) was added to the reaction mixture, and the mixture was extracted with ethyl acetate (20 mL × 3). The organic phases were combined and washed with saturated brine (20 mL). The organic phase was dried over anhydrous sodium sulfate, filtered, and concentrated under reduced pressure. The crude product was purified by silica gel column chromatography (petroleum ether/ethyl acetate, 3/1, v/v) to obtain compound **66-12.** ESI-MS calculated for: [M+H]⁺ = 432.24, found 432.1.

### Step 11

Compound **66-12** (580 mg, 1.34 mmol) was dissolved in tetrahydrofuran (5 mL), ethanol (5 mL), and water (2 mL). Lithium hydroxide monohydrate (281 mg, 6.70 mmol) was added, and the reaction mixture was stirred at 25°C for 2 hours. Water (20 mL) was added, and the mixture was extracted with ethyl acetate (20 mL × 3). The organic phase was dried over anhydrous sodium sulfate, filtered, and concentrated under reduced pressure. The crude product was purified by silica gel column chromatography (dichloromethane/methanol, 20/1, v/v) to obtain compound **66-13.** ESI-MS calculated for: [M+H]⁺ = 332.19, found 332.1.

### Step 12

Compound **66-13** (150 mg, 0.45 mmol) was dissolved in tetrahydrofuran (5 mL). A solution of lithium aluminum hydride in tetrahydrofuran (1.8 mL, 4.50 mmol, 2.5 mol/L) was slowly added dropwise at 0°C. Under a nitrogen atmosphere, the reaction mixture was stirred at 60°C for 2 hours. The reaction mixture was cooled to room temperature, quenched by the addition of ice water (0.18 mL), and then 15% aqueous sodium hydroxide solution (0.18 mL) and water (0.54 mL) were added. The mixture was filtered, and the filtrate was concentrated under reduced pressure to obtain a crude product containing the target compound. The crude product was purified by high-performance liquid chromatography (Waters-SunFire-C18-10 µm-19*250 mm, mobile phase: acetonitrile-10 mmol/L aqueous ammonium bicarbonate solution, gradient: 13-38%, retention time: 8.2-10.0 min, run time: 16 min) to obtain compound **66.** ¹H NMR (400 MHz, DMSO-*d*₆): δ 11.16 (s, 1H), 7.92 (d, J = 2.8 Hz, 1H), 7.51 (d, J = 2.8 Hz, 1H), 7.21 (s, 1H), 3.82 (s, 3H), 3.41 (t, J = 6.4 Hz, 1H), 2.86-2.82 (m, 1H), 2.78-2.73 (m, 1H), 2.71-2.67 (m, 1H), 2.24-2.21 (m, 1H), 2.13-2.06 (m, 4H), 0.81 (d, J = 6.8 Hz, 3H). ESI-MS calculated for: [M+H]⁺ = 246.15, found 246.0.

### Example 67

### Synthetic route:

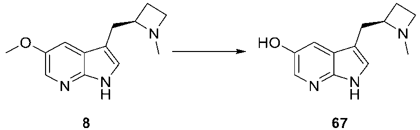

Compound **8** (100 mg, 0.432 mmol) was dissolved in dichloromethane (5 mL). A solution of boron tribromide in dichloromethane (2.16 mL, 2.16 mmol, 1.0 mol/L) was slowly added dropwise at 0°C, and the reaction mixture was stirred at 25°C for 2 hours under a nitrogen atmosphere. The reaction mixture was cooled to 0°C, and methanol (5 mL) was slowly added dropwise to quench the reaction. The reaction mixture was stirred at 25°C for 0.5 hours, concentrated under reduced pressure, and the crude product was purified by silica gel column chromatography (dichloromethane/methanol, 10/1, v/v) to obtain compound **67.** ¹¹H NMR (400 MHz, DMSO-*d*₆): δ 11.31 (s, 1H), 9.86 (s, 1H), 7.88 (d, J = 2.8 Hz, 1H), 7.41 (d, J = 2.8 Hz, 1H), 7.30 (d, J = 2.0 Hz, 1H), 4.48-4.44 (m, 1H), 4.02-3.98 (m, 1H), 3.80-3.76 (m, 1H), 3.26-3.22 (m, 1H), 3.10-3.06 (m, 1H), 2.64 (s, 3H), 2.39-2.31 (m, 1H), 2.30-2.23 (m, 1H). ESI-MS calculated for: [M+H]⁺ = 218.12, found 218.0.

### Example 68

### Structural formula:

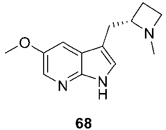

Following the synthetic route and method in Example 8, compound **68** could be prepared by replacing (*R*)-*N*-*tert*-butoxycarbonyl-azetidine-2-carboxylic acid with (*S*)*-N-tert-*butoxycarbonyl-azetidine-2-carboxylic acid. ¹H NMR (400 MHz, DMSO-*d*₆): δ 11.17 (s, 1H), 7.92 (d, J = 2.8 Hz, 1H), 7.50 (d, J = 2.8 Hz, 1H), 7.19 (d, J = 2.0 Hz, 1H), 3.81 (s, 3H), 3.28-3.25 (m, 1H), 3.24-3.20 (m, 1H), 2.91-2.85 (m, 1H), 2.81-2.74 (m, 1H), 2.72-2.69 (m, 1H), 2.13 (s, 3H), 1.98-1.93 (m, 1H), 1.87-1.79 (m, 1H). ESI-MS calculated for: [M+H]⁺ = 232.14, found 232.0.

### Example 69

### Structural formula:

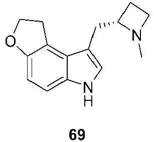

Following the synthetic route and method in Example 11, compound **69** could be prepared by replacing (*R*)-*N*-*tert*-butoxycarbonyl-azetidine-2-carboxylic acid with (*S*)*-N-tert-*butoxycarbonyl-azetidine-2-carboxylic acid. ¹H NMR (400 MHz, DMSO-*d*₆): δ 10.58 (s, 1H), 7.06-7.02 (m, 2H), 6.56 (d, J = 8.4 Hz, 1H), 4.51 (t, J = 8.8 Hz, 2H), 3.48-3.44 (m, 2H), 3.26-3.18 (m, 1H), 3.12-3.03 (m, 1H), 2.97-2.93 (m, 1H), 2.73-2.65 (m, 1H), 2.60-2.54 (m, 1H), 2.12 (s, 3H), 1.96-1.89 (m, 1H), 1.83-1.75 (m, 1H). ESI-MS calculated for: [M+H]⁺ = 243.14, found 243.0.

### Example 70

### Structural formula:

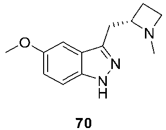

Following the synthetic route and method in Example 14, compound **70** could be prepared by replacing *tert-*butyl (2R)-2-(bromomethyl)azetidine-1-carboxylate with *tert-*butyl (2S)-2-(bromomethyl)azetidine-1-carboxylate. ¹H NMR (400 MHz, DMSO-*d*₆): δ 12.50 (s, 1H), 7.34 (d, J = 8.8 Hz, 1H), 7.13 (s, 1H), 6.95 (dd, J = 8.8, 2.0 Hz, 1H), 3.78 (s, 3H), 3.25-3.20 (m, 2H), 3.08-3.03 (m, 1H), 2.99-2.96 (m, 1H), 2.61-2.53 (m, 1H), 2.10 (s, 3H), 1.90-1.75 (m, 2H). ESI-MS calculated for: [M+H]⁺ = 232.14, found 232.0.

### Example 71

### Structural formula:

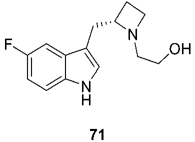

Following the synthetic routes and methods in Example 3 and Example 37, compound 71 could be prepared by replacing *tert*-butyl (2*R*)-2-(bromomethyl)azetidine-1-carboxylate with *tert-*butyl (2S)-2-(bromomethyl)azetidine-1-carboxylate. ¹H NMR (400 MHz, DMSO-*d*₆): δ 10.87 (s, 1H), 7.31-7.24 (m, 2H), 7.18 (s, 1H), 6.90-6.86 (m, 1H), 4.34 (s, 1H), 3.28-3.22 (m, 4H), 2.93-2.89 (m, 1H), 2.78-2.62 (m, 2H), 2.58-2.53 (m, 1H), 2.34-2.25 (m, 1H), 1.96-1.87 (m, 1H), 1.83-1.73 (m, 1H). ESI-MS calculated for: [M+H]+ = 249.13, found 249.0.

### Effect Example 1: Evaluation of Calcium Flux Agonist Activity of Compounds on 5-HT_{2A} Receptor

### Experimental objective:

To determine the activity of compounds on the 5-HT_{2A} receptor using a stable cell line (HEK293 cells) expressing the human 5-HT_{2A} receptor.

### Experimental reagents and consumables are shown in Table 1:

**Table 1**

| **Reagents and consumables** | **Supplier** | **Catalog No.** |
|---|---|---|
| Glutamine-free DMEM | Gibco | 11960 |
| Fetal bovine serum (FBS) | Biosera | FB1058 |
| MatiGel^{®} matrix | BD Bioscience | 356234 |
| Dimethyl sulfoxide | Sigma | D8799 |
| 10 cm culture dish | Corning | 430167 |
| Cryogenic storage tube | Corning | 430289 |
| 384-well plate | Greiner-BioOne | 781091 |

### Experimental instruments are shown in Table 2:

**Table 2**

| **Instruments and equipment** | **Supplier** | **Catalog No.** |
|---|---|---|
| Cell viability analyzer | Beckman Coulter | Vi-CELL XR |
| CO₂ incubator | Thermo Scientific | HERACELL 240i |
| Biological safety cabinet | AIRTECH | BSC-160411A2 |
| Inverted microscope | Nikon | ECLIPSE Ts2 |
| Liquid handling system | Agilent | Bravo V11 |
| Plate reader | PerkinElmer | Envision |

### Experimental procedure:

### Day 1: Cell seeding and compound preparation

The test compounds were diluted with DMSO to prepare stock solutions at a concentration of 400 times the highest test concentration in a 384-well LDV plate. The compound solutions were then transferred to a 384-well plate.

HEK-293/5-HT_{2A} cells were cultured in DMEM medium (10% FBS). When the cells reach a density of 80%, the cells were detached using 0.25% Trypsin-EDTA.

The cell density was measured, and the cells were diluted using DMEM (10% FBS).

Using a Multidrop, 30 µL of cells (25,000 cells per well) were added to each well of a Matrigel-coated 384-well plate and incubated at 37°C with 5% CO₂ for 20-24 hours.

### Day 2: Cell-based experimental procedure

The culture medium was removed from the cell plate, and 40 µL of fluorescent dye (AAT Bioquest, Catalog No. 21080) were added to each well of the cell plate. The plate was incubated in the dark at 37°C with 5% CO₂ for 0.5 hours.

20 µL of assay buffer (1× HBSS + 20 mM HEPES + 0.1% BSA) were added to each well of the compound plate to prepare a 5-fold concentrated agonist working solution for calcium signal reading. The instrument was programmed to add 10 µL of the compound to the cell plate (10 µL + 40 µL) for activation data collection.

The data were read and saved using FLIPR at room temperature with the specified settings.

### Data analysis:

Compound dilutions were prepared from 20 mmol dimethyl sulfoxide (DMSO) stock solutions. Compound addition was performed on a fluorescence imaging plate reader, and the fluorescence changes reflecting calcium ion release were monitored at 1-second intervals for a total of 130 seconds (excitation wavelength = 470-495 nm, emission wavelength = 515-575 nm). Data were exported as the difference between the maximum and minimum fluorescence for each well. The results were calculated using non-linear regression to determine the relative EC₅₀ and Emax values (using XL-fit and Graphpad Prism software).

**The experimental results are shown in Table 3:**

**Table 3**

| **Final Products of Examples** | **EC₅₀ (µM)** | **Emax** |
|---|---|---|
| Example 1 | 0.138 | 34.9% |
| Example 2 | 0.031 | 73.5% |
| Example 3 | 0.109 | 36.5% |
| Example 5 | 0.197 | 38.7% |
| Example 6 | 0.063 | 20.8% |
| Example 7 | 0.046 | 72.8% |
| Example 8 | 0.030 | 49.8% |
| Example 9 | 0.006 | 23.7% |
| Example 10 | 0.038 | 72.0% |
| Example 11 | 0.001 | 56.3% |
| Example 12 | 0.009 | 41.6% |
| Example 13 | 0.107 | 92.0% |
| Example 14 | 0.065 | 40.8% |
| Example 16 | 0.017 | 37.1% |
| Example 17 | 0.003 | 81.8% |
| Example 18 | 0.121 | 36.0% |
| Example 19 | 0.008 | 65.3% |
| Example 21 | 0.140 | 77.1% |
| Example 22 | 0.071 | 40.0% |
| Example 25 | 0.007 | 21.4% |
| Example 26 | 0.006 | 39.3% |
| Example 27 | 0.092 | 24.5% |
| Example 29 | 0.017 | 52.7% |
| Example 30 | 0.045 | 13.6% |
| Example 33 | 0.082 | 58.8% |
| Example 34 | 0.133 | 30.1% |
| Example 35 | 0.065 | 41.9% |
| Example 36 | 0.160 | 63.7% |
| Example 37 | 0.012 | 52.3% |
| Example 38 | 0.100 | 39.6% |
| Example 39 | 0.010 | 81.5% |
| Example 41 | 0.004 | 66.8% |
| Example 42 | 0.014 | 64.8% |
| Example 43 | 0.071 | 73.5% |
| Example 46 | 0.045 | 65.2% |
| Example 47 | 0.004 | 59.2% |
| Example 48 | 0.042 | 14.9% |
| Example 50 | 0.113 | 32.7% |
| Example 51 | 0.020 | 30.2% |
| Example 52 | 0.011 | 22.8% |
| Example 53 | 0.014 | 35.6% |
| Example 54 | 0.005 | 77.4% |
| Example 55 | 0.008 | 79.8% |
| Example 56 | 0.024 | 34.8% |
| Example 57 | 0.008 | 53.6% |
| Example 58 | 0.014 | 71.0% |
| Example 59 | 0.012 | 15.8% |
| Example 60 | 0.082 | 54.2% |
| Example 62 | 0.109 | 28.8% |
| Example 64 | 0.188 | 20.7% |
| Example 67 | 0.020 | 78.3% |
| Example 68 | 0.220 | 16.1% |
| Disclosed compound | 0.152 | 58.0% |

### Structure of the disclosed compound:

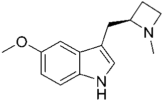

### Experimental conclusion:

The experimental samples (compounds) were prepared according to the corresponding examples, and the results are shown in the table above. In this test system, the compounds of the present disclosure exhibited agonist activity on the 5-HT_{2A} receptor.

### Effect Example 2: Evaluation of Calcium Flux Agonist Activity of Compounds on 5-HT_{2B} Receptor

### Experimental objective:

To determine the activity of compounds on the 5-HT_{2B} receptor using a stable cell line (HEK293 cells) expressing the human 5-HT_{2B} receptor.

**Experimental reagents and consumables are shown in Table 4:**

**Table 4**

| **Reagents and consumables** | **Supplier** | **Catalog No.** |
|---|---|---|
| Glutamine-free DMEM | Gibco | 11960 |
| Fetal bovine serum (FBS) | Biosera | FB1058 |
| MatiGel^{®} matrix | BD Bioscience | 356234 |
| Dimethyl sulfoxide | Sigma | D8799 |
| 10 cm culture dish | Corning | 430167 |
| Cryogenic storage tube | Corning | 430289 |
| 384-well plate | Greiner-BioOne | 781091 |

**Experimental instruments are shown in Table 5:**

**Table 5**

| **Instruments and equipment** | **Supplier** | **Catalog No.** |
|---|---|---|
| Cell viability analyzer | Beckman Coulter | Vi-CELL XR |
| CO₂ incubator | Thermo Scientific | HERACELL 240i |
| Biological safety cabinet | AIRTECH | BSC-160411A2 |
| Inverted microscope | Nikon | ECLIPSE Ts2 |
| Liquid handling system | Agilent | Bravo V11 |
| Plate reader | Molecular Device | FLIPR Penta |

### Experimental procedure:

### Day 1: Cell seeding and compound preparation

The test compounds were diluted with DMSO to prepare stock solutions at a concentration of 400 times the highest test concentration in a 384-well LDV plate. The compound solutions were then transferred to a 384-well plate.

HEK-293/5-HT_{2b} cells were cultured in DMEM medium (10% FBS). When the cells reached a density of 80%, the cells were detached using 0.25% Trypsin-EDTA.

The cell density was measured, and the cells were diluted using DMEM (10% FBS).

Using a Multidrop, 30 µL of cells (25,000 cells per well) were added to each well of a Matrigel-coated 384-well plate and incubated at 37°C with 5% CO₂ for 20-24 hours.

### Day 2: Cell-based experimental procedure

The culture medium was removed from the cell plate, and 40 µL of fluorescent dye (AAT Bioquest, Catalog No. 21080) were added to each well of the cell plate. The plate was incubated in the dark at 37°C with 5% CO₂ for 0.5 hours.

20 µL of assay buffer (1× HBSS + 20 mM HEPES + 0.1% BSA) were added to each well of the compound plate to prepare a 5-fold concentrated agonist working solution for calcium signal reading. The instrument program was used to add 10 µL of the compound to the cell plate (10 µL + 40 µL) for activation data collection.

The data were read and saved using FLIPR at room temperature with the specified settings.

### Data analysis:

Compound dilutions were prepared from 20 mmol dimethyl sulfoxide (DMSO) stock solutions. Compound addition was performed on a fluorescence imaging plate reader, and the fluorescence changes reflecting calcium ion release were monitored at 1-second intervals for a total of 130 seconds (excitation wavelength = 470-495 nm, emission wavelength = 515-575 nm). Data were exported as the difference between the maximum and minimum fluorescence for each well. The results were calculated using non-linear regression to determine the relative EC₅₀ and Emax values (using XL-fit and Graphpad Prism software).

**The experimental results are shown in Table 6:**

**Table 6**

| **Final Products of Examples** | **EC₅₀ (µM)** | **Emax** |
|---|---|---|
| Example 1 | >10 | / |
| Example 2 | 2.412 | 67.4% |
| Example 3 | >10 | / |
| Example 5 | >10 | / |
| Example 6 | >10 | / |
| Example 8 | >10 | / |
| Example 9 | >10 | / |
| Example 10 | 2.241 | 39.9% |
| Example 11 | >10 | / |
| Example 12 | >10 | / |
| Example 13 | >10 | / |
| Example 14 | >10 | / |
| Example 17 | 3.498 | 34.4% |
| Example 18 | >10 | / |
| Example 21 | >10 | / |
| Example 25 | >10 | / |
| Example 26 | >10 | / |
| Example 27 | >10 | / |
| Example 29 | >10 | / |
| Example 33 | >10 | / |
| Example 34 | >10 | / |
| Example 37 | >10 | / |
| Example 38 | >10 | / |
| Example 39 | >10 | / |
| Example 42 | >10 | / |
| Example 46 | >10 | / |
| Example 47 | >10 | / |
| Example 49 | >10 | / |
| Example 50 | >10 | / |
| Example 51 | >10 | / |
| Example 52 | >10 | / |
| Example 54 | >10 | / |
| Example 55 | >10 | / |
| Example 57 | >10 | / |
| Example 58 | >10 | / |
| Example 60 | >10 | / |
| Example 62 | >10 | / |
| Disclosed compound | >10 | / |

Structure of the disclosed compound:

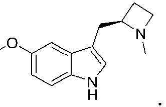

"/" indicates no corresponding value.

### Experimental conclusion:

The experimental samples (compounds) were prepared according to the corresponding examples, and the results are shown in the table above. In this test system, the compounds of the present disclosure exhibited no agonist activity on the 5-HT_{2B} receptor.

### Effect Example 3: Evaluation of Calcium Flux Agonist Activity of Compounds on 5-HT_{2C} Receptor

### Experimental objective:

To determine the activity of compounds on the 5-HT_{2C} receptor using a stable cell line (HEK293 cells) expressing the human 5-HT_{2C} receptor.

**Experimental reagents and consumables are shown in Table 7:**

**Table 7**

| **Reagents and consumables** | **Supplier** | **Catalog No.** |
|---|---|---|
| Glutamine-free DMEM | Gibco | 11960 |
| Fetal bovine serum (FBS) | Biosera | FB1058 |
| MatiGel^{®} matrix | BD Bioscience | 356234 |
| Dimethyl sulfoxide | Sigma | D8799 |
| 10 cm culture dish | Corning | 430167 |
| Cryogenic storage tube | Corning | 430289 |
| 384-well plate | Greiner-BioOne | 781091 |

**Experimental instruments are shown in Table 8:**

**Table 8**

| **Instruments and equipment** | **Supplier** | **Catalog No.** |
|---|---|---|
| Cell viability analyzer | Beckman Coulter | Vi-CELL XR |
| CO₂ incubator | Thermo Scientific | HERACELL 240i |
| Biological safety cabinet | AIRTECH | BSC-160411A2 |
| Inverted microscope | Nikon | ECLIPSE Ts2 |
| Liquid handling system | Agilent | Bravo V11 |
| Plate reader | Molecular Device | FLIPR Penta |

### Experimental procedure:

### Day 1: Cell seeding and compound preparation

The test compounds were diluted with DMSO to prepare stock solutions at a concentration of 400 times the highest test concentration in a 384-well LDV plate. The compound solutions were then transferred to a 384-well plate.

HEK-293/5-HT_{2C} cells were cultured in DMEM medium (10% FBS). When the cells reached a density of 80%, the cells were detached using 0.25% Trypsin-EDTA.

The cell density was measured, and the cells were diluted using DMEM (10% FBS).

Using a Multidrop, 30 µL of cells (25,000 cells per well) were added to each well of a Matrigel-coated 384-well plate and incubated at 37°C with 5% CO₂ for 20-24 hours.

### Day 2: Cell-based experimental procedure

The culture medium was removed from the cell plate, and 40 µL of fluorescent dye (AAT Bioquest, Catalog No. 21080) were added to each well of the cell plate. The plate was incubated in the dark at 37°C with 5% CO₂ for 0.5 hours.

20 µL of assay buffer (1× HBSS + 20 mM HEPES + 0.1% BSA) were added to each well of the compound plate to prepare a 5-fold concentration of the agonist working solution for calcium signal reading. The instrument program was used to add 10 µL of the compound to the cell plate (10 µL + 40 µL) for activation data collection.

The data were read and saved using FLIPR at room temperature with the specified settings.

### Data analysis:

Compound dilutions were prepared from 20 mmol dimethyl sulfoxide (DMSO) stock solutions. Compound addition was performed on a fluorescence imaging plate reader, and the fluorescence changes reflecting calcium ion release were monitored at 1-second intervals for a total of 130 seconds (excitation wavelength = 470-495 nm, emission wavelength = 515-575 nm). Data were exported as the difference between the maximum and minimum fluorescence for each well. The results were calculated using non-linear regression to determine the relative EC₅₀ and Emax values (using XL-fit and Graphpad Prism software).

**The experimental results are shown in Table 9:**

**Table 9**

| **Final Products of Examples** | **EC₅₀ (µM)** | **Emax** |
|---|---|---|
| Example 1 | 0.019 | 54.0% |
| Example 2 | 0.015 | 80.5% |
| Example 3 | 0.003 | 34.8% |
| Example 5 | 0.344 | 22.8% |
| Example 6 | 0.081 | 56.1% |
| Example 8 | >10 | / |
| Example 9 | 0.122 | 12.8% |
| Example 10 | 0.113 | 37.1% |
| Example 11 | 0.002 | 16.9% |
| Example 12 | 0.026 | 42.1% |
| Example 13 | >10 | / |
| Example 14 | >10 | / |
| Example 16 | 0.019 | 34.6% |
| Example 17 | >10 | / |
| Example 18 | 0.113 | 70.2% |
| Example 19 | 0.010 | 70.8% |
| Example 21 | >10 | / |
| Example 25 | >10 | / |
| Example 26 | >10 | / |
| Example 27 | >10 | / |
| Example 29 | 0.423 | 20.9% |
| Example 33 | 0.078 | 61.1% |
| Example 34 | >10 | / |
| Example 37 | 0.004 | 20.9% |
| Example 38 | >10 | / |
| Example 39 | 0.011 | 47.3% |
| Example 42 | >10 | / |
| Example 46 | >10 | / |
| Example 47 | >10 | / |
| Example 49 | >10 | / |
| Example 50 | >10 | / |
| Example 51 | >10 | / |
| Example 52 | >10 | / |
| Example 54 | 0.050 | 17.6% |
| Example 55 | 0.088 | 29.8% |
| Example 57 | >10 | / |
| Example 58 | 0.023 | 18.3% |
| Example 60 | >10 | / |
| Example 62 | >10 | / |
| Disclosed compound | 0.015 | 54.8% |

Structure of the disclosed compound:

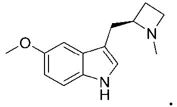

"/" indicates no corresponding value.

### Experimental conclusion:

The experimental samples (compounds) were prepared according to the corresponding examples, and the results are shown in the table above. In this test system, some compounds of the present disclosure exhibited agonist activity on the 5-HT_{2C} receptor.

### Effect Example 4: Evaluation of Calcium Flux Agonist Activity of Compounds on 5-HT_{1A} Receptor

### Experimental objective:

To determine the agonist activity of compounds on the 5-HT_{1A} receptor using a stable cell line (CHO cells) expressing the human 5-HT_{1A} receptor.

**Experimental reagents and consumables are shown in Table 10:**

**Table 10**

| **Reagents and consumables** | **Supplier** | **Catalog No.** |
|---|---|---|
| F12 medium | Sigma | N6658 |
| Fetal bovine serum | ExCell | FSP500 |
| Geneticin G418 | Gibco | 10131-027 |
| Blasticidin S | InvivoGen | ant-bl-05 |
| Dual antibiotics (Penicillin-Streptomycin) | Hyclone | SV30010 |
| Fluo-4 Direct Kit | Invitrogen | F10471 |
| 384-well plate | Greiner | 781090 |
| 384-well compound plate | Eakan | SVP120A-384-T |

**Experimental instruments are shown in Table 11:**

**Table 11**

| **Instruments and equipment** | **Supplier** | **Catalog No.** |
|---|---|---|
| Cell counter | Beckman | Vi-CELL XR |
| CO₂ incubator | Thermo | 371GP |
| Acoustic liquid handling system | Labcyte | ECHO555 |
| FLIPR | Molecular Devices | Penta |

### Experimental procedure:

### Day 1: Cell plating

CHO/5-HT_{1A} cells were cultured in culture medium. When the cells reached a density of 80-90%, the cells were digested with trypsin, followed by the addition of an appropriate amount of medium to terminate the digestion. The cells were centrifuged at 1000 rpm for 5 minutes at room temperature. After the supernatant was discarded, the cells were resuspended in culture medium, and 1 mL was taken for cell counting. The cell count and viability were determined using a cell counter. The cells were diluted to 1 × 10⁶/mL with culture medium, and 20 µL/well of the cell suspension was added to a 384-well cell plate (approximately 20,000 cells/well). The cells were incubated overnight at 37°C with 5% CO₂.

### Day 2: FLIPR assay

The compounds were subjected to 3-fold 10-point gradient dilution via Echo, and 750 nL of each compound was transferred to a compound plate in duplicate.

The culture medium was removed from the cell plate, and 20 µL of buffer and 20 µL of dye were added to each well of the cell plate. The plate was incubated in the dark at 37°C, 5% CO₂ for 50 minutes, followed by incubation at room temperature for 10 minutes.

30 µL of assay buffer (1× HBSS+20 mM HEPES+0.5% BSA) were added to each well of the compound plate to prepare a 5-fold concentrated agonist working solution for calcium signal reading. 10 µL of the compound was added to the cell plate (10 µL + 40 µL) using FLIPR for activation data collection.

The data were read and saved using FLIPR at room temperature with the specified settings.

### Data analysis:

Compound dilutions were prepared from 20 mmol dimethyl sulfoxide (DMSO) stock solutions. Compound addition was performed on a fluorescence imaging plate reader, and the fluorescence changes reflecting calcium ion release were monitored at 1-second intervals for 60 seconds and then at 6-second intervals for 30 seconds (excitation wavelength = 470-495 nm, emission wavelength = 515-575 nm). Data were exported as the difference between the maximum and minimum fluorescence for each well. The results were calculated using non-linear regression to determine the relative EC₅₀ and Emax values (using Xl-fit or Graphpad Prism software).

**The experimental results are shown in Table 12:**

**Table 12**

| **Final Products of Examples** | **EC₅₀ (µM)** | **Emax** |
|---|---|---|
| Example 8 | >10 | / |
| Example 11 | >10 | / |
| Example 14 | 1.88 | 65.0% |
| Example 37 | >10 | / |

| | | |
|---|---|---|
| **[0773]** "/" indicates no corresponding value. | | |

### Experimental conclusion:

The experimental samples (compounds) were prepared according to the corresponding examples, and the results are shown in the table above. In this test system, some compounds of the present disclosure exhibited no agonist activity on the 5-HT_{lA} receptor.

### Effect Example 5: Evaluation of Compound Affinity for the 5-HT_{2A} Receptor

### Experimental objective:

The primary objective of this experiment is to demonstrate the affinity of the test samples for human 5-HT_{2A} through a radioactive binding experiment.

**Experimental reagents and consumables are shown in Table 13:**

**Table 13**

| **Reagent name** | **Supplier** | **Catalog No.** |
|---|---|---|
| 30% PEI (Poly ethyleneimine) | TCI | P0381 |
| Microscint 20 cocktail | Revvity | 6013329 |
| Unifilter-96 GF/C filter plates | Revvity | 6055690 |
| 96 well conical polypropylene plates | Agilent | 267280 181876 |

### Experimental instruments are shown in Table 14:

**Table 14**

| **Instrument name** | **Supplier** | **Model** |
|---|---|---|
| Cell harvester | PerkinElmer | 96-well harvester |
| Liquid flash counter | PerkinElmer | MicroBeta2 |

### Experimental procedure:

The compounds were subjected to 3-fold 10-point gradient dilution, and 1 µL of each compound was transferred to a compound plate in duplicate. 100 µL of 5 µg/well 5-HT_{2A} cell membrane was added to each well of the reaction plate, and 100 µL of 0.5 nM ³H-LSD isotope was added to each well of the reaction plate. The reaction plate was sealed with a plate-sealing film and then incubated at room temperature for 1 hour. The GF/C filter plate was soaked in 50 µL of 0.3% PEI soaking solution for at least 0.5 hours. After the reaction plate was incubated, the reaction mixture was collected onto the GF/C filter plate using a cell harvester, and the plate was washed four times with cold wash buffer and dried in an oven at 50°C for 1 hour. The bottom of the dried GF/C filter plate was sealed, and 50 µL of scintillation fluid was added to each well, followed by sealing. The plate was read using Microbeta2.

### Data analysis:

The percentage activity was calculated using Microsoft Excel software with the formula: % inhibition = (1 - (signal of sample well - mean signal value of High Control) / (mean signal value of Low Control - mean signal value of High Control) × 100

The IC₅₀ and Ki values for each test sample were calculated using XLfitXL-FIT according to the four-parameter fitting model.

**The experimental results are shown in Table 15:**

**Table 15**

| **Final Products of Examples** | **IC₅₀ (µM)** | **Ki (µM)** |
|---|---|---|
| Example 8 | 0.304 | 0.246 |
| Example 11 | 0.026 | 0.021 |
| Example 14 | 0.958 | 0.775 |
| Example 37 | 0.165 | 0.133 |
| Disclosed compound | 0.056 | 0.046 |

Structure of the disclosed compound:

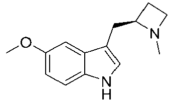

### Experimental conclusion:

The experimental samples (compounds) were prepared according to the corresponding examples, and the results are shown in the table above. In this test system, the compounds of the present disclosure exhibited affinity for the 5-HT_{2A} receptor.

### Effect Example 6: Evaluation of the Agonist Effect of Compounds on the 5-HT_{2A} Receptor Through Beta-Arrestin 2 Recruitment

### Experimental objective

The primary objective of this experiment is to demonstrate the activity of the compounds in the 5-HT_{2A} receptor/beta-arrestin 2 recruitment pathway.

**Experimental reagents and consumables are shown in Table 16:**

**Table 16**

| **Reagent name** | **Supplier** | **Catalog No.** |
|---|---|---|
| DMEM medium | Corning | 10-013-CV |
| Fetal bovine serum | Excell | FSP500 |
| Puromycin dihydrochloride | Beyotime | ST551-50mg |
| Blasticidin S | Beyotime | ST018-1mL |
| Opti-MEM | Gbico | 31985-070 |
| Furimazine | Aladdin | F302511 |
| 384-well plate | Greiner | 781090 |

**Experimental instruments are shown in Table 17:**

**Table 17**

| **Instrument name** | **Supplier** | **Model** |
|---|---|---|
| Cell counter | Beckmann | Vi Cell counter |
| CO₂ incubator | Thermo Scientific | HEPA CLASS 100 |
| Liquid handling workstation | Agilent | Bravo |
| Acoustic liquid handling system | Labcyte | Echo655 |
| Microplate reader | PerkinElmer | Envision |

### Experimental procedure:

5-HT_{2A}/ARRB2 OE HEK293T cells were cultured using a culture medium, followed by digestion with trypsin, and then an appropriate amount of culture medium was added to terminate the digestion. The cells were centrifuged at 1000 rpm for 5 minutes at room temperature. After the supernatant was discarded, the cells were resuspended in Opti-MEM, and 1 mL was taken for cell counting. The cell count and viability were determined using a cell counter. The cells were diluted to 0.75 × 10⁶/mL, and 40 µL/well of the cell suspension was added to a 384-well cell plate. The cells were incubated overnight at 37°C with 5% CO₂.

The compounds were subjected to 3-fold 10-point gradient dilution via Echo, and 200 nL of each compound was transferred to a compound plate in duplicate. 20 µL of Opti-MEM was added to each well to prepare a 10-fold concentrated agonist working solution.

5 µL of luminescent substrate (Furimazine) and 5 µL of the compound were added to each well of the cell plate. The plate was incubated at 37°C with 5% CO₂ for 30-40 minutes, and the chemiluminescence signals were read using Envision.

### Data analysis:

The EC₅₀ of each test sample on each plate was calculated by XL-FIT according to the four-parameter fitting model. 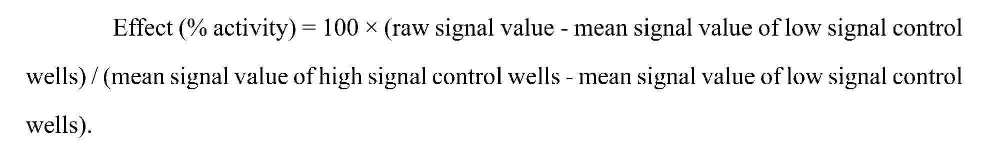Effect (% activity) = 100 × (raw signal value - mean signal value of low signal control wells) / (mean signal value of high signal control wells - mean signal value of low signal control wells).

Signal ratio of reference standard or sample = mean raw data value at maximum dose / mean raw data value at minimum dose.

**The experimental results are shown in Table 18:**

**Table 18**

| **Compound** | **EC₅₀ (µM)** | **Emax** |
|---|---|---|
| Example 8 | 0.014 | 85.5% |
| Example 11 | 0.0009 | 88.7% |
| Example 14 | 0.092 | 78.3% |
| Example 37 | 0.006 | 75.8% |

### Experimental conclusion:

The test articles were prepared according to the corresponding examples, and the results are shown in the table above. In this test system, the compounds of the present disclosure exhibited agonist activity on the 5-HT_{2A} receptor.

### Effect Example 7: Evaluation of the Effects of Compounds on Dendrites of Primary Rat Cortical Neurons

### Experimental objective:

To evaluate the effects of the compounds on dendritogenesis (dendritic growth and branching) in primary rat cortical neurons, quantified by high-content imaging and Sholl analysis.

**Experimental materials are shown in Table 19:**

**Table 19**

| **Reagents and consumables** | **Supplier** | **Catalog No.** |
|---|---|---|
| Poly-D-lysine hydrobromide | Sigma | P6407 |
| Mouse laminin | Sigma | L2020 |
| Neurobasal^{™} medium | Gibco | 21103049 |
| MAP2 antibody (AP20) | Invitrogen | MA5-12823 |
| Alexa Fluor^{™} 568 secondary antibody | Invitrogen | A-11004 |

**Experimental instruments are shown in Table 20:**

**Table 20**

| **Instruments and equipment** | **Supplier** |
|---|---|
| Envision^{®} Multimode Plate Reader | Perkin Elmer |
| Vi-Cell^{™} XR Cell Viability Analyzer | Beckman |
| ECLIPSE Ts2 Inverted Microscope | Nikon |
| CellVoyager^{™} CQ1 High-Content Imaging System | Yokogawa Electric |

### Experimental procedure:

### Primary neuron isolation

### Dissection and tissue collection:

Pregnant SD rats on gestational day 18 were euthanized, and the brains were rapidly collected using pre-chilled dissection buffer (DMEM + 10% fetal bovine serum + 1% Penicillin-Streptomycin). The cortices were isolated and cut into approximately 1 mm³ tissue blocks.

### Enzymatic digestion:

Digestion was performed at 37°C for 45 minutes using collagenase A (2 mg/mL) + DNase I (100 µg/mL). The digestion was terminated with TrypLE^{™} Express, and after washing, the cells were resuspended in complete Neurobasal medium.

### Preparation of culture plate

A 96-well plate was coated with polylysine (100 µg/mL) + laminin (10 µg/mL), incubated for 4 hours, and then left overnight. After UV sterilization, the cells were seeded at a density of 7,500 cells per well.

### Compound treatment

Days *in vitro* 5 (DIV5): blank controls and test compounds (1 µM) were added, with three replicates per group. Days *in vitro* 12 (DIV12): the cells were fixed and subjected to MAP2/DAPI immunofluorescence staining.

### Imaging and analysis

Images were acquired using a 20× objective on a CellVoyager^{™} CQ1 system.

ImageJ plugin analysis: Simple Neurite Tracer: tracing dendritic morphology.

Sholl analysis: calculating the area under the curve (AUC) of intersections within a radius range of 5 to 40 µm.

**The experimental results are shown in Table 21.**

**Table 21**

| **Group** | **Area under the curve (AUC) of intersections within a radius range of 5 to 40 µm** |
|---|---|
| Blank control | 276 |
| 1 µM Compound 8 | 335** |
| 1 µM Compound 11 | 359*** |
| 1 µM Compound 37 | 352** |
| 1 µM Compound 65 | 337 |

In the Sholl analysis, the area under the curve (AUC) of intersections of neuronal dendrites within the radius range of 5 µm to 40 µm was calculated. Data were processed using GraphPad Prism 8.3 and Excel. Statistical differences between groups were analyzed by one-way ANOVA. Compared with the blank control group, *p* < 0.05 indicates a significant difference, while p < 0.01 and p < 0.001 indicate highly significant differences. * indicates *p* < 0.05, ** indicates *p* < 0.01, and *** indicates *p* < 0.001.

**Experimental conclusion:** The test articles were prepared according to the corresponding example. The results showed that compared with the blank control group, the area under the curve of intersections within the radius range increased. The test articles can promote the growth of primary rat cortical neurons and possess the ability to regulate the neural plasticity of neuronal dendrites.

### Effect Example 8: Study on the Metabolic Stability of Compounds in Mice, Rats, Dogs, Monkeys, and Human Liver Microsomes

### Experimental procedure

### 1. Incubation procedure

The test compounds or positive controls (including testosterone, diclofenac, and propafenone) were incubated once at 1.0 µM (in acetonitrile) with liver microsomes (from Corning, Xenotech, or other reliable suppliers, with microsomes from each species derived from a pool of multiple donors) at a final protein concentration of 0.5 mg/mL (in 100 mM phosphate buffer (PB buffer)).

The mixture was pre-warmed at 37°C for 10 minutes, and the reaction was initiated by the addition of a cofactor system (1.0 mM NADPH). The test compound incubated with liver microsomes at 37°C without the addition of a cofactor system served as a negative control reaction.

### 2. Sampling

Reaction samples were sampled at multiple time points (*e.g.*, 0, 5, 15, 30, 45, and 60 minutes), while samples without the cofactor system (NCF) were sampled at 60 minutes. All samples were immediately mixed with pre-chilled acetonitrile containing an internal standard (IS) to terminate the reactions.

### 3. Single-point testing

Each test condition was determined in a single measurement (n = 1).

### 4. Sample analysis

Samples were analyzed by LC-MS/MS; the disappearance of the test compounds was evaluated based on the peak area ratio of analyte/internal standard (IS) (no standard curve was required).

### 5. Data summary

An Excel data summary was provided, including the calculated intrinsic clearance and half-life (T_{1/2}) values.

### 6. Microsomal clearance calculation

The microsomal clearance was calculated using the following formula:
$\begin{matrix}Remaining \left(%\right) = \frac{peak area ratio of compound to internal standard at any time point}{peak area ratio of compound to internal standard at 0 min} \times 100 \\ {C}_{t} = {C}_{0} \times {e}^{- {k}_{e} \times t}\end{matrix}$ when ${C}_{t} = \frac{1}{2} {C}_{0}$ ${T}_{1 / 2} = \frac{Ln 2}{{k}_{e}} = \frac{0.693}{{k}_{e}}$
Ct: represents the drug concentration at time t;
C₀: represents the drug concentration at the initial time point (t = 0);
e: represents the base of the natural logarithm, approximately equal to 2.71828;
kₑ: represents the elimination rate constant, describing the rate at which the drug is eliminated from the body;
t: represents time.
Liver weight: 40 g/kg (rat), 30 g/kg (monkey), 32 g/kg (dog), 20 g/kg (human), and 88 g/kg (mouse).
Hepatic clearance was calculated using CL_{int(mic)}:
Microsomal protein/liver weight: 45 mg/g (applicable to five species).

**The experimental results are shown in Table 22:**

**Table 22**

| Compound | CLᵢₙₜ (Species: human, mouse, rat, dog, monkey. Unit: mL/min/kg) |
|---|---|
| Compound 8 | <8.6, 54.1, 69.4, 33.1, 54.1 |
| Compound 9 | <8.6, <38.0, 31.3, 25.4, 21.9 |
| Compound 11 | <8.6, <38.0, 75.1, <13.8, 13.1 |
| Compound 14 | <8.6, 58.3, 204.5, 14.9, 57.5 |
| Compound 37 | <8.6, <38.0, 212.5, 37.4, 59.4 |
| Compound 42 | <8.6, <38.0, 97.3, 18.2, 29.7 |

### Experimental conclusion:

The results indicate that the compounds of the present disclosure exhibit low hepatic microsomal clearance and favorable metabolic stability.

### Effect Example 9: Evaluation of the Inhibitory Activity of Compounds on the hERG Potassium Channel

### Experimental objective:

The inhibitory effects of the examples of the present disclosure on the hERG (human ether-à-go-go-related gene) potassium channel were detected using the whole-cell manual patch-clamp technique.

### Experimental procedure:

### Cell culture and treatment:

CHO cells stably expressing hERG were cultured in 35-mm-diameter cell culture dishes and maintained in an incubator at 37°C with 5% CO₂. The cells were passaged every 48 hours at a ratio of 1:5. The culture medium composition was as follows: 90% F12 (Invitrogen), 10% fetal bovine serum (Gibco), 100 µg/mL G418 (Invitrogen), and 100 µg/mL Hygromycin B (Invitrogen). On the day of the experiment, the cell culture medium was aspirated, the cells were rinsed once with extracellular solution, and then 0.25% Trypsin-EDTA (Invitrogen) solution was added for digestion at room temperature for 3-5 minutes. The digestion solution was aspirated, and the cells were resuspended in extracellular solution before being transferred to an experimental dish for electrophysiological recording.

### Compound preparation:

On the day of testing, the compounds were prepared as 20 mM stock solutions in DMSO, then serially diluted three-fold in DMSO to obtain intermediate concentrations, and finally diluted 500-fold with extracellular fluid to achieve the desired final test concentrations.

Preparation of the positive control compound cisapride: 10 µL of a 150 µM cisapride DMSO stock solution was added to 4990 µL of extracellular solution and diluted 500-fold to obtain the required final test concentration of 300 nM.

The DMSO content in the final test concentration did not exceed 1%, and this concentration of DMSO had no effect on the hERG potassium channel.

### Electrophysiological recording process:

CHO cells stably expressing the hERG potassium channel were used to record bERG potassium channel currents at room temperature using the whole-cell voltage-clamp technique. Glass microelectrodes were fabricated from glass electrode blanks (BF150-86-10, Sutter) using a puller. After filling with the electrode internal solution, the tip resistance was approximately 2-5 MΩ. The glass microelectrodes were inserted into the amplifier headstage to connect to the patch-clamp amplifier. The clamping voltage and data recording were controlled and recorded by pClamp software via a computer, with a sampling frequency of 10 kHz and a filter frequency of 2 kHz. After obtaining the whole-cell recording, the cells were clamped at -100 mV. The hERG potassium current (I hERG) was elicited by a step voltage protocol: a 2-second depolarizing voltage from -100 mV to +20 mV, followed by repolarization to -50 mV for 1 second, and then returned to -100 mV. This voltage stimulus was applied every 5 seconds. After the hERG potassium current was confirmed to be stable (1 minute), the drug administration process was initiated. Each test concentration of the compound was applied for at least 1 minute until a steady-state effect was achieved, or for a maximum of 3 minutes. At least two cells were tested for each concentration (n ≥ 2).

### Data processing:

Data analysis and processing were conducted using pClamp, GraphPad Prism 8, and Excel software. The degree of inhibition of the hERG potassium currents (peak hERG tail current evoked at -50 mV) by different compound concentrations was calculated using the following formula:
$Inhibition % = \left[1-\left(I/Io\right)\right] \times 100 %$
where Inhibition % represents the percentage inhibition of hERG potassium current by the compound, and I and Io represent the amplitudes of hERG potassium current after and before compound administration, respectively.

The IC₅₀ values of the compounds were calculated by fitting using GraphPad Prism 8 software with the following equation:
$Y = Bottom + \left(Top-Bottom\right) / \left(1+{10}^{∧}\left(\left({LogIC}_{50}-X\right)*HillSlope\right)\right)$
where X represents the Log value of the test article concentration, Y represents the percentage inhibition at the corresponding concentration, and Bottom and Top represent the minimum and maximum percentage inhibition, respectively.

**The experimental results are shown in Table 23:**

**Table 23**

| Compound | hERG IC₅₀ (µM) |
|---|---|
| Compound 8 | >30 |
| Compound 11 | 18.6 |
| Compound 14 | 27.6 |
| Compound 42 | >30 |

### Experimental conclusion:

The inhibitory effects of the examples of the present disclosure on the hERG potassium channel are shown in the table above. It can be seen that the compounds of the present disclosure have a low risk of inhibiting the hERG potassium channel, even reaching 10 µM or above.

### Effect Example 10: Evaluation of Pharmacokinetic Properties of Compounds in Mice In Vivo

### Experimental objective:

To evaluate the pharmacokinetic properties of the examples of the present disclosure in CD-1 mice *in vivo.*

**Experimental materials are shown in Table 24:**

**Table 24**

| Materials | Supplier | Catalog No. |
|---|---|---|
| CD-1 mice (male, 20-40 g, 6-9 weeks old) | Shanghai Bikai Keyi Biotechnology Co., Ltd. | 20230009014679 |
| EDTA-2K anticoagulant tube | Jiangsu KANGJIAN Medical Apparatus Co., Ltd. | KJ201 |

### Experimental procedure:

The pharmacokinetic characteristics of the compounds in rodents following intravenous injection and oral gavage administration were evaluated according to a standard protocol. In the experiment, the candidate compounds were formulated as clear solutions or suspensions using the designated vehicle and were administered to three mice by single intravenous injection or oral gavage, respectively. The vehicle for both intravenous injection and oral gavage administration was a 10% sulfobutyl-β-cyclodextrin aqueous solution. Whole blood samples within 8 hours were collected into commercially available EDTA2K anticoagulant tubes and centrifuged to obtain the upper plasma samples. Acetonitrile solution containing an internal standard was added to precipitate proteins, and the mixture was centrifuged. The supernatant was collected and an equal volume of water was added. After another centrifugation, the supernatant was taken for injection. The plasma drug concentrations were quantitatively analyzed by an LCMS/MS analytical method to calculate the pharmacokinetic parameters.

**The routes of administration are shown in Table 25:**

**Table 25**

| Test sample | Dose |
|---|---|
| Example compound | Intravenous injection: 1.0 mg/kg |
| | Oral gavage: 5.0 mg/kg |

**The experimental results are shown in Table 26:**

**Table 26**

| Test sample | Peak concentration Cₘₐₓ, oral gavage (ng/mL) | Half-life T_{1/2}, oral gavage (hr) | Tissue distribution Vdₛₛ (L/kg) | Clearance Cl (mL/min/kg) | Area under the concentration-time curve AUC₀₋ₗₐₛₜ, oral gavage (hr*ng/mL) | Bioavailability F, oral gavage (%) |
|---|---|---|---|---|---|---|
| Example 1 | 534 | 4.82 | 3.19 | 46.5 | 2033 | 111 |
| Example 2 | 686 | 3.77 | 1.70 | 50.7 | 1803 | 112 |
| Example 5 | 283 | 4.97 | 4.90 | 56.1 | 1333 | 91.4 |
| Example 8 | 568 | 1.01 | 2.12 | 90.1 | 1038 | 111 |
| Example 9 | 853 | 3.95 | 1.46 | 27.8 | 2389 | 119 |
| Example 11 | 423 | 1.71 | 2.22 | 82.8 | 769 | 75.8 |
| Example 14 | 313 | 1.66 | 2.93 | 76.1 | 595 | 54.8 |
| Example 19 | 394 | 1.55 | 2.00 | 56.7 | 691 | 46.8 |
| Example 21 | 231 | 3.29 | 6.83 | 85.4 | 832 | 87.1 |
| Example 29 | 274 | 1.55 | 2.52 | 61.5 | 584 | 43.3 |
| Example 37 | 368 | 0.804 | 2.46 | 94.4 | 488 | 56.0 |
| Example 42 | 407 | 1.21 | 3.71 | 114 | 582 | 88.2 |
| Example 57 | 406 | 1.04 | 2.62 | 86.9 | 868 | 113 |

### Experimental conclusion:

The test articles were prepared according to the corresponding examples. The results demonstrate that the examples of the present disclosure exhibit favorable pharmacokinetic properties in mice *in vivo.*

### Effect Example 11: Evaluation of Pharmacokinetic Properties of Compounds in Rats In Vivo

### Experimental objective:

To evaluate the pharmacokinetic properties of the compounds obtained from the examples of the present disclosure in SD rats *in vivo.*

**Experimental materials are shown in Table 27:**

**Table 27**

| **Materials** | **Supplier** | **Catalog No.** |
|---|---|---|
| SD rats (male, 150-400 g, 6-9 weeks old) | Beijing Vital River | DMPK-RPK202411-19 |
| EDTA-2K anticoagulant tube | Jiangsu KANGJIAN Medical Apparatus Co., Ltd. | Jiangsu Kangjian-KJ202 |

### Experimental procedure:

The pharmacokinetic characteristics of the compounds in rodents following intravenous injection and oral administration were evaluated according to a standard protocol. In the experiment, the candidate compounds were formulated as clear solutions or suspensions using the designated vehicle and were administered to three rats by single intravenous injection or oral administration, respectively. The vehicle for both intravenous injection and oral administration was a 10% sulfobutyl-β-cyclodextrin aqueous solution. Whole blood samples within 24 hours were collected into commercially available EDTA2K anticoagulant tubes and centrifuged to obtain the upper plasma samples. Acetonitrile solution containing an internal standard was added to precipitate proteins, and the mixture was centrifuged. The supernatant was collected and an equal volume of water was added. After another centrifugation, the supernatant was taken for injection. The plasma drug concentrations were quantitatively analyzed by an LCMS/MS analytical method to calculate the pharmacokinetic parameters.

**The routes of administration are shown in Table 28:**

**Table 28**

| Test sample | Dose |
|---|---|
| Example compound | Intravenous injection: 2.0 mg/kg |
| | Oral gavage: 5.0 mg/kg |

**The experimental results are shown in Table 29:**

**Table 29**

| Test sample | Peak concentration Cₘₐₓ, oral gavage (ng/mL) | Half-life T_{1/2}, oral gavage (hr) | Tissue distribution Vdₛₛ (L/kg) | Clearance Cl (mL/min/kg) | Area under the concentration-time curve AUC₀₋ₗₐₛₜ, oral gavage (hr*ng/mL) | Bioavailability F, oral gavage (%) |
|---|---|---|---|---|---|---|
| Example 8* | 664 | 0.735 | 2.91 | 102 | 1246 | 77.6 |
| Example 11 | 271 | 2.49 | 4.66 | 115 | 1051 | 145 |

| | | | | | | |
|---|---|---|---|---|---|---|
| *: Oral gavage dose: 10.0 mg/kg | | | | | | |

### Experimental results:

The test articles were prepared according to the corresponding examples. The results demonstrate that the compounds of the present disclosure exhibit favorable pharmacokinetic properties in rats *in vivo.*

### Activity Test 12: Evaluation of Pharmacokinetic Properties of Compounds in Dogs In Vivo

### Experimental objective:

To evaluate the pharmacokinetic properties of the compounds of the examples of the present disclosure in beagle dogs *in vivo.*

**Experimental materials are shown in Table 30:**

**Table 30**

| **Materials** | **Supplier** | **Catalog No.** |
|---|---|---|
| Beagle dogs (male, 5-12 kg, ≥5 months old) | Jiangsu Marshall | 24000072XP |
| EDTA-2K anticoagulant tube | Jiangsu KANGJIAN Medical Apparatus Co., Ltd. | KJ202 |

### Experimental procedure:

The pharmacokinetic characteristics of the compounds in beagle dogs following intravenous injection and oral administration were evaluated according to a standard protocol. In the experiment, the candidate compounds were formulated as clear solutions or suspensions using the designated vehicle and were administered to two beagle dogs by single intravenous injection or oral administration, respectively. The vehicle for intravenous injection was a 10% sulfobutyl-β-cyclodextrin aqueous solution, and the vehicle for oral administration was a 10% sulfobutyl-β-cyclodextrin aqueous solution. Whole blood samples within 24 hours were collected into commercially available EDTA2K anticoagulant tubes and centrifuged to obtain the upper plasma samples. Acetonitrile solution containing an internal standard was added to precipitate proteins, and the mixture was centrifuged. The supernatant was collected and an equal volume of water was added. After another centrifugation, the supernatant was taken for injection. The plasma drug concentrations were quantitatively analyzed by an LCMS/MS analytical method to calculate the pharmacokinetic parameters.

The test articles were prepared according to the corresponding examples. The compounds of the present disclosure exhibit favorable pharmacokinetic properties.

### Effect Example 13: Compound-Induced Head Twitch Response Model in Mice

### Experimental objective:

To evaluate the changes in the number of head twitches in C57BL/6J mice induced by the compounds using the head twitch response (HTR) model in mice, thereby assessing the hallucinogenic effect of the compounds of the examples of the present disclosure.

**Experimental materials are shown in Table 31:**

**Table 31**

| **Materials** | **Supplier** | **Catalog No. or Model** |
|---|---|---|
| C57BL/6J mice (male, 7-8 weeks old) | Shanghai Lingchang Biotechnology Co., Ltd. | 20230003016759 |
| Analytical balance | METTLER TOLEDO | MS205DU |
| Head twitch recording chamber | - | Height*diameter: 25 cm*10 cm |
| Body weight scale | Changzhou Keyuan Electronic Instrument | YH20002 |
| Multi-channel physiological recorder | AD Instruments | PLC01 |
| Instrument Interface | AD Instruments | PLC1 |

### Experimental procedure:

Upon arrival at the animal facility, the animals were acclimated for 3 days.

Based on the body weight of the animals, Zoletil 50 (20 mg/kg, intraperitoneal injection) and xylazine hydrochloride (5 mg/kg, intraperitoneal injection) were administered. After the animals were anesthetized (no withdrawal response was observed when the paw was lightly pressed), the upper part of the scalp was opened, and a magnetic bead (diameter: 5 mm, height: 2 mm) was implanted subcutaneously beneath the scalp. The HTR test was conducted 3 to 7 days after the animal had recovered from the surgery.

On the day of testing, the animals were randomly grouped according to body weight using an Excel random grouping table. The grouping is shown in Table 32 below.

**Table 32**

| Group | Test article (route of administration, dose) |
|---|---|
| 1 (Vehicle group) | 10% sulfobutyl-β-cyclodextrin aqueous solution (oral gavage) |
| 2 (Control group) | Psilocybin (intraperitoneal injection, 1 mg/kg) |
| 3 (Administration group) | Example Compound 8 (oral gavage, 1 mg/kg) |
| 4 (Administration group) | Example Compound 8 (oral gavage, 3.2 mg/kg) |
| 5 (Administration group) | Example Compound 8 (oral gavage, 10 mg/kg) |
| 6 (Administration group) | Example Compound 11 (oral gavage, 3 mg/kg) |
| 7 (Administration group) | Example Compound 11 (oral gavage, 10 mg/kg) |
| 8 (Administration group) | Example Compound 11 (oral gavage, 30 mg/kg) |
| 9 (Administration group) | Example Compound 14 (oral gavage, 3 mg/kg) |
| 10 (Administration group) | Example Compound 14 (oral gavage, 10 mg/kg) |
| 11 (Administration group) | Example Compound 14 (oral gavage, 30 mg/kg) |

### Experimental test:

Prior to the experimental test, the animals were acclimated to the testing room for 1 hour. For the Psilocybin group, head twitch behavior was recorded for 60 minutes immediately after administration using Noldus software. For the vehicle group and compound group, head twitch behavior was recorded for 60 minutes beginning 30 minutes after administration using Noldus software. A peak threshold was set to analyze the number of head twitches in mice, and the cumulative counts were recorded every 10 minutes.

### Data analysis:

Data were collected using Excel and analyzed and plotted using GraphPad Prism. One-way ANOVA and/or two-way ANOVA followed by post-hoc multiple comparison analysis were employed to analyze the number of head twitches in animals of each group under different drug treatments. Compared with the vehicle group, ****p < 0.0001 was considered statistically significant. **The experimental results are shown in Table 33.**

**Table 33**

| **Group** | **Number of head twitches in mice** |
|---|---|
| Vehicle group | 6.0 |
| 1 mg/kg Psilocybin | 35.7**** |
| 1 mg/kg Compound 8 | 5.0 |
| 3.2 mg/kg Compound 8 | 4.0 |
| 10 mg/kg Compound 8 | 3.3 |
| 3 mg/kg Compound 11 | 3.0 |
| 10 mg/kg Compound 11 | 2.0 |
| 30 mg/kg Compound 11 | 0.3 |
| 3 mg/kg Compound 14 | 10.7 |
| 10 mg/kg Compound 14 | 6.0 |
| 30 mg/kg Compound 14 | 2.7 |

### Experimental conclusion:

The test articles were prepared according to the corresponding examples. The results showed that psilocybin could significantly increase the number of head twitches in mice. The compounds of the present disclosure did not increase the number of head twitches in mice and exhibited no hallucinogenic effects.

### Effect Example 14: Testing the Effects of Compounds on Locomotor Activity in Mice

### Experimental objective:

To study the effects of a single oral administration of the compounds of the examples of the present disclosure on locomotor activity in C57BL/6J mice within 24 hours.

**Experimental materials are shown in Table 34:**

**Table 34**

| **Materials** | **Supplier** | **Catalog No. or Model** |
|---|---|---|
| C57BL/6J mice (male, 6-8 weeks old) | Zhejiang Vital River Laboratory Animal Technology Co., Ltd. | 20250324Abzz06190000043 |
| Phenotyper experimental chamber | Noldus | Pheno Typer 3000 |
| Ethovision XT video analysis software | Noldus | Ethovision XT 15 |

### Experimental grouping:

The experimental animals were housed in the animal facility 5 days in advance. The animals were randomly grouped based on the principle of similar body weight, with 8 animals per group, all of which were male. A randomized grouping sequence was generated prior to the experiment, and the animals were assigned according to the randomized grouping sequence on the day of the experiment. The grouping information is shown in Table 35:

**Table 35**

| Group | Test article (route of administration, dose) |
|---|---|
| 1 (Vehicle group) | 10% sulfobutyl-β-cyclodextrin aqueous solution (oral gavage) |
| 2 (Administration group) | Example Compound 8 (oral gavage, 30 mg/kg) |
| 3 (Administration group) | Example Compound 11 (oral gavage, 100 mg/kg) |
| 4 (Administration group) | Example Compound 14 (oral gavage, 10 mg/kg) |
| 5 (Administration group) | Example Compound 37 (oral gavage, 30 mg/kg) |

### Experimental procedure:

The vehicle control group was administered the vehicle control substance by oral gavage, while the test article groups were administered different test articles by oral gavage.

The animals were tested immediately after administration, and locomotor activity within 24 hours after administration was measured. Mice were placed in a Phenotyper home cage for free movement over 24 hours. Videos were recorded and analyzed using Ethovision XT software to determine the time spent in the central zone and the total distance traveled of the animals. Detection index: time spent in the central zone (s).

After testing was completed, the animals were removed, and the Phenotyper home cages were wiped and cleaned with 75% ethanol.

### Data analysis:

The data results required to be measured and observed according to the protocol were manually recorded in appropriate forms or directly collected by computer. Statistical analysis was then performed, with measurement data expressed as mean ± standard deviation. All data statistics were conducted using SPSS 19.0 or Graphpad prism 8.0 statistical software. **The experimental results are shown in Table 36.**

**Table 36**

| **Group** | Total distance traveled (cm) |
|---|---|
| Vehicle group | 126291 |
| 10 mg/kg Compound 8 | 119256 |
| 10 mg/kg Compound 11 | 114826 |
| 10 mg/kg Compound 14 | 116580 |
| 10 mg/kg Compound 37 | 118105 |

.

### Experimental conclusion:

The results showed that there was no significant difference in the total distance traveled between mice treated with the compounds of the present application and those in the vehicle control group, indicating no effect on the spontaneous locomotor activity of the mice.

### Effect Example 15: Evaluation of Antidepressant Efficacy of Compounds in the Mouse Forced Swim Model

### Experimental objective:

To evaluate the antidepressant efficacy of the examples of the present disclosure in the forced swim model in C57BL/6J mice.

**Experimental materials are shown in Table 37:**

**Table 37**

| **Materials** | **Supplier** | **Catalog No. or Model** |
|---|---|---|
| C57BL/6J mice (male, 20-25 g, 6-8 weeks old) | SPF (Beijing) Biotechnology Co., Ltd. | 0000251112 |
| Forced swim test apparatus and analysis software | Vistrack | XR-VT |

**The experimental grouping is shown in Table 38:**
The animals were acclimated to the experimental environment for 7 days; body weights were recorded before the experiment, and the animals were randomly grouped. The grouping information is shown in the table below:

**Table 38**

| Group | Test article (route of administration, dose) |
|---|---|
| 1 (Vehicle group) | 10% sulfobutyl-β-cyclodextrin aqueous solution (oral gavage) |
| 2 (Psilocybin) | Control compound (intraperitoneal injection, 5 mg/kg) |
| 3 (Administration group) | Example Compound 8 (oral gavage, 5 mg/kg) |
| 4 (Administration group) | Example Compound 11 (oral gavage, 5 mg/kg) |
| 5 (Administration group) | Example Compound 14 (oral gavage, 5 mg/kg) |

### Experimental procedure:

Water at the adjusted temperature was added to a cylindrical swimming bucket for mice, with the water level reaching two-thirds of the height of the cylindrical bucket; the mice were placed into the cylindrical swimming bucket and allowed to pre-swim for 10 min; the mice were then removed, dried, and returned to their cages, with formal testing conducted on the following day.

On Day 2, water at the adjusted temperature was added to the cylindrical swimming bucket for mice; the positions of the forced swim apparatus for mice and the camera were adjusted, and the computer was connected to record the experimental video; one hour after administration, the mice were gently removed from the housing cages, soothed for 1 min, and when the animals were no longer nervous, they were placed into the water, and the operator immediately left the video recording area.

The swimming video of the mice within 6 min was recorded by the software. After the test was completed, the mice were removed, thoroughly dried, and returned to their cages. The above procedure was repeated until all mice had been tested.

After the experiment was completed, all animals were euthanized.

The immobility behavior of the mice was evaluated by data analysts through the video. The immobility duration of the mice during the 2-6 min of the test phase was analyzed, and the latency to the onset of immobility in this time period was recorded. Longer immobility duration in the mice indicated a greater degree of depression.

### Data analysis:

All raw data were entered into Excel and statistically analyzed using GraphPad Prism 9.0. The data were expressed as Mean ± S.E.M. Statistical differences between groups were analyzed by one-way ANOVA. Compared with the vehicle group, *p* < 0.05 indicates a significant difference, while p < 0.01 and p < 0.001 indicate highly significant differences. * indicates *p* < 0.05, ** indicates *p <* 0.01, and *** indicates *p* < 0.001.

**The experimental results are shown in Table 39:**

**Table 39**

| **Group** | **Immobility time (s)** |
|---|---|
| Vehicle group | 125 |
| 0.5 mg/kg Psilocybin | 102* |
| 5 mg/kg Compound 8 | 77*** |
| 5 mg/kg Compound 11 | 87** |
| 5 mg/kg Compound 14 | 102* |

**.**

### Experimental conclusion:

The test articles were prepared according to the corresponding examples. The results showed that the compounds of the present application could significantly reduce the immobility time of mice in the forced swim test and exhibited favorable antidepressant efficacy in the forced swim model in C57BL/6J mice.

### Effect Example 16: Evaluation of the Efficacy of Compounds in the Learned Helplessness Depression Model in Mice

### Experimental objective:

To evaluate the efficacy of the examples of the present disclosure in the learned helplessness depression model in C57BL/6J mice.

**Experimental materials are shown in Table 40:**

**Table 40**

| **Materials** | **Supplier** | **Catalog No. or Model** |
|---|---|---|
| C57BL/6J mice (male, 20-25 g, 6-8 weeks old) | Zhejiang Vital River Laboratory Animal Technology Co., Ltd. | Production License No.: SCXK (Zhe) 2020-0002 |
| Adjustable pipette | Eppendorf | J61188J |
| Constant-temperature magnetic stirrer | Shanghai Sile Instrument Co., Ltd. | 85-1 |
| Vortex mixer | Titan Scientific Lab | Vortex Mixer T1 |
| Electrical stimulation chamber and analysis software | Shanghai Jiliang Software Technology Co., Ltd. | JLBehv-STG-4 |

### Experimental procedure:

### 1. Electric shock training and model establishment:

The experimental mice were placed into an electrical stimulation shuttle box and subjected to training once daily for 8 consecutive days. Each session consisted of 120 unpredictable and inescapable foot shocks (current intensity: 0.3 mA; duration of each shock: randomly 1-3 seconds; random interval between two shocks: 1-15 seconds, with no light or sound cues provided before or during each shock).

### 2. Electric shock escape test and grouping:

Twenty-four hours after completion of the final electric shock training session, the experimental mice were subjected to a 30-trial shuttle escape test. Each trial began with a 5-second light stimulus, followed by a 10-second foot shock at an intensity of 0.3 mA, with an inter-trial interval of 30 seconds. During the shock, the electric shock was terminated when the mouse shuttled from the shocked side to the non-shocked side. Active escape during the shock under the light stimulus cue was recorded as a successful escape, while failure to escape during the shock was recorded as escape failure. In the 30 test trials, exhibiting more than 15 escape failures were defined as learned helplessness (LH). Finally, animals confirmed to exhibit LH behavior were randomly grouped, with 8 mice in each group, ensuring that the mean numbers of escape failures among groups were similar and showed no statistically significant differences. The grouping information is shown in Table 41 below:

**Table 41**

| Group | Test article (route of administration, dose) |
|---|---|
| 1 (Model group) | 10% sulfobutyl-β-cyclodextrin aqueous solution (oral gavage) |
| 2 (Psilocybin) | Control compound (intraperitoneal injection, 3 mg/kg) |
| 3 (S-ketamine) | Control compound (intraperitoneal injection, 10 mg/kg) |
| 4 (Administration group) | Example Compound 8 (oral gavage, 5 mg/kg) |
| 5 (Administration group) | Example Compound 11 (oral gavage, 5 mg/kg) |

### 3. Administration and testing:

Twenty-four hours after grouping and screening, the animals were administered according to the group dosing information, and the shuttle escape electric shock tests were conducted at 1 hour and 24 hours post-administration, respectively (using the same method as described above). After the experiment was completed, the experimental apparatus was properly stored, and the mice were euthanized.

### Data analysis:

All data were statistically analyzed and plotted using GraphPad Prism 10.0 software, with results expressed as mean ± standard error of the mean (Mean ± SEM). Post-administration data were analyzed using Two-way ANOVA, and inter-group comparisons were conducted using Fisher's LSD method. Compared with the vehicle group, *p* < 0.05 indicates a significant difference, while p < 0.01 and p < 0.001 indicate highly significant differences. * indicates *p* < 0.05, ** indicates *p* < 0.01, and *** indicates *p* < 0.001.

**The experimental results are shown in Table 42.**

**Table 42**

| **Group** | **Number of escape failures** | |
|---|---|---|
| | **1 hour Post-administration** | **24 hours Post-administration** |
| Model group | 22.25 | 17.00 |
| 3 mg/kg Psilocybin | 11.25^{**} | 1.13 *** |
| 10 mg/kg S-ketamine | 5.88*** | 5.88** |
| 5 mg/kg Compound 8 | 4.13*** | 3.38*** |
| 5 mg/kg Compound 11 | 3.88*** | 3.25*** |

.

**Experimental conclusion:** The test articles were prepared according to the corresponding examples. The results showed that the compounds of the present disclosure significantly reduced the number of escape failures in the learned helplessness test at 1 hour and 24 hours post-administration, alleviated depression-like behaviors, and exhibited favorable antidepressant efficacy in the learned helplessness depression model in C57BL/6J mice.

### Effect Example 17: Evaluation of the Efficacy of Compounds in the Chronic Unpredictable Mild Stress Depression Model in Mice

### Experimental objective:

A chronic unpredictable mild stress (CUMS) depression model was established in mice. The efficacy of the examples of the present invention in the depression model was evaluated by the sucrose preference test (SPT) and the forced swim test (FST).

**Experimental materials are shown in Table 43:**

**Table 43**

| **Materials** | **Supplier** | **Catalog No. or Model** |
|---|---|---|
| C57BL/6J mice (male, 20-25 g, 6-8 weeks old) | Shanghai Lingchang Biotechnology Co., Ltd. | 20230003015489 |
| Weighing balance | Changzhou Keyuan Electronic Instrument Co., Ltd. | Model YH20002-2212703 |
| Electronic balance | Sartorius | Model SQP SECURA225D-1CN-1189301 |
| Dahua video recorder | Zhejiang Dahua Technology Co., Ltd. | 8F03D09PAZ8CBA6 |
| Noldus software | Noldus (Beijing) Information Technology Co., Ltd. | EV170-08974-AAMI |

### Experimental procedure:

### 1. Acclimation

The animals were acclimated to the environment for 7 days, during which they were subjected to gentle handling for 1-2 minutes daily for 5 consecutive days for adaptation.

### 2. Baseline sucrose preference test

Before the experiment, the mice were singly housed and acclimated in the laboratory for 24 hours. On Day 1, the mice were acclimated to two bottles of regular water. On Day 2, the mice were acclimated to two bottles of 1% sucrose solution^{a,b}. On Day 3, the mice were acclimated to one bottle of 1% sucrose solution and one bottle of regular water. On Day 4 at 5:00 PM, the sucrose solution was weighed and the initial bottle weights were recorded. On Day 5 at 9:30 AM, the sucrose solution was weighed, and the bottle weights after consumption were recorded. If the preference level was not high (the mean sucrose preference index of each group was required to be 0.85 or above), sucrose preference testing continued from 5:00 PM on Day 5 to 9:30 AM on Day 6. After each weighing, the positions of the sucrose solution and regular water were exchanged to prevent the formation of positional preference.

Note: a. Sucrose Preference Index = sucrose solution intake / (sucrose solution intake + regular drinking water intake). b. 1% sucrose solution: 0.01 g/mL aqueous sucrose solution.

### 3. Grouping

According to the baseline sucrose preference test results, the test animals were randomly divided into a normal vehicle control group, a model group, a positive control drug group, and example compound groups.

### 4. Model establishment

After random grouping according to the baseline sucrose preference test results obtained before model establishment, animals in all groups except the normal vehicle control group were exposed to randomly assigned mild stressors daily for 4 consecutive weeks. After completion of the weekly stress training, the animals were subjected to sucrose preference tests and body weight measurements. When the tested sucrose preference index was ≤ 0.70, the mice were considered to have developed anhedonia-like depressive behavior, indicating successful establishment of the CUMS model. Based on the sucrose preference test results and body weights in the fourth week, all groups except the normal vehicle control group were regrouped, with 12 mice per group. The grouping information is shown in Table 44 below:

**Table 44**

| Group | Test article (route of administration, dose) |
|---|---|
| 1 (Vehicle group) | 10% sulfobutyl-β-cyclodextrin aqueous solution (oral gavage) |
| 2 (Model group) | 10% sulfobutyl-β-cyclodextrin aqueous solution (oral gavage) |
| 3 (Ketamine) | Control compound (intraperitoneal injection, 20 mg/kg) |
| 4 (Administration group) | Example Compound 8 (oral gavage, 5 mg/kg) |
| 5 (Administration group) | Example Compound 11 (oral gavage, 5 mg/kg) |
| 6 (Administration group) | Example Compound 14 (oral gavage, 5 mg/kg) |

### 5. Administration testing

On Day 1, the forced swim test (FST) was conducted 1 hour after administration, followed by the sucrose preference test (SPT) upon completion of the FST. The animals were administered for 7 consecutive days. On Day 7, behavioral tests were conducted 1 hour after administration in the same order as on Day 1. During this period, the animals continued to receive randomly assigned mild stress.

### Data analysis:

Data were collected using Excel software. Data analysis and graphing were performed using Prism 10.1.2 (GraphPad Software, Inc.), with bar charts generated for the sucrose preference and forced swim data. Data from each animal group were compared using one-way ANOVA, with LSD post hoc test for multiple comparisons. Compared with the model group, p < 0.05 indicates a significant difference, while *p* < 0.01, *p* < 0.001, and *p <* 0.0001 indicate highly significant differences. * represents p < 0.05, ** represents p < 0.01, *** represents p < 0.001, and **** represents p < 0.0001.

**The experimental results are shown in Table 45.**

**Table 45**

| **Group** | **Day 1 Administration** | | **Day 7 Administration** | |
|---|---|---|---|---|
| | **Sucrose preference index** | **Immobility time (s)** | **Sucrose preference index** | **Immobility time (s)** |
| Vehicle group | 0.84*** | 82* | 0.85^{****} | 84* |
| Model group | 0.54 | 126 | 0.36 | 117 |
| 20 mg/kg ketamine | 0.84*** | 70*** | 0.81 ^{****} | 43^{****} |
| 5 mg/kg Compound 8 | 0.68 | 93* | 0.72**** | 56*** |
| 5 mg/kg Compound 11 | 0.79** | 24**** | 0.78**** | 19**** |
| 5 mg/kg Compound 14 | 0.74* | 41^{****} | 0.72**** | 50^{****} |

**Experimental conclusion:** The test articles were prepared according to the corresponding examples. The results showed that, compared with the model group, single oral gavage administration or continuous oral gavage administration for 7 days of the compounds of the present application significantly improved depression-like behaviors such as despair and anhedonia in model mice (the sucrose preference test evaluated the degree of anhedonia in animals, and the forced swim test evaluated the degree of behavioral despair in animals), demonstrating favorable antidepressant efficacy in the mouse CUMS model.

Although specific embodiments of the present disclosure are described above, those skilled in the art should understand that these are merely illustrative, and various changes or modifications may be made to these embodiments without departing from the principles and essence of the present disclosure. Therefore, the scope of protection of the present disclosure is defined by the appended claims.

## Claims

1. A compound represented by formula (II), a pharmaceutically acceptable salt thereof, a solvate thereof, or a solvate of the pharmaceutically acceptable salt thereof: 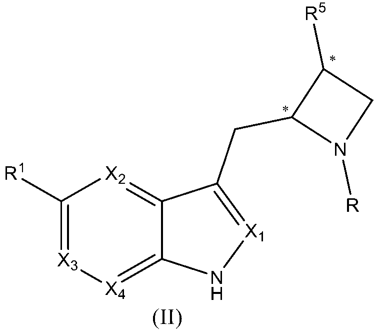 wherein
the configuration of the carbon atom marked with "*" is R configuration, S configuration, or a mixture thereof;
R is C₁-C₆ alkyl, C₃-C₆ cycloalkyl, C₂-C₆ alkynyl, or C₁-C₆ alkyl substituted with one or more R^{a};
R^{a} is D, OH, C₃-C₆ cycloalkyl, cyano, halogen, or -S(=O)₂C₁-C₆ alkyl;
X₁ is N or CH;
X₂ is N or CR²;
R² is H or halogen;
R¹ is H, hydroxyl, halogen, CN, C₁-C₆ alkyl, -S(=O)₂C₁-C₆ alkyl, C₁-C₆ alkoxy, C₃-C₆ cycloalkyl, C₁-C₆ alkyl substituted with one or more R¹⁻¹, or C₁-C₆ alkoxy substituted with one or more R¹⁻²;
R¹⁻¹ and R¹⁻² are each independently halogen;
alternatively, R¹ and R², together with the atoms to which they are attached, form a 5- to 6-membered heterocycloalkene, wherein the heteroatom in the 5- to 6-membered heterocycloalkene is one or more types selected from the group consisting of N, O, and S, and the number of heteroatoms is 1, 2, or 3;
X₃ is N or CR³;
R³ is H or halogen;
alternatively, R³ and R¹, together with the atoms to which they are attached, form a 5- to 6-membered heterocycloalkene, wherein the heteroatom in the 5- to 6-membered heterocycloalkene is one or more types selected from the group consisting of N, O, and S, and the number of heteroatoms is 1, 2, or 3;
X₄ is N or CR⁴;
R⁴ is H or halogen;
R⁵ is H or C₁-C₆ alkyl;
the compound represented by formula (I) is not any one of the following compounds: 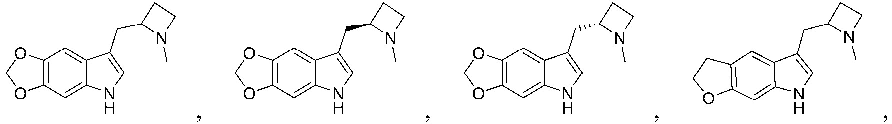 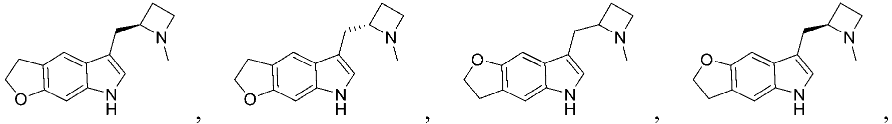 or

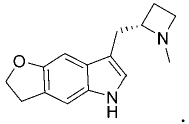

2. The compound represented by formula (II), the pharmaceutically acceptable salt thereof, the solvate thereof, or the solvate of the pharmaceutically acceptable salt thereof according to claim 1, wherein
R¹ is H, halogen, CN, C₁-C₆ alkyl, -S(=O)₂C₁-C₆ alkyl, C₁-C₆ alkoxy, C₃-C₆ cycloalkyl, C₁-C₆ alkyl substituted with one or more R¹⁻¹, or C₁-C₆ alkoxy substituted with one or more R¹⁻².

3. The compound, the pharmaceutically acceptable salt thereof, the solvate thereof, or the solvate of the pharmaceutically acceptable salt thereof according to claim 1, wherein the compound is a compound represented by formula (I): 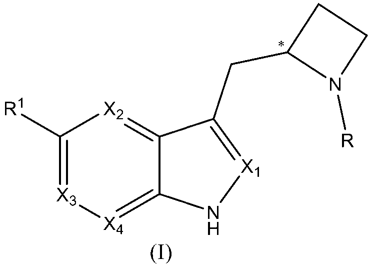 wherein 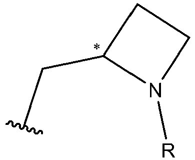 is 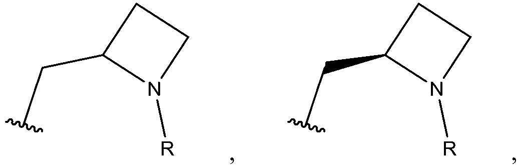 or 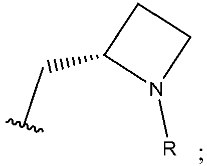 preferably 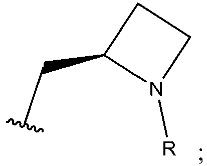
R is C₁-C₆ alkyl, C₃-C₆ cycloalkyl, C₂-C₆ alkynyl, or C₁-C₆ alkyl substituted with one or more R^{a};
R^{a} is D, OH, C₃-C₆ cycloalkyl, cyano, halogen, or -S(=O)₂C₁-C₆ alkyl;
X₁ is N or CH;
X₂ is N or CR²;
R² is H or halogen;
R¹ is H, halogen, CN, C₁-C₆ alkyl, -S(=O)₂C₁-C₆ alkyl, C₁-C₆ alkoxy, C₃-C₆ cycloalkyl, C₁-C₆ alkyl substituted with one or more R¹⁻¹, or C₁-C₆ alkoxy substituted with one or more R¹⁻²;
R¹⁻¹ and R¹⁻² are each independently halogen;
alternatively, R¹ and R², together with the atoms to which they are attached, form a 5- to 6-membered heterocycloalkene, wherein the heteroatom in the 5- to 6-membered heterocycloalkene is one or more types selected from the group consisting of N, O, and S, and the number of heteroatoms is 1, 2, or 3;
X₃ is N or CR³;
R³ is H or halogen;
alternatively, R³ and R¹, together with the atoms to which they are attached, form a 5- to 6-membered heterocycloalkene, wherein the heteroatom in the 5- to 6-membered heterocycloalkene is one or more types selected from the group consisting of N, O, and S, and the number of heteroatoms is 1, 2, or 3;
X₄ is N or CR⁴;
R⁴ is H or halogen;
the compound represented by formula (I) is not any one of the following compounds: 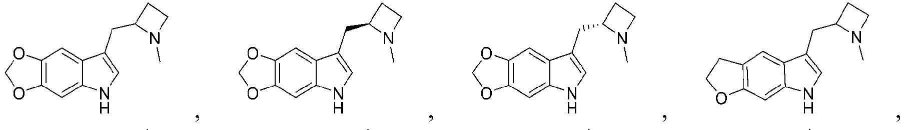 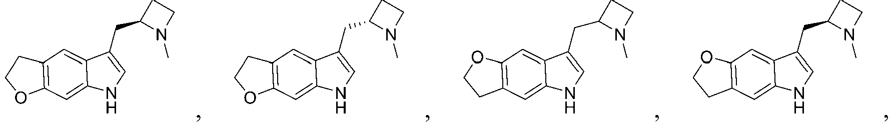 or

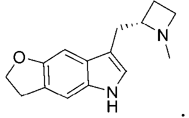

4. The compound, the pharmaceutically acceptable salt thereof, the solvate thereof, or the solvate of the pharmaceutically acceptable salt thereof according to at least one of claims 1 to 3, wherein the compound is further not any one of the following compounds:

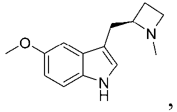

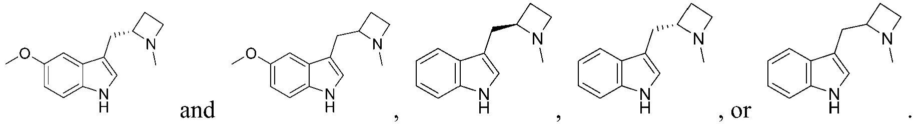

5. The compound, the pharmaceutically acceptable salt thereof, the solvate thereof, or the solvate of the pharmaceutically acceptable salt thereof according to at least one of claims 1 to 4, wherein the compound, the pharmaceutically acceptable salt thereof, the solvate thereof, or the solvate of the pharmaceutically acceptable salt thereof satisfies one or more of the following conditions:
(1) each "halogen" is independently F, Cl, Br, or I, for example, F;
(2) each "C₁-C₆ alkoxy" is independently methoxy, ethoxy, *n*-propoxy, isopropoxy, *n-*butoxy, isobutoxy, *sec*-butoxy, or *tert*-butoxy, for example, methoxy, ethoxy, or isopropoxy;
(3) each "C₁-C₆ alkyl" is independently methyl, ethyl, *n*-propyl, isopropyl, *n*-butyl, isobutyl, sec-butyl, or *tert-*butyl, for example, methyl, ethyl, isopropyl, or isobutyl;
(4) each "C₂-C₆ alkynyl" is independently ethynyl, propynyl, or propargyl, for example, ethynyl;
(5) each "C₃-C₆ cycloalkyl" is independently cyclopropyl, cyclobutyl, cyclopentyl, or cyclohexyl, for example, cyclopropyl;
(6) each "5- to 6-membered heterocycloalkene" is independently a 5- to 6-membered heterocycloalkene with 1 or 2 heteroatoms being O, for example, a dihydrofuran ring or a dihydropyran ring, further for example,

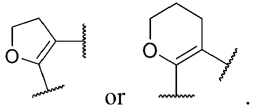

6. The compound, the pharmaceutically acceptable salt thereof, the solvate thereof, or the solvate of the pharmaceutically acceptable salt thereof according to at least one of claims 1 to 4, wherein the compound, the pharmaceutically acceptable salt thereof, the solvate thereof, or the solvate of the pharmaceutically acceptable salt thereof satisfies one or more of the following conditions:
(1) 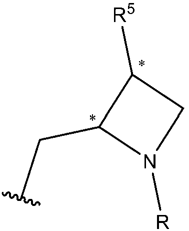 is 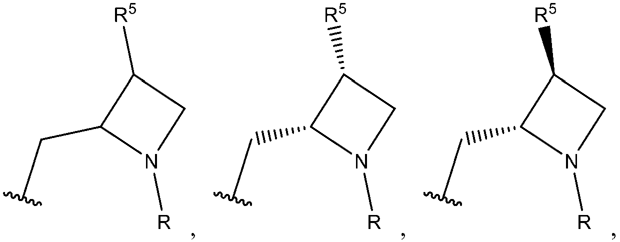 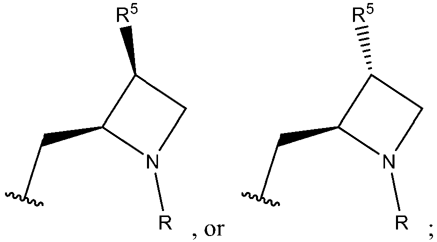
(2) R is -CH₃, -CD₃, -CH₂CH₃, 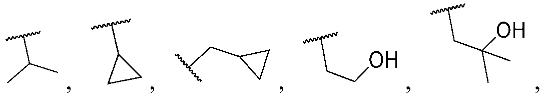 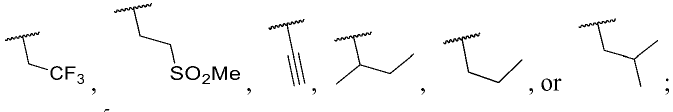
(3) R³ is -H or -CH₃;
(4) R¹ is C₁-C₆ alkoxy or halogen.

7. The compound, the pharmaceutically acceptable salt thereof, the solvate thereof, or the solvate of the pharmaceutically acceptable salt thereof according to at least one of claims 1 to 4, wherein the compound, the pharmaceutically acceptable salt thereof, the solvate thereof, or the solvate of the pharmaceutically acceptable salt thereof satisfies one or more of the following conditions:
(1) 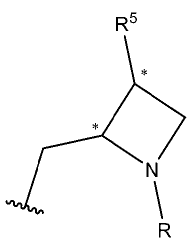 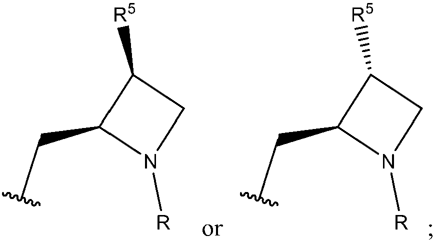
(2) R is -CH₃ or 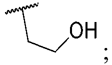
(3) R¹ is methoxy or F.

8. The compound, the pharmaceutically acceptable salt thereof, the solvate thereof, or the solvate of the pharmaceutically acceptable salt thereof according to at least one of claims 1 to 4, wherein the compound, the pharmaceutically acceptable salt thereof, the solvate thereof, or the solvate of the pharmaceutically acceptable salt thereof satisfies one or more of the following conditions:
(1) R is -CH₃, -CD₃, -CH₂CH₃, 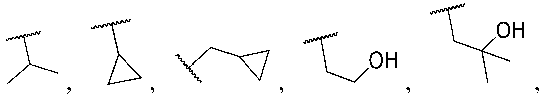 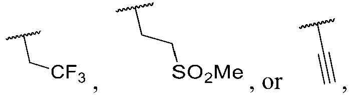 for example -CH₃;
(2) R² is H or F; alternatively, R¹ and R², together with the atoms to which they are attached, form 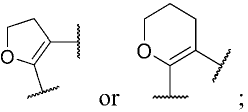
(3) R¹ is -H, -F, -CN, -CH₃, -CF₃, -OCH₃, 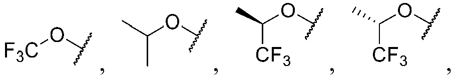 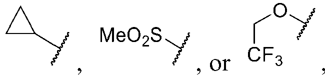 preferably -H, -F, -CN, -CF₃, -OCH₃, 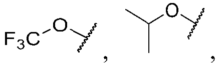 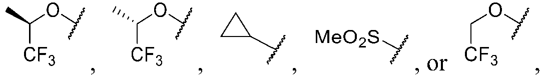 alternatively, R¹ and R², together with the atoms to which they are attached, form 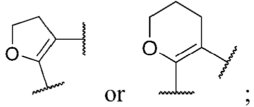
(4) R³ is H or F;
(5) R⁴ is H or F.

9. The compound, the pharmaceutically acceptable salt thereof, the solvate thereof, or the solvate of the pharmaceutically acceptable salt thereof according to at least one of claims 1 to 4, wherein the compound, the pharmaceutically acceptable salt thereof, the solvate thereof, or the solvate of the pharmaceutically acceptable salt thereof satisfies one or more of the following conditions:
(1) R is C₁-C₆ alkyl;
(2) R² is H;
(3) R¹ is C₁-C₆ alkoxy, for example, methoxy;
(4) R³ is H;
(5) R⁴ is H.

10. The compound, the pharmaceutically acceptable salt thereof, the solvate thereof, or the solvate of the pharmaceutically acceptable salt thereof according to at least one of claims 1 to 9, wherein the compound, the pharmaceutically acceptable salt thereof, the solvate thereof, or the solvate of the pharmaceutically acceptable salt thereof satisfies any one of the following conditions:
(1) 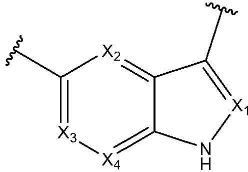 is 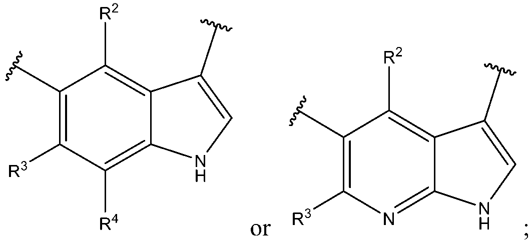
(2) 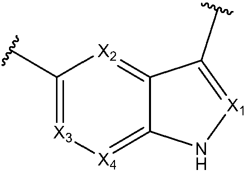 is 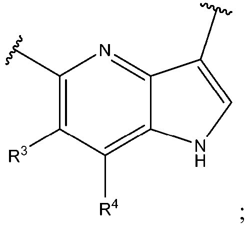
(3) 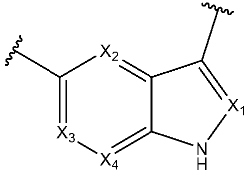 is 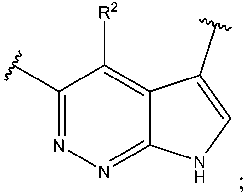
(4) 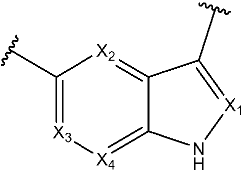 is 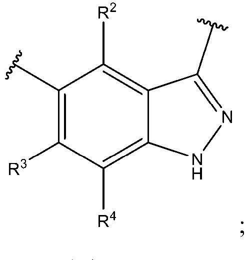
(5) X₂ is CR²; R¹ and R², together with the atoms to which they are attached, form a 5- to 6-membered heterocycloalkene, for example,

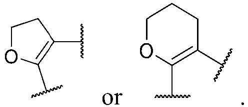

11. The compound, the pharmaceutically acceptable salt thereof, the solvate thereof, or the solvate of the pharmaceutically acceptable salt thereof according to at least one of claims 1 to 9, wherein 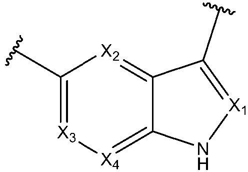 is

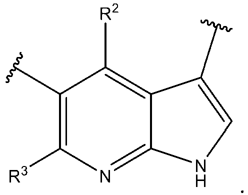

12. The compound, the pharmaceutically acceptable salt thereof, the solvate thereof, or the solvate of the pharmaceutically acceptable salt thereof according to at least one of claims 1 to 4, wherein the compound is any one of the following cases:
Case 1: 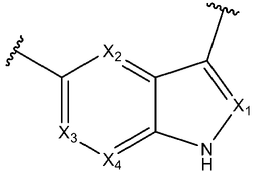 is 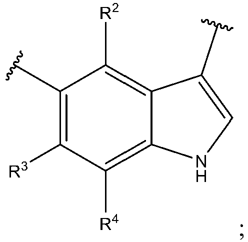
R is C₁-C₆ alkyl or C₁-C₆ alkyl substituted with one or more hydroxyl groups, for example, C₁-C₆ alkyl;
R¹ is halogen or C₁-C₆ alkoxy;
R², R³, and R⁴ are each independently H or halogen;
Case 2: 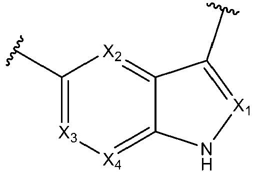 is 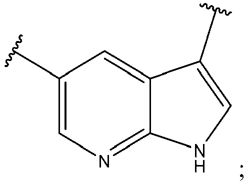
R¹ is halogen or C₁-C₆ alkoxy;
R is C₁-C₆ alkyl;
Case 3: 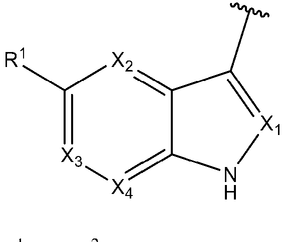 is 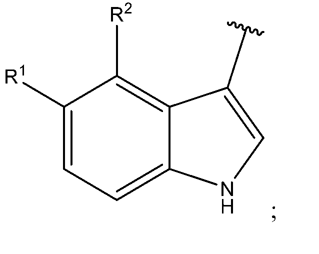
R¹ and R², together with the atoms to which they are attached, form a 5- to 6-membered heterocycloalkene, wherein the heteroatom in the 5- to 6-membered heterocycloalkene is O, and the number of heteroatoms is 1;
R is C₁-C₆ alkyl or C₁-C₆ alkyl substituted with one or more hydroxyl groups;
Case 4: 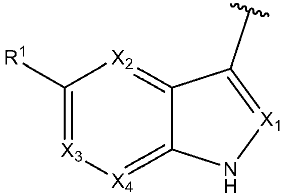 is 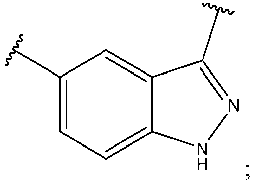
R¹ is C₁-C₆ alkoxy;
R is C₁-C₆ alkyl.

13. The compound, the pharmaceutically acceptable salt thereof, the solvate thereof, or the solvate of the pharmaceutically acceptable salt thereof according to claim 1, wherein the compound is any one of the following compounds:

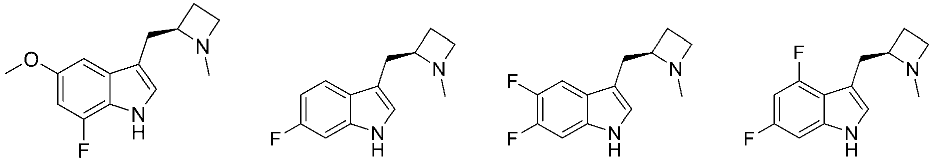

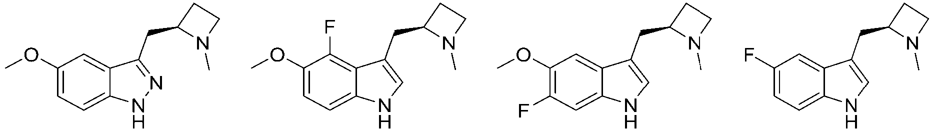

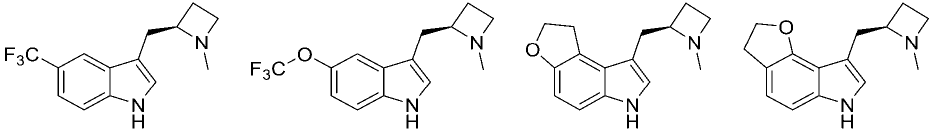

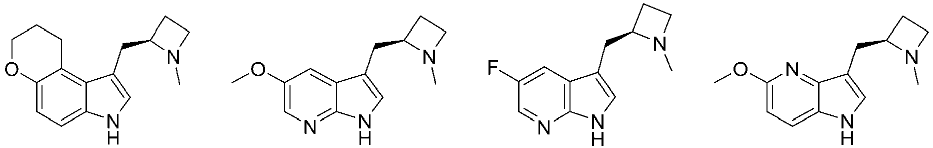

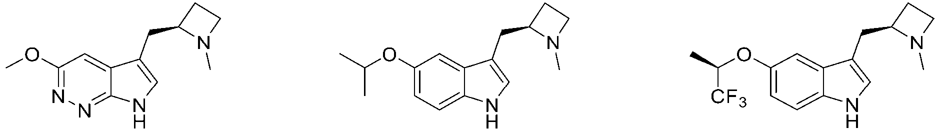

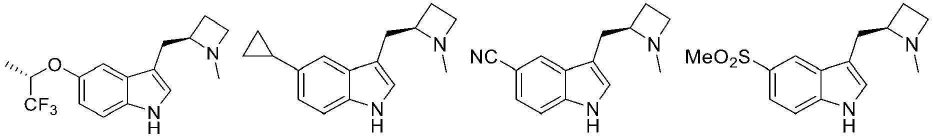

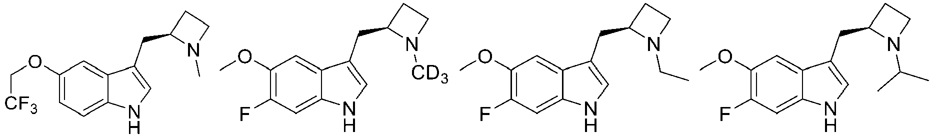

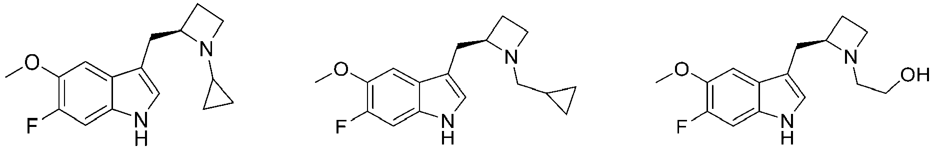

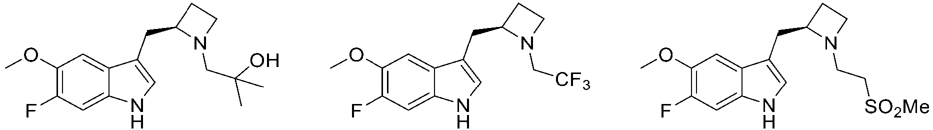

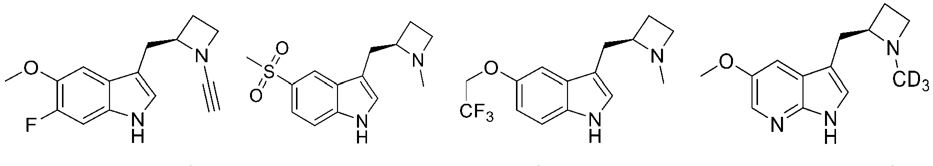

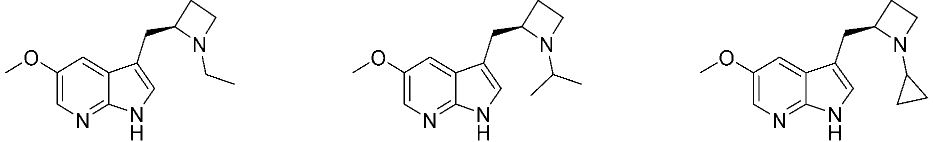

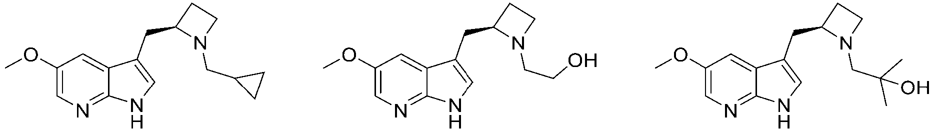

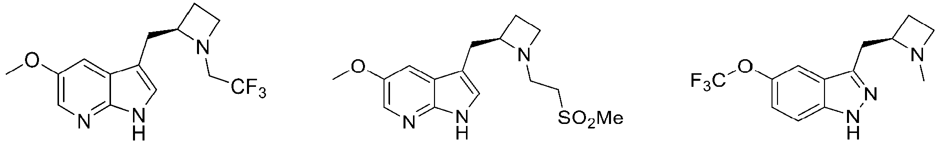

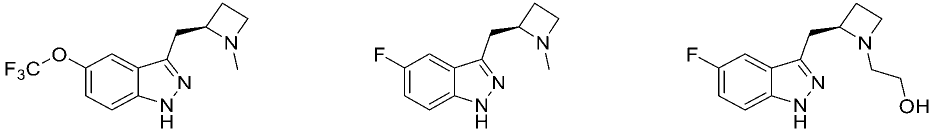

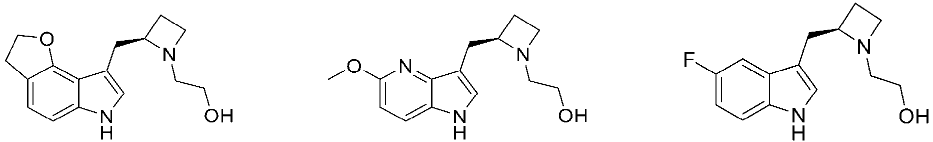

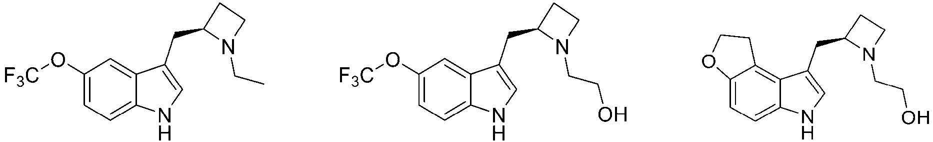

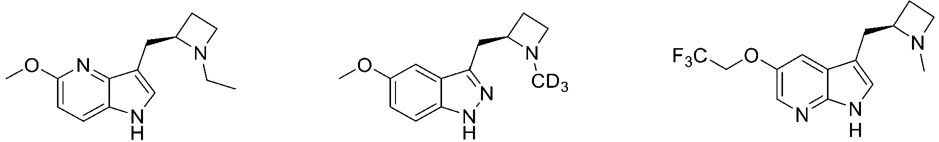

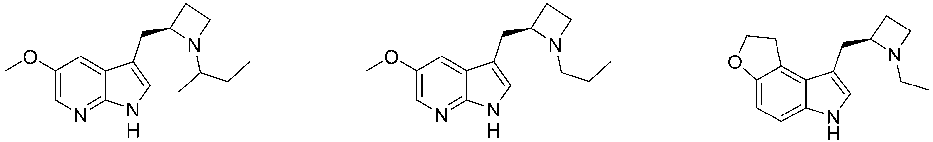

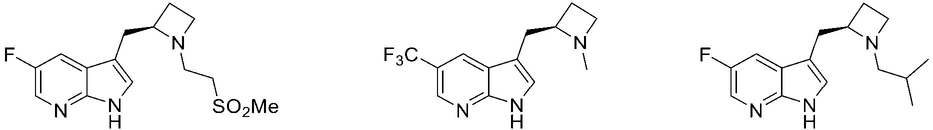

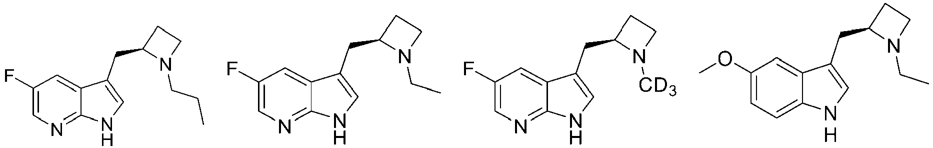

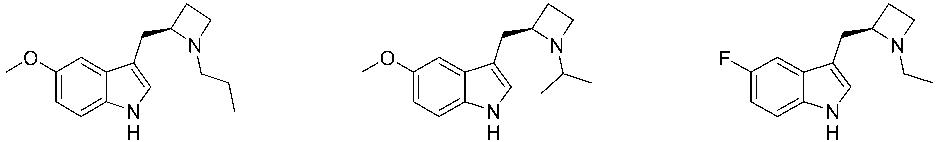

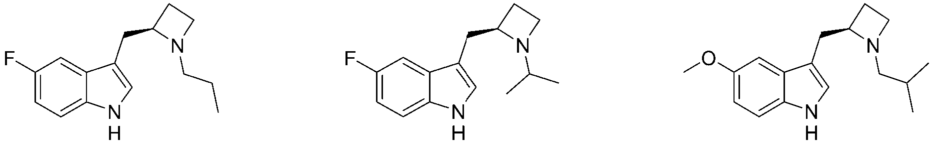

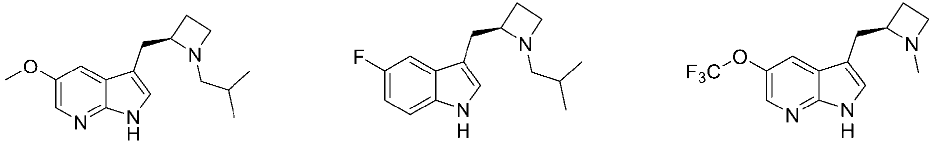

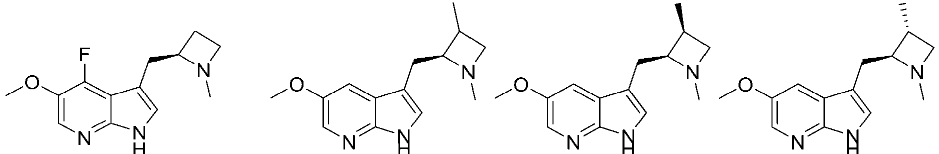

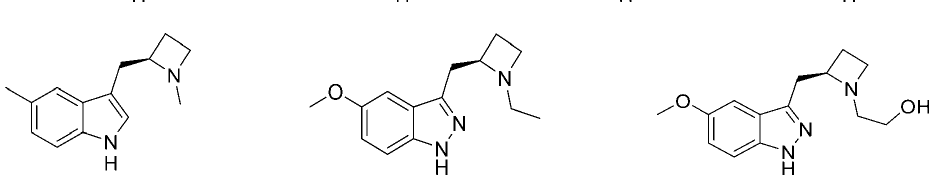

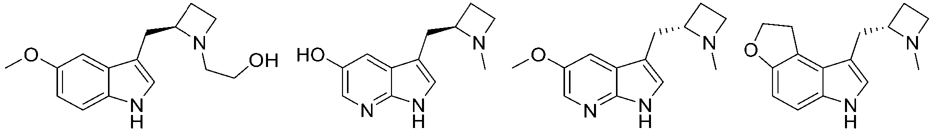

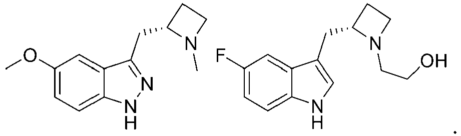

14. A pharmaceutical composition comprising the compound, the pharmaceutically acceptable salt thereof, the solvate thereof, or the solvate of the pharmaceutically acceptable salt thereof according to at least one of claims 1 to 13, and a pharmaceutically acceptable excipient.

15. A use of the compound, the pharmaceutically acceptable salt thereof, the solvate thereof, or the solvate of the pharmaceutically acceptable salt thereof according to at least one of claims 1 to 13, or the pharmaceutical composition according to claim 14, wherein the use is one or more selected from the group consisting of:
(1) use in the manufacture of a medicament for regulating neuronal plasticity;
(2) use in the manufacture of a medicament for preventing and/or treating depression, schizophrenia, anxiety, or post-traumatic stress disorder;
(3) use in the manufacture of a 5-HT_{2A} receptor agonist; preferably, the 5-HT_{2A} receptor agonist is a 5-HT_{2A} selective receptor agonist, for example, a selective receptor agonist for 5-HT_{2A} relative to 5-HT_{2B};
(4) use in the manufacture of a medicament for preventing and/or treating a disease associated with a 5-HT_{2A} receptor; preferably, the disease associated with the 5-HT_{2A} receptor is depression, schizophrenia, anxiety, or post-traumatic stress disorder.
